(19) Europäisches Patentamt
European Patent Office
Office européen des brevets

(11) EP 3 964 253 B1

(12) EUROPÄISCHE PATENTSCHRIFT

(45) Veröffentlichungstag und Bekanntmachung des Hinweises auf die Patenterteilung:
**28.06.2023 Patentblatt 2023/26**

(21) Anmeldenummer: **21193003.7**

(22) Anmeldetag: **25.08.2021**

(51) Internationale Patentklassifikation (IPC):
***A61M 16/00*** *(2006.01)* ***A61B 5/087*** *(2006.01)*
***A61B 5/00*** *(2006.01)* ***A61B 5/0205*** *(2006.01)*
***A61B 5/085*** *(2006.01)* ***A61B 5/08*** *(2006.01)*
***G16H 20/00*** *(2018.01)* ***A61M 16/10*** *(2006.01)*
***A61B 5/091*** *(2006.01)* ***A61M 16/04*** *(2006.01)*

(52) Gemeinsame Patentklassifikation (CPC):
(C-Sets verfügbar)
**A61M 16/024; A61B 5/037; A61B 5/082; A61B 5/085; A61B 5/087; A61B 5/091; A61B 5/4836;** A61B 5/08; A61M 16/0415; A61M 16/0461; A61M 2016/0027; A61M 2016/0033; A61M 2205/502; A61M 2209/082; A61M 2209/084;

(Forts.)

(54) **COMPUTERPROGRAMM UND VORRICHTUNG ZUR AUTOMATISCHEN FESTLEGUNG DER SOLLFREQUENZ EINES BEATMUNGSGERÄTS**

COMPUTER PROGRAM AND DEVICE FOR AUTOMATICALLY DETERMINING THE TARGET FREQUENCY OF A BREATHING APPARATUS

PROGRAMME D'ORDINATEUR ET DISPOSITIF DE DÉTERMINATION AUTOMATIQUE DE LA FRÉQUENCE DE CONSIGNE D'UN RESPIRATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.09.2020 DE 102020123138**

(43) Veröffentlichungstag der Anmeldung:
**09.03.2022 Patentblatt 2022/10**

(73) Patentinhaber: **Drägerwerk AG & Co. KGaA**
**23558 Lübeck (DE)**

(72) Erfinder:
- **von Blumenthal, Tilman**
  **23558 Lübeck (DE)**
- **Stender, Birgit**
  **23558 Lübeck (DE)**

(56) Entgegenhaltungen:
**EP-A1- 2 246 087** **WO-A1-2007/131314**
**WO-A1-2013/027137** **US-A1- 2008 236 582**

- **REES S E ET AL: "Using physiological models and decision theory for selecting appropriate ventilator settings", JOURNAL OF CLINICAL MONITORING AND COMPUTING, SPRINGER NETHERLANDS, NL, Bd. 20, Nr. 6, 15. September 2006 (2006-09-15), XP037122867, ISSN: 1387-1307, DOI: 10.1007/S10877-006-9049-5 [gefunden am 2006-09-15]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
A61M 2230/005; A61M 2230/04; A61M 2230/40; A61M 2230/42; A61M 2230/432; A61M 2230/46; A61M 2230/60

C-Sets
A61M 2230/04, A61M 2230/005;
A61M 2230/40, A61M 2230/005;
A61M 2230/42, A61M 2230/005;
A61M 2230/60, A61M 2230/005

## Beschreibung

Die Erfindung betrifft eine Signalverarbeitungseinheit und ein Computerprogramm, um automatisch einen Sollwert für die Frequenz festzulegen, mit welcher ein Beatmungsgerät eine Abfolge von Beatmungshüben ausführt und dadurch einen Patienten künstlich beatmet. Weiterhin betrifft die Erfindung ein Beatmungsgerät, welches automatisch eine Soll-Beatmungsfrequenz für die eigenen Beatmungshübe berechnet.

Ein Verfahren und eine Vorrichtung, um eine Soll-Beatmungsfrequenz zu berechnen, werden in EP 3 332 827 A1 beschrieben. Die Frequenz, mit der ein Patient künstlich beatmet werden soll, wird automatisch berechnet. Die Berechnung hängt von einem angestrebten Minutenvolumen (Volumenfluss in die und aus der Lunge des Patienten) sowie einem vorgegebenen funktionalen Totraum ab, außerdem von einem vorgegebenen Lungenmodell, einer abgespeicherten Lungenzeitkonstante, z.B. R*C, und einem vorgegebenen Zeitdauerverhältnis, z.B. Inspiration I zu Exspiration E. Die Frequenz wird so berechnet, dass ein von der Frequenz abhängender Parameter minimiert wird. In einem Ausführungsbeispiel ist dieser Parameter die Summe aus Wc und $W'_R$, also die Summe aus der Leistung Wc, die zum Dehnen der Lunge erforderlich ist, und der Leistung $W'_R$, die zur Überwindung des pneumatischen Widerstands im Atemweg erforderlich ist. Um die Sollfrequenz festzulegen, wird eine Optimierung durchgeführt, im Ausführungsbeispiel eine iterative Optimierung.

In US 2009 / 0 007 915 A1 werden eine Vorrichtung und ein Verfahren beschrieben, um automatisch mehrere Werte für Parameter einer künstlichen Beatmung zu berechnen. Eine gewünschte Frequenz $RR^{sp}$ der Beatmung wird festgelegt, und eine Frequenz $RR_{spon}$ der spontanen Atmung wird ermittelt. Die beiden Frequenzen werden miteinander verglichen. Abhängig von der Abweichung wird ein Zielwert für die Beatmung festgelegt. Der Zielwert kann insbesondere die gesamte alveoläre Ventilation oder das absolute oder relative Minutenvolumen sein. Falls die Frequenz der spontanen Atmung signifikant von der Sollfrequenz abweicht, so wird der Zielwert für die Beatmung vergrößert.

In US 2008 / 0 236 582 A1 werden ein Verfahren und eine Vorrichtung beschrieben, um die künstliche Beatmung (mechanical ventilation) eines Patienten zu optimieren. Die Vorrichtung empfängt Patientendaten, darunter den pneumatischen Widerstand im Atemweg, die respiratorische Compliance und das Idealgewicht des Patienten. Hieraus werden ein optimales Niveau und eine optimale Frequenz für die gesamte Atmung des Patienten hergeleitet. Diese gesamte Atmung wird durch die spontane Atmung und die künstliche Beatmung bewirkt. Außerdem werden der Sauerstoffgehalt, die Frequenz der spontanen Atmung, das Tidalvolumen, der eingeatmete Anteil an Sauerstoff sowie der positive endexspiratorischen Druck (PEEP) gemessen. Aus diesen Daten wird ein Niveau der künstlichen Beatmung hergeleitet.

Der Erfindung liegt die Aufgabe zugrunde, eine Vorrichtung und ein Computerprogramm bereitzustellen, welche automatisch die Soll-Beatmungsfrequenz eines Beatmungsgeräts festlegen, wobei diese Festlegung für einen relativ breiten Anwendungsbereich gültig ist und wobei es nicht erforderlich sein soll, das Beatmungsgerät während der künstlichen Beatmung eines Patienten zwischen unterschiedlichen Modi umzuschalten.

Die Aufgabe wird durch eine Signalverarbeitungseinheit mit den Merkmalen des Anspruchs 1, durch ein Beatmungsgerät mit den Merkmalen des Anspruchs 12 und durch ein Computerprogramm mit den Merkmalen des Anspruchs 13 gelöst. Vorteilhafte Ausgestaltungen sind in den Unteransprüchen angegeben. Vorteilhafte Ausgestaltungen der erfindungsgemäßen Signalverarbeitungseinheit sind, soweit sinnvoll, auch vorteilhafte Ausgestaltungen des erfindungsgemäßen Beatmungsgeräts und des erfindungsgemäßen Computerprogramms und umgekehrt.

Im Folgenden wird der Begriff "eigene Atmungsaktivität" des Patienten verwendet. Diese eigene Atmungsaktivität wird von der Atmungsmuskulatur des Patienten selber bewirkt und kann von Signalen hervorgerufen werden, die im Körper des Patienten erzeugt werden (spontane Atmung), und / oder von Signalen, die ein medizinisches Gerät erzeugt, um die eigene Atmungsmuskulatur des Patienten von außen zu stimulieren.

Zwischen der Lunge eines Patienten, welcher künstlich zu beatmen ist oder beatmet wird, und dem erfindungsgemäßen Beatmungsgerät ist wenigstens zeitweise eine Fluidverbindung hergestellt oder lässt sich herstellen. Das Beatmungsgerät ist wenigstens zeitweise mit mindestens einem Patienten-Sensor verbunden oder lässt sich mit mindestens einem Patienten-Sensor verbinden. Der oder jeder Patienten-Sensor vermag jeweils mindestens einen respiratorischen Parameter des künstlich zu beatmenden oder künstlich beatmeten Patienten zu messen.

Die erfindungsgemäße Signalverarbeitungseinheit sowie das erfindungsgemäße Beatmungsgerät umfassen einen Datenspeicher oder weisen wenigstens zeitweise Lesezugriff auf einen Datenspeicher auf. In diesem Datenspeicher ist in einer von einem Rechner auswertbaren Form ein Maß für einen angestrebten Volumenfluss in die Lunge des Patienten abgespeichert. Ein solcher angestrebter Volumenfluss wird dem erfindungsgemäßen Verfahren vorgegeben.

Ein Volumenfluss ist das Volumen von Fluid, das in einer bestimmten Zeiteinheit in einen und / oder aus einem Raum fließt, z.B. in [l/min], hier: in die und aus der Lunge. Der angestrebte Volumenfluss ist insbesondere ein gefordertes alveoläres oder proximales Minutenvolumen.

Das erfindungsgemäße Computerprogramm ist dazu ausgestaltet, bei seiner Ausführung die folgenden Schritte automatisch durchzuführen, und die erfindungsgemäße Signalverarbeitungseinheit und das erfindungsgemäße Bearbeitungsgerät sind dazu ausgestaltet und eingerichtet, die folgenden Schritte automatisch durchzuführen:

- Eine Lungenzeitkonstante für die Lunge des Patienten wird ermittelt. Für diese Ermittlung werden gemessene Werte mindestens eines respiratorischen Parameters des Patienten verwendet, insbesondere Werte von dem oder mindestens einem Patienten-Sensor.
  Die Lungenzeitkonstante ist ein Maß für die Zeitdauer der alveolären Belüftung oder Entlüftung der Lunge. Sie hängt von der Elastizität und der Compliance der Lunge ab und ist umso größer, je größer die Elastizität und größer die Compliance ist. Beispielsweise ist die Lungenzeitkonstante das Produkt aus Elastizität und Compliance. Bevorzugt gibt die Lungenzeitkonstante die Zeit an, nach der beim Ausatmen zwei Drittel des Atemzugvolumens aus der Lunge ausgeströmt sind, wobei die Lunge als ein lineares pneumatisches System modelliert wird. Nach dem Dreifachen der Lungenzeitkonstante sind 95 % des Atemzugvolumens aus der Lunge ausgeströmt.
- Das Volumen eines Totraums wird ermittelt. Dieser Totraum befindet sich in der Fluidverbindung und dort zwischen einem Bereich der Lunge, der zum Gasaustausch mit dem Blut des Patienten geeignet ist, und dem Beatmungsgerät. Bevorzugt befindet der Totraum sich zwischen einem Messpunkt außerhalb des Patienten und dem zum Gasaustausch geeigneten Bereich der Lunge. Dieser Messpunkt befindet sich am Mund des Patienten oder in einem Bereich der Fluidverbindung, der zwischen dem Mund und dem Beatmungsgerät ist.
- Eine mandatorische Sollfrequenz für die mandatorische Beatmung des Patienten durch das Beatmungsgerät wird berechnet. Diese mandatorische Sollfrequenz wird abhängig von dem vorgegebenen angestrebten Volumenfluss, der ermittelten Lungenzeitkonstante und dem ermittelten Totraum-Volumen berechnet. Mit dieser mandatorischen Sollfrequenz lässt sich allein durch künstliche Beatmung der angestrebte Volumenfluss in die und aus der Lunge des Patienten erzielen, also ohne dass eine eigene Atmungsaktivität des Patienten zu diesem Volumenflusses beiträgt.
- Eine ideale Spontanatmungs-Frequenz für die eigene Atmungsaktivität (spontane Atmung und / oder stimulierte Atmung) wird berechnet, also für die Atmungsaktivität, die der Patient mit seine Atmungsmuskulatur erzielt. Mit dieser idealen Spontanatmungs-Frequenz vermag der Patient den angestrebten Volumenfluss zu erzielen, und zwar allein durch eigene Atmungsaktivität. Diese ideale Spontanatmungs-Frequenz wird abhängig von der ermittelten Lungenzeitkonstante und dem ermittelten Totraum-Volumen berechnet, bevorzugt unter Verwendung eines rechnerauswertbaren lungenmechanischen Modells für die eigene Atmungsaktivität eines Patienten.
- Ein Maß für die aktuelle tatsächliche Intensität der eigenen Atmungsaktivität des Patienten wird ermittelt. Dieses Maß gibt an, welchen absoluten oder relativen Beitrag die eigene Atmungsaktivität des Patienten zu der gesamten Belüftung und Entlüftung der Lunge des Patienten liefert. Falls die eigene Atmungsaktivität des Patienten von außen stimuliert wird, so trägt die stimulierte Atmungsaktivität ebenfalls zu diesem Maß für die aktuelle Intensität bei. Die künstliche Beatmung durch das Beatmungsgerät liefert den restlichen Beitrag zu der Belüftung und Entlüftung der Lunge.
- Die gesuchte Soll-Beatmungsfrequenz wird als gewichtetes Mittel der mandatorischen Sollfrequenz und der idealen Spontanatmungs-Frequenz berechnet. Welche Gewichtung, insbesondere welcher Gewichtsfaktor, in diesem gewichteten Mittel verwendet wird, hängt von dem ermittelten Maß für die aktuelle tatsächliche Intensität der eigenen Atmungsaktivität des Patienten ab. Erfindungsgemäß ist der Gewichtsfaktor, mit dem die ideale Spontanatmungs-Frequenz in die Soll-Beatmungsfrequenz eingeht, umso größer, je größer das Maß für die aktuelle tatsächliche Intensität der eigenen Atmungsaktivität des Patienten ist.

Das Beatmungsgerät vermag eine Abfolge von Beatmungshüben abhängig von der berechneten Soll-Beatmungsfrequenz durchzuführen. Insbesondere ist das Beatmungsgerät dazu ausgestaltet, eine Abfolge von Beatmungshüben mit der berechneten Soll-Beatmungsfrequenz durchzuführen.

Erfindungsgemäß wird automatisch eine Soll-Beatmungsfrequenz für das Beatmungsgerät berechnet. Daher vermeidet die Erfindung die Notwendigkeit, eine Soll-Beatmungsfrequenz für einen bestimmten Patienten von Hand festzulegen. Die Erfindung vermeidet auch die Notwendigkeit, eine Soll-Beatmungsfrequenz für einen Patienten abhängig von Frequenzen, mit denen in der Vergangenheit Patienten beatmet wurden, festzulegen. Vielmehr lässt sich die Soll-Beatmungsfrequenz an den aktuell zu beatmenden Patienten und insbesondere an dessen Lunge anpassen.

Möglich ist, dass das Bearbeitungsgerät mit dieser automatisch berechneten Soll-Beatmungsfrequenz betrieben wird, insbesondere Beatmungshübe gemäß dieser Soll-Beatmungsfrequenz ausführt. Bevorzugt ist es möglich, dass einem Benutzer des Beatmungsgeräts die automatisch berechnete Soll-Beatmungsfrequenz angezeigt wird und dass eine Eingabe des Benutzers erfasst und ausgewertet wird, insbesondere erfasst wird, ob der Benutzer die angezeigte Soll-Beatmungsfrequenz bestätigt oder mit einem anderen Wert überschreibt. Das Beatmungsgerät führt dann die Beatmungshübe abhängig von der erfindungsgemäß berechneten und vom Benutzer bestätigten Soll-Beatmungsfrequenz oder mit einer abweichenden Soll-Beatmungsfrequenz, die der Benutzer eingegeben hat, durch.

In einer Anwendung der Erfindung steuert die Signalverarbeitungseinheit das Beatmungsgerät an. Das Ziel bei dieser Ansteuerung ist, dass die tatsächliche Frequenz, mit welcher das Beatmungsgerät eine Abfolge von Beatmungshüben durchführt, gleich der Soll-Beatmungsfrequenz ist, welche die Signalverarbeitungseinheit erfindungsgemäß berechnet hat. Möglich ist auch, dass die erfindungsgemäß berechnete Soll-Beatmungsfrequenz an ein untergeordnetes Steuergerät des Beatmungsgeräts übermittelt wird und das Steuergerät ein Stellglied des Beatmungsgeräts so ansteuert, dass die tatsächliche Frequenz der Beatmungshübe gleich der erfindungsgemäß berechneten oder vom Benutzer eingegebenen Soll-Beatmungsfrequenz ist.

Erfindungsgemäß wird ein Maß für einen angestrebten Volumenfluss in die Lunge und aus der Lunge des Patienten vorgegeben, also das in die Lunge und das aus der Lunge strömende Volumen von Gas pro Zeiteinheit. Dieses Maß für den Volumenfluss ist in einer von einem Rechner auswertbaren Form im Datenspeicher abgespeichert. Bevorzugt ist der angestrebte Volumenfluss ein angestrebtes alveoläres oder proximales Minutenvolumen. Bevorzugt ist dieser angestrebte Volumenfluss der Fluss von Volumen in denjenigen Bereich der Lunge hinein und aus demjenigen Bereich der Lunge heraus, der für den Austausch von Gasen mit dem Blut des Patienten zur Verfügung steht. Die Gase sind insbesondere Atemluft, $O_2$ und $CO_2$ sowie optional mindestens ein Anästhesiemittel.

Erfindungsgemäß werden eine mandatorische Sollfrequenz für die mandatorische Beatmung des Patienten sowie eine ideale Spontanatmungs-Frequenz für die eigene Atmungsaktivität des Patienten berechnet. Unter einer mandatorischen Beatmung wird die künstliche Beatmung eines vollständig narkotisierten Patienten verstanden, also eines Patienten, der aktuell überhaupt nicht spontan atmet und dessen Atmungsmuskulatur auch nicht extern stimuliert wird. Eine künstliche Beatmung mit der mandatorischen Sollfrequenz vermag den vorgegebenen angestrebten Volumenflusses zu erzielen, ohne dass der Patient mit seiner eigenen Atmungsmuskulatur einen Beitrag zur Belüftung und Entlüftung der Lunge leistet. Mit der idealen Spontanatmungs-Frequenz für die eigene Atmungsaktivität vermag der Patient den angestrebten Volumenfluss ohne eine künstliche Beatmung zu erzeugen, wobei bevorzugt die ideale Spontanatmungs-Frequenz eine vorgegebene Zielfunktion optimiert. Bevorzugt ist die ideale Spontanatmungs-Frequenz diejenige Frequenz, mit welcher der Patient den angestrebten Volumenfluss mit der geringsten mechanischen Arbeit oder der geringsten mechanischen Leistung zu erzielen vermag, besonders bevorzugt die Frequenz, die zu einer minimalen mittleren mechanischen Leistung während der Inspiration führt (energieeffiziente Frequenz).

Die Soll-Beatmungsfrequenz wird erfindungsgemäß als gewichtete Zusammenfassung, insbesondere als gewichtetes arithmetisches Mittel, der mandatorischen Sollfrequenz und der idealen Spontanatmungs-Frequenz berechnet. Die Soll-Beatmungsfrequenz hängt also von einem Gewichtungsfaktor ab, wobei dieser Gewichtungsfaktor wiederum von einem ermittelten Maß für die aktuelle tatsächliche Intensität der eigenen Atmungsaktivität des Patienten abhängt. Je größer dieses Maß für die eigene Atmungsaktivität ist, je intensiver also aktuell die eigene Atmungsaktivität des Patienten ist, desto größer ist der Gewichtungsfaktor und damit der Beitrag der idealen Spontanatmungs-Frequenz zur berechneten Soll-Beatmungsfrequenz.

Das erfindungsgemäße Merkmal, dass eine gewichtete Zusammenfassung berechnet wird, trägt dem Umstand Rechnung, dass oft ein Patient während der künstlichen Beatmung nicht durchgehend vollständig narkotisiert ist, sondern wenigstens zeitweise eine eigene Atmungsaktivität ausführt, nämlich durch spontane Atmung und / oder extern stimulierte Atmung. Die künstliche Beatmung unterstützt diese eigene Atmungsaktivität des Patienten P. Der Patient atmet dann aufgrund einer Überlagerung von seiner eigenen Atmungsaktivität und der künstlichen Beatmung. In der Regel ist das Beatmungsgerät so ausgestaltet oder eingestellt, dass ein spontaner oder durch externe Stimulation ausgelöster Atemzug einen Beatmungshub des Beatmungsgeräts auslöst. Bei schwacher oder fehlender eigener Atmungsaktivität führt das Bearbeitungsgerät zusätzliche Beatmungshübe aus, also Beatmungshübe, die nicht durch die eigene Atmungsaktivität ausgelöst werden. Von der erfindungsgemäß berechneten Soll-Beatmungsfrequenz und der eigenen Atmungsfrequenz des Patienten hängt es ab, wie viele solcher zusätzlichen Beatmungshübe ausgeführt werden. Die Gefahr wird reduziert, dass zu wenige oder zu viele Beatmungshübe ausgeführt werden, der Patient also zu wenig oder zu viel Luft oder Luft zum falschen Zeitpunkt erhält.

Eine künstliche Beatmung kann eine mandatorische Beatmung (der Patient führt überhaupt keine eigene Atmungsaktivität aus) oder eine unterstützende Beatmung (künstliche Beatmung unterstützt die eigene Atmungsaktivität des Patienten) sein. Dank der Erfindung ist es nicht erforderlich, das Beatmungsgerät während der künstlichen Beatmung des Patienten zwischen mindestens zwei unterschiedlichen Modi umzuschalten, nämlich mindestens einem Modus für die mandatorische Beatmung und mindestens einem Modus für die unterstützende Beatmung. Ein solches Umschalten könnte zu einer abrupten Änderung der künstlichen Beatmung führen. Dank der Erfindung lässt sich die künstliche Beatmung vielmehr an die aktuelle Intensität der eigenen Atmungsaktivität anpassen, und zwar auch dann, wenn der angestrebte Volumenfluss gleich bleibt und die Intensität der eigenen Atmungsaktivität sich ändert. Die Beatmungsfrequenz lässt sich allmählich ändern und wird nicht schlagartig verändert.

Die erfindungsgemäß berechnete Soll-Beatmungsfrequenz lässt sich auch für die beiden Sonderfälle anwenden,

- dass der Patient vollständig narkotisiert ist und
- dass die Beatmungshübe ausschließlich von der eigenen Atmungsaktivität des Patienten ausgelöst werden.

Im ersten Fall wird also gar kein Beatmungshub von der eigenen Atmungsaktivität ausgelöst, im zweiten Fall jeder Beatmungshub. Diese beiden Sonderfälle brauchen dank der Erfindung nicht notwendigerweise detektiert zu werden, um die Soll-Beatmungsfrequenz festzulegen. Es ist ausreichend, das Maß für die eigene Atmungsaktivität zu detektieren. Die erfindungsgemäßen Vorrichtungen und das erfindungsgemäße Verfahren lassen sich gleichermaßen für die beiden gerade erwähnten Sonderfälle sowie für viele Zustände zwischen diesen beiden Sonderfällen anwenden, also insbesondere bei einer unterstützenden Beatmung bei einer relativ schwachen eigenen Atmungsaktivität des Patienten.

Die mandatorische Sollfrequenz wird erfindungsgemäß in Abhängigkeit von dem angestrebten Volumenfluss sowie von dem ermittelten Totraum-Volumen und der ermittelten Lungenzeitkonstante $\tau$ berechnet. Die ideale Spontanatmungs-Frequenz hängt mindestens von der Lungenzeitkonstante und dem Totraum-Volumen ab. Aus diesem Grund hängt die berechnete mandatorische Sollfrequenz von pneumatischen Eigenschaften der Lunge sowie von einem angestrebten Volumenfluss in die Lunge und aus der Lunge ab. Daher wird die mandatorische Sollfrequenz an die Lunge eines künstlich zu beatmenden Patienten angepasst. Möglich, aber dank der Erfindung nicht erforderlich ist, einen Standardwert oder einen durchschnittlichen Wert zu verwenden, der für mehrere Patienten gültig ist und daher nicht individuell auf den aktuell zu beatmenden Patienten zugeschnitten ist.

Weil erfindungsgemäß die Lungenzeitkonstante des Patienten ermittelt und verwendet wird, werden die beiden folgenden Gefahren bei der künstlichen Beatmung reduziert:

- Aufgrund einer zu hohen Beatmungsfrequenz erreicht zu wenig Atemluft den alveolären Lungenraum. Dieser unerwünschte Effekt tritt insbesondere bei einer Lunge mit einem hohen Lungenwiderstand (resistance) R und daher einer großen Lungenzeitkonstante $\tau$ auf. Insbesondere kann die unerwünschte Situation auftreten, dass bei der Exspiration zu wenig verbrauchte Atemluft aus der Lunge herausfließt. Die Lunge wird quasi immer weiter aufgeblasen. Dies tritt vor allem bei verstopften Atemwegen, insbesondere bei einer Raucherlunge, auf.
- Aufgrund einer zu niedrigen Beatmungsfrequenz wird die Lunge überdehnt, insbesondere weil das tatsächlich erzielte Tidalvolumen zu groß ist. Dieser unerwünschte Effekt tritt insbesondere bei einer Lunge mit einer geringen Elastizität (Compliance) C und daher einer kleinen Lungenzeitkonstante $\tau$ auf.

Die Erfindung ermöglicht es, erspart aber die Notwendigkeit, für die Berechnung der mandatorischen Sollfrequenz die Elastizität oder die Compliance oder den pneumatischen Widerstand der Lunge getrennt voneinander zu ermitteln. Eine solche Ermittlung ist in der Praxis nämlich häufig gar nicht möglich, mit relativ großen Fehlern behaftet und / oder belastet den Patienten erheblich, insbesondere wenn zur Ermittlung mindestens ein Manöver durchgeführt werden müsste. Dank der Erfindung ist es hingegen ausreichend, eine Lungenzeitkonstante zu ermitteln.

Auch die erfindungsgemäß berechnete ideale Spontanatmungs-Frequenz für die eigene Atmungsaktivität des Patienten hängt von dem Patienten ab, nämlich von dem ermittelten Totraum-Volumen, der zu einem erheblichen Teil im Körper des Patienten auftritt und von Patient zu Patient unterschiedlich sein kann, sowie von dem angestrebten Volumenfluss.

In einer Ausgestaltung führt die Signalverarbeitungseinheit die Berechnung der Soll-Beatmungsfrequenz für einen Patienten wiederholt durch, beispielsweise wenn seit der letzten Berechnung eine vorgegebene Zeitspanne verstrichen ist oder die eigene Atmungsaktivität des Patienten oder ein Zustand bei der künstlichen Beatmung des Patienten oder ein Zustand des Patienten sich verändert haben.

In einer bevorzugten Ausgestaltung berechnet die Signalverarbeitungseinheit die mandatorische Sollfrequenz abhängig von einer idealen mandatorischen Sollfrequenz sowie einer oberen Schranke für die mandatorische Sollfrequenz. Besonders bevorzugt ist die mandatorische Sollfrequenz gleich der idealen mandatorischen Sollfrequenz oder der oberen Schranke, je nachdem, welcher dieser beiden Werte kleiner ist.

Die ermittelte obere Schranke für die mandatorische Sollfrequenz hängt von der ermittelten Lungenzeitkonstante und bevorzugt zusätzlich von dem ermittelten Totraum-Volumen und / oder von dem angestrebten Volumenfluss ab. Die obere Schranke kann das Minimum von mehreren einzelnen oberen Schranken sein, wobei eine erste einzelne obere Schranke von der Lungenzeitkonstante und eine weitere einzelne obere Schranke von dem Totraum-Volumen und / oder vom Volumenfluss abhängt.

Diese Ausgestaltung ermöglicht es, verschiedene Randbedingungen zu berücksichtigen. Weil die mandatorische Sollfrequenz nicht größer ist als diese obere Schranke, wird die Gefahr weiter verringert, dass die Lunge durch eine zu hohe Frequenz oder durch ein zu großes erzieltes Tidalvolumen zu stark mechanisch belastet wird oder nicht genügend Atemluft erhält. Insbesondere wird vermieden, dass die künstliche Beatmung durch eine zu hohe Frequenz lediglich Atemluft in beide Richtungen durch den Totraum bewegt, ohne dass eine ausreichende Menge von Atemluft den zum Gastaustausch geeigneten Bereich der Lunge erreicht und ohne dass eine ausreichende Menge von verbrauchter Luft aus der Lunge abgeführt wird.

Falls aber stets die obere Schranke als die mandatorische Sollfrequenz verwendet würde, so bestünde die Gefahr, dass die Lunge durch die künstliche Beatmung stärker als erforderlich mechanisch belastet wird, also stärker als nötig, um den angestrebten Volumenfluss zu erzielen. Daher kann die mandatorische Sollfrequenz kleiner als die obere Schranke sein, nämlich insbesondere gleich einer berechneten idealen mandatorischen Sollfrequenz.

In einer Ausgestaltung berechnet die Signalverarbeitungseinheit die ideale mandatorische Sollfrequenz abhängig von einem vorgegebenen geforderten Einatmungsanteil. Der Einatmungsanteil gibt ein Maß dafür vor, welchen zeitlichen Anteil an einem Atemzyklus ein Einatmungsvorgang durchschnittlich aufweist. Möglich ist, das Verhältnis zwischen der durchschnittlichen Dauer eines Einatmungsvorgangs und der durchschnittlichen Dauer eines Ausatmungsvorgangs vorzugeben. Möglich ist auch, die durchschnittliche Dauer eines Einatmungsvorgangs oder die eines Ausatmungsvorgangs vorzugeben. Die Dauer des Einatmungsvorgangs bzw. Ausatmungsvorgangs sowie die Beatmungsfrequenz legen einen Einatmungsanteil fest.

In einer bevorzugten Ausgestaltung, wie die ideale mandatorische Sollfrequenz berechnet wird, wird ein Lungenmodell in einer von einem Rechner auswertbaren Form vorgegeben und im Datenspeicher abgespeichert. Dieses Lungenmodell beschreibt näherungsweise das pneumatische Verhalten der Lunge eines Menschen und damit auch der Lunge des künstlich beatmeten Patienten. Bevorzugt enthält dieses Lungenmodell mindestens einen Modell-Parameter, insbesondere die Lungenzeitkonstante und / oder das Totraum-Volumen. Der jeweilige Wert des oder jedes Modell-Parameters kann von Patient zu Patient variieren. Unter Verwendung dieses Lungenmodells lässt sich automatisch vorhersagen, welche resistive Leistung und welche elastische Leistung bei einer bestimmten tatsächlichen Beatmungsfrequenz auf die Lunge einwirken. Die ideale mandatorische Sollfrequenz wird unter Verwendung des vorgegebenen Lungenmodells und des vorgegebenen Einatmungsanteils berechnet.

Unter Verwendung des vorgegebenen Lungenmodells und des geforderten Einatmungsanteils berechnet die Signalverarbeitungseinheit eine resistive Leistung und eine elastische Leistung. Die resistive Leistung ist die Arbeit pro Zeiteinheit, die bei einem Einatmungsvorgang aufzuwenden ist, um den pneumatischen Widerstand der Lunge zu überwinden. Die elastische Leistung ist die Arbeit pro Zeiteinheit, die aufzuwenden ist, um die Lunge zu dehnen. Um die ideale mandatorische Sollfrequenz zu berechnen, wird automatisch vorhergesagt, welche Frequenz bei dem Lungenmodell und bei dem Einatmungsanteil zu welcher resistiven Leistung und zu welcher elastischen Leistung führt.

Bevorzugt sagt die Signalverarbeitungseinheit für mindestens eine mögliche ideale mandatorische Sollfrequenz, bevorzugt für mehrere mögliche ideale mandatorische Sollfrequenzen, die jeweils resultierende resistive Leistung und die jeweils resultierende elastische Leistung vorher. Diese vorhergesagten resultierenden Leistungen verwendet die Signalverarbeitungseinheit, um die ideale mandatorische Sollfrequenz zu berechnen.

In einer besonders bevorzugten Ausgestaltung legt die Signalverarbeitungseinheit diese ideale mandatorische Sollfrequenz so fest, dass die bewirkte resistive Leistung sich von der bewirkten elastischen Leistung um höchstens einen vorgegebenen Leistungsfaktor unterscheidet, wobei der Leistungsfaktor bevorzugt der Quotient aus den beiden bewirkten mechanischen Leistungen ist. Der Leistungsfaktor lässt sich so klein wählen, dass die resistive Leistung größer, aber nur wenig größer ist als die elastische Leistung, bevorzugt höchstens 20 % größer. Der Leistungsfaktor lässt sich vorab vorgegeben und wird bevorzugt im Datenspeicher abgespeichert. Diese Ausgestaltung führt in vielen Fällen zu einer relativ geringen mechanischen Belastung der Lunge, insbesondere weil jedes erzielte Tidalvolumen relativ klein ist. Dennoch bewirkt die Ausgestaltung einen ausreichend großen Volumenfluss in den und aus dem zum Gastaustausch geeigneten Lungen-Bereich, insbesondere den alveolären Lungenraum.

Die vorteilhafte Ausgestaltung mit dem Leistungsfaktor erspart die Notwendigkeit, eine Funktion zu minimieren, welche die gesamte Arbeit oder die gesamte Leistung beschreibt, welche bei der künstlichen Beatmung auf die Lunge einwirkt. Insbesondere wird die Notwendigkeit erspart, zur Laufzeit, also während der künstlichen Beatmung, eine solche Funktion zu minimieren. Die Minimierung einer Funktion ist zeitaufwendig und erfordert eine relativ große Rechenkapazität. Falls bei der Minimierung ein iteratives Verfahren angewendet wird und falls zur Laufzeit das Verfahren abgebrochen wird, wenn ein Abbruchkriterium erfüllt ist, so kann die Minimierung zu einem ungünstigen Ergebnis führen. Ohne ein geeignetes Abbruchkriterium kann die Minimierung zu lange dauern. Die vorteilhafte Ausgestaltung vermeidet die Nachteile einer solchen Minimierung zur Laufzeit.

In einer bevorzugten Realisierung führt die Signalverarbeitungseinheit eine Initialisierungsphase und für die künstliche Beatmung des Patienten eine nachfolgende Nutzungsphase durch. In der Initialisierungsphase berechnet die Signalverarbeitungseinheit eine Konstante. Um die Konstante zu berechnen, verwendet die Signalverarbeitungseinheit das Lungenmodell und bevorzugt den optionalen vorgegebenen Leistungsfaktor. In der nachfolgenden Nutzungsphase berechnet die Signalverarbeitungseinheit die ideale mandatorische Sollfrequenz. Hierfür verwendet die Signalverarbeitungseinheit den vorgegebenen Einatmungsanteil, die für den Patienten ermittelte Lungenzeitkonstante sowie die Konstante, die in der Initialisierungsphase berechnet worden ist. Diese Ausgestaltung führt häufig zu einem geringeren Rechenaufwand in der Nutzungsphase. Möglich ist, dass die Signalverarbeitungseinheit in der Nutzungsphase erneut die Konstante berechnet, beispielsweise aufgrund einer geänderten Vorgabe für die künstliche Beatmung. Möglich ist, die in der Initialisierungsphase gewonnene Konstante in mehreren Nutzungsphasen, auch für mehrere Patienten zu verwenden. Möglich ist, die Ergebnisse der Initialisierungsphase für mehrere Patienten zu verwenden, während die Nutzungsphase und die Berechnungen in dieser Nutzungsphase für jeden Patienten erneut durchgeführt werden und daher zu Ergebnissen führen, die spezifisch für den Patienten sind.

In einer Fortbildung dieser Realisierung berechnet eine erste Signalverarbeitungseinheit in der Initialisierungsphase die Konstante. Eine zweite Signalverarbeitungseinheit berechnet in der Nutzungsphase die ideale mandatorische Sollfrequenz und hieraus die Soll-Beatmungsfrequenz. Die erste Signalverarbeitungseinheit ist nicht notwendigerweise ein Bestandteil des Beatm ungsgeräts.

Gemäß einer gerade beschriebenen Ausgestaltung berechnet die Signalverarbeitungseinheit eine ideale mandatorische Sollfrequenz abhängig von einem vorgegebenen geforderten Einatmungsanteil. Bevorzugt wird die ideale mandatorische Sollfrequenz dergestalt berechnet, dass sie umso größer ist, je größer der vorgegebene geforderte Einatmungsanteil ist.

Erfindungsgemäß berechnet die Signalverarbeitungseinheit eine mandatorische Sollfrequenz für die mandatorische Beatmung des Patienten sowie eine ideale Spontanatmungs-Frequenz für die eigene Atmungsaktivität des Patienten. Bevorzugt berechnet die Signalverarbeitungseinheit die mandatorische Sollfrequenz dergestalt, dass die mandatorische Sollfrequenz größer oder gleich der idealen Spontanatmungs-Frequenz ist. Dann ist auch die festgelegte Soll-Beatmungsfrequenz mindestens so groß wie die ideale Spontanatmungs-Frequenz. Dadurch wird eine zu kleine Beatmungsfrequenz vermieden. Insbesondere wird sichergestellt, dass mindestens jeder spontane oder extern stimulierte Atemzug des Patienten einen Beatmungshub des Beatmungsgeräts auslöst. Möglich ist, dass das Bearbeitungsgerät mindestens einen Beatmungshub ausführt, der nicht von einem spontanen oder stimulierten Atemzug des Patienten ausgelöst wird, insbesondere wenn die eigene Atmungsaktivität des Patienten relativ schwach ist.

Erfindungsgemäß berechnet die Signalverarbeitungseinheit eine Soll-Beatmungsfrequenz. Ein Betriebsparameter, der häufig an einem Beatmungsgerät eingestellt wird, ist das geforderte Tidalvolumen. Dieses geforderte Tidalvolumen ist ein Maß für dasjenige Volumen, welches das Beatmungsgerät bei einem einzigen Beatmungshub in die Fluidverbindung einspeisen soll. In einer Ausgestaltung der Erfindung berechnet die Signalverarbeitungseinheit einen Wert für das geforderte Tidalvolumen abhängig von

- dem angestrebten und im Datenspeicher abgespeicherten oder anderweitig vorgegebenen Volumenfluss,
- dem ermittelten Totraum-Volumen und
- der erfindungsgemäß berechnete Soll-Beatmungsfrequenz.

Das Beatmungsgerät wird mit dem Ziel angesteuert, dass bei mindestens einem Beatmungshub das tatsächlich erzielte Tidalvolumen gleich dem berechneten Soll-Tidalvolumen ist. Bevorzugt soll bei jedem Beatmungshub während der künstlichen Beatmung das tatsächliche Tidalvolumen gleich dem Soll-Tidalvolumen sein, zumindest solange, bis ein neues Soll-Tidalvolumen berechnet und vorgegeben wird. Bevorzugt wird ein Stellglied des Beatmungsgeräts entsprechend dem Soll-Tidalvolumen angesteuert. Bevorzugt wird eine Regelung durchgeführt, bei der das Ziel ist, dass das tatsächliche Tidalvolumen gleich dem Soll-Tidalvolumen ist. Bevorzugt soll bei jedem Beatmungshub, der nach der Berechnung der Soll-Beatmungsfrequenz und bis zur Beendigung der künstlichen Beatmung oder bis zu einer erneuten Berechnung der Soll-Beatmungsfrequenz durchgeführt wird, das erzielte Tidalvolumen gleich dem berechneten Soll-Tidalvolumen sein. Bevorzugt führt die erfindungsgemäße Signalverarbeitungseinheit diese Regelung durch. Möglich ist auch, dass ein untergeordnete Steuergerät des Beatmungsgeräts diese Regelung durchführt.

Bei einem Beatmungshub speist das Beatmungsgerät Fluid in die Fluidverbindung ein. Der Druck an einem Messpunkt in der Fluidverbindung steigt, bis der Druck einen Maximalwert erreicht hat. In einer Ausgestaltung wird der tatsächlich erzielte Druck geregelt oder gesteuert, wobei ein zeitlicher Verlauf des geforderten Drucks vorgegeben wird und wobei das Beatmungsgerät mit dem Ziel angesteuert wird, dass der tatsächliche zeitliche Verlauf des Drucks an dem Messpunkt gleich dem vorgegebenen zeitlichen Verlauf ist. Bevorzugt wird eine Regelung mit dem Ziel durchgeführt, dass der tatsächliche Druckverlauf gleich dem Soll-Druckverlauf ist.

Falls der vorgegebene zeitliche Verlauf des Drucks schlagartig den Maximalwert erreicht, so besteht die Gefahr, dass die Lunge des Patienten geschädigt wird. Daher berechnet die Signalverarbeitungseinheit bevorzugt für mindestens einen Beatmungshub, bevorzugt für jedem Beatmungshub, eine geforderte Rampenzeit. Diese Soll-Rampenzeit legt im geforderten zeitlichen Verlauf des Drucks die geforderte Zeitspanne fest, die zwischen

- dem Beginn des Beatmungshubs und
- dem Zeitpunkt, an dem im Verlaufe dieses Beatmungshubs der Maximalwert des Drucks erreicht wird,

verstreichen soll. Die Regelung oder Steuerung des tatsächlichen zeitlichen Druckverlaufs wird mit dem Regelungsziel durchgeführt, dass die tatsächlich erzielte Rampenzeit gleich der berechneten Soll-Rampenzeit ist. Bevorzugt wird diese Soll-Rampenzeit für die durchgeführten Beatmungshübe verwendet, bis erneut eine Soll-Beatmungsfrequenz berechnet wird oder bis die künstliche Beatmung beendet wird. Bevorzugt regelt oder steuert die erfindungsgemäße Signalverarbeitungseinheit die tatsächliche Rampenzeit.

Bevorzugt berechnet die Signalverarbeitungseinheit die Soll-Rampenzeit abhängig von der ermittelten Lungenzeitkonstante des Patienten. Möglich ist auch, dass die Signalverarbeitungseinheit zusätzlich zu der oder anstelle der Lungenzeitkonstante ein Idealgewicht (ideal body weight) des Patienten verwendet. Dieses Idealgewicht lässt sich aus leicht messbaren Parametern des Patienten herleiten.

Erfindungsgemäß wird das Totraum-Volumen in der Fluidverbindung ermittelt. Der größte Teil dieses Totraums tritt im Körper des Patienten auf, und zwar zwischen dem Mund und denjenigen Bereichen der Lunge, die zum Gasaustausch mit Luft geeignet sind. In einer Ausgestaltung wird der Fluss von Volumen während eines Ausatmungsvorgangs gemessen. Gemessen wird also, wie groß der Volumenfluss des Gases, das aus der Lunge des Patienten fließt, ist. Außerdem wird die Zeitspanne gemessen, die seit dem Beginn des Ausatemzugs verstreicht, bis die ausgeatmete Luft $CO_2$ enthält. Aus diesen beiden gemessenen Werten wird näherungsweise das gesuchte Totraum-Volumen im Körper des Patienten hergeleitet. Der Hintergrund: Solange die ausgeatmete Luft keinen wesentlichen Anteil an $CO_2$ enthält, ist diese ausgeatmete Luft aus dem Totraum geflossen und nicht aus dem zum Austausch geeigneten Bereichen der Lunge. Möglich ist auch, für das Totraum-Volumen einen Standardwert oder eine grobe Abschätzung abhängig von einem Idealgewicht oder dem tatsächlichen Gewicht des Patienten zu verwenden. Andere Ausgestaltungen, um das Totraum-Volumen zu messen, sind ebenfalls möglich.

Die Erfindung betrifft weiterhin ein Computerprogramm, welches sich auf einer Signalverarbeitungseinheit ausführen lässt. Das Computerprogramm wird beispielsweise auf einem mobilen Datenträger oder einen Datenträger eines Rechners abgespeichert.

Wenn die Signalverarbeitungseinheit das Computerprogramm ausführt, so wird bewirkt, dass die Signalverarbeitungseinheit erfindungsgemäß die Soll-Beatmungsfrequenz berechnet und bei dieser Berechnung das erfindungsgemäße Verfahren zur Berechnung der Soll-Beatmungsfrequenz durchführt. Diese Wirkung tritt ein, wenn die Signalverarbeitungseinheit Messwerte von mindestens einem Patienten-Sensor empfängt, optional von mehreren Patienten-Sensoren, wobei der oder jeder Patienten-Sensor jeweils mindestens einen respiratorischen Parameter des Patienten zu messen vermag.

Bevorzugt ist die Signalverarbeitungseinheit eine universelle Verarbeitungseinheit. Dadurch, dass die Signalverarbeitungseinheit wenigstens zeitweise Lesezugriff auf das Computerprogramm hat, wird sie zu einer erfindungsgemäßen Signalverarbeitungseinheit.

Im Folgenden wird die Erfindung anhand eines Ausführungsbeispiels beschrieben. Hierbei zeigt

Figur 1 beispielhaft einen künstlich beatmeten Patienten, ein Beatmungsgerät sowie mehrere Sensoren;

Figur 2 beispielhaft die resistive und die elastische Leistung in Abhängigkeit von der tatsächlichen Beatmungsfrequenz;

Figur 3 beispielhaft den jeweiligen zeitlichen Verlauf von Druck, Volumenfluss, resistiver Arbeit und elastischer Arbeit bei einem relativ geringen Lungenwiderstand;

Figur 4 beispielhaft den jeweiligen zeitlichen Verlauf von Druck, Volumenfluss, resistiver Arbeit und elastischer Arbeit bei einem relativ hohen Lungenwiderstand und relativ hoher Beatmungsfrequenz;

Figur 5 beispielhaft den jeweiligen zeitlichen Verlauf von Druck, Volumenfluss, resistiver Arbeit und elastischer Arbeit bei einem relativ geringen Lungenwiderstand, wobei die Rampenzeit zu Beginn eines Beatmungshubs berücksichtigt ist;

Figur 6 wie das Maß für die Intensität der eigenen Atmungsaktivität des Patienten abhängig von der geleisteten Atemarbeit ermittelt wird;

Figur 7 wie das Maß für die Intensität der eigenen Atmungsaktivität abhängig von dem auftretenden Druck-Zeit-Produkt ermittelt wird;

Figur 8 die gesamte mechanische Leistung sowie die Leistung aufgrund der eigenen Atmungsaktivität in Abhängigkeit von der Beatmungsfrequenz;

Im Ausführungsbeispiel wird die Erfindung eingesetzt, um einen Patienten künstlich zu beatmen. Zwischen der Lunge des Patienten und einem Beatmungsgerät ist eine Fluidverbindung hergestellt. Durch diese Fluidverbindung wird dem Patienten Atemluft oder ein sonstiges Gasgemisch zugeführt. Optional ist dieses Gasgemisch mit mindestens einem Narkosemittel versetzt, so dass der Patient teilweise oder vollständig narkotisiert wird. Optional gehört die Fluidverbindung zu einem Beatmungskreislauf, der die ausgeatmete Luft wieder aufnimmt, insbesondere wenn ein Narkosemittel zugesetzt ist. Im Falle eines Beatmungskreislaufs filtert das Beatmungsgerät aus der ausgeatmeten Atemluft Kohlendioxid ($CO_2$) heraus, optional auch das oder jedes Narkosemittel.

Figur 1 zeigt beispielhaft einen Patienten P, der künstlich beatmet wird. Schematisch sind die Lunge Lu, die Speiseröhre Sp, der Magen Ma und das Zwerchfell Zw des Patienten P dargestellt. Ein Beatmungsgerät 1 mit einer Anzeige- und Bedieneinheit 12 sowie mit einer datenverarbeitenden Signalverarbeitungseinheit 10 beatmet den Patienten P künstlich. Die Signalverarbeitungseinheit 10 umfasst mindestens einen Prozessor sowie einen Datenspeicher 11. Oder der Prozessor hat wenigstens zweitweise Lesezugriff auf einen Datenspeicher 11. Die Beatmungsschläuche zwischen dem Beatmungsgerät 1 und dem Patienten P sind nicht dargestellt. Im Mund des Patienten P befindet sich während der Beatmung ein flexibles Anschlussstück 4. In einer Ausgestaltung ist in die Speiseröhre des Patienten P ein flexibler Messkatheter 6 gelegt, wobei der Messkatheter 6 in dem Anschlussstück 4 beginnt.

Verschiedene Sensoren messen diverse pneumatische Vitalparameter des Patienten P und / oder Parameter der Fluidverbindung zwischen dem Beatmungsgerät 1 und der Lunge Lu des Patienten P. Um die Erfindung auszuführen, brauchen nicht notwendigerweise alle diese Sensoren vorhanden zu sein. Folgende Sensoren werden in Figur 1 gezeigt:

- Eine pneumatische Sensoreinheit 2 umfasst einen Messwertaufnehmer 2.1 umfassend eine Öffnung, die in der Nähe des Mundes des Patienten P angeordnet ist und Luft aus der Fluidverbindung abgreift. Die abgegriffene Luft wird über einen nicht gezeigten Schlauch an einen Drucksensor 2.2 übermittelt, der ein Maß für den Atemwegsdruck $P_{aw}$ (pressure in airway) in der Fluidverbindung und optional ein Maß für den Volumenfluss Vol' misst. In einer Ausgestaltung ist der Messwertaufnehmer 2.1 in oder an einem Y-Stück nahe dem Anschlussstück 4 angeordnet, also nahe am Mund des Patienten P.
- Optional misst ein Sensor 15 in oder am Beatmungsgerät 1 ein Maß für das Volumen Vol' pro Zeiteinheit des Flusses von Atemluft vom Beatmungsgerät 1 zum Patienten P (z.B. inspiratorisches Minutenvolumen) und / oder zurück vom Patienten P zum Beatmungsgerät 1 (z.B. exspiratorisches Minutenvolumen).
- Eine Sonde 3 in der Speiseröhre Sp des Patienten P, bevorzugt umfassend einen Messballon, misst ein Maß für den zeitlich veränderlichen pneumatischen Druck $P_{es}$ (pressure in esophagus) in der Speiseröhre Sp. Die Sonde 3 steht über den Messkatheter 6 in einer Fluidverbindung mit dem Anschlussstück 4 oder ist ein Bestandteil des Messkatheters 6.
- Ein weiterer Messballon der Sonde 3 oder eine optionale gastrale Sonde 7 in Form eines Messballons, der in den Magen Ma gelegt ist, messen ein Maß für den gastralen Druck $P_{ga}$ im Magen Ma.
- Auf der Brust des Patienten P sind mehrere Messelektroden befestigt. Figur 1 zeigt beispielhaft ein herznahes Paar 5.1.1, 5.1.2 von Messelektroden und ein zwerchfellnahes Paar 5.2.1, 5.2.2 von Messelektroden. Mithilfe dieser optionalen Messelektroden 5.1.1, ..., 5.2.2 sowie einer nicht gezeigten Messelektrode für elektrische Masse werden ein Elektrokardiogramm (EKG) und / oder ein Elektromyogramm (EMG) des Patienten P erzeugt.

Die Signalverarbeitungseinheit 10 vermag automatisch zu ermitteln, wann Luft oder ein sonstiges Gasgemisch in das Atemsystem des Patienten P strömt und wann das Gasgemisch wieder aus dem Atemsystem strömt, vermag also jede Inspirationsphase und jeden Exspirationsphase zu detektieren. Hierfür verwendet die Signalverarbeitungseinheit 10 Messwerte von den Sensoren 2, 3, 7 und 15 und optional von den Messelektroden 5.1.1 bis 5.2.2.

In einer Anwendung ist der Patient P vollständig narkotisiert und führt keine eigene Atmungsaktivität aus, führt also selber überhaupt keine oder höchstens einzelne spontane Atemzüge durch, und seine Atmungsmuskulatur wird auch nicht extern stimuliert. Bei dieser Anwendung führt das Beatmungsgerät 1 eine mandatorische Beatmung durch. In einer anderen Ausgestaltung atmet der Patient P selber wenigstens zeitweise spontan, und optional wird seine Atmungsmuskulatur extern stimuliert. Das Beatmungsgerät 1 unterstützt die eigene Atmungsaktivität des Patienten P, indem das Beatmungsgerät 1 ein Gasgemisch in die Lunge Lu fördert. Möglich sind auch zeitliche Übergänge zwischen diesen beiden Beatmungsmodi.

In beiden Modi der Beatmung führt das Beatmungsgerät 1 eine Abfolge von Beatmungshüben durch. Bei jedem Beatmungshub speist das Beatmungsgerät 1 in die Fluidverbindung Atemluft oder ein sonstiges Gasgemisch ein. Die künstliche Beatmung wird durch mehrere pneumatische Betriebsparameter gekennzeichnet, u.a.

- einen geforderten zeitlichen Verlauf des Drucks, den das Beatmungsgerät 1 bei den Beatmungshüben erzeugt,
- einen geforderten zeitlichen Verlauf des Volumens des Gasgemisches, die bei den Beatmungshüben in die Fluidverbindung eingespeist wird,
- eine Frequenz der Beatmungshübe oder auch
- die Amplitude, also den maximalen Druckunterschied bei den Beatmungshüben.

Ein Mensch nimmt bei der spontanen oder stimulierte Atmung mit der eigenen Atmungsmuskulatur oder bei der künstlichen Beatmung, die mit einer Frequenz $f_{ist}$ durchgeführt wird, Luft oder ein sonstiges Gasgemisch auf, wobei dieses Gasgemisch Sauerstoff und optional mindestens ein Narkosemittel, das mit einem Atemzug aufgenommen wird, wird als (tatsächliches) Atemzugvolumen oder Tidalvolumen $Vol_{Tid,ist}$ bezeichnet. Der alveoläre Lungenraum steht für den Austausch von Sauerstoff und Kohlendioxid zwischen dem zugeführten Gasgemisch und dem Blut zur Verfügung. Das Volumen von Gas, das tatsächlich den alveolären Lungenraum durchströmt, wird im Folgenden als "erzieltes alveoläres Lungenvolumen" bezeichnet. Der Vorgang, dass Gas den alveolären Lungenraum durchströmt, wird oft als "alveoläre Ventilation" bezeichnet. Im oberen und mittleren Atemweg des Patienten P, in einem Bereich der Lunge Lu, in dem kein Gas mit dem Blut ausgetauscht wird, sowie - bei einer künstlichen Beatmung - in demjenigen Bereich der Fluidverbindung, der in beiden Richtungen durchströmt wird, tritt ein Totraum mit dem gesamten Volumen $V_D$ auf, wobei dieser Totraum zwar in beiden Richtungen von Gas durchströmt wird, aber nicht für den Austausch von Sauerstoff und Kohlendioxid zur Verfügung steht. Als tatsächliches alveoläres Minutenvolumen $V'_{A,ist}$ wird das erzielte alveoläre Lungenvolumen pro Zeiteinheit, im Ausführungsbeispiel in [l/min], bezeichnet. Dann gilt

$$(1) \qquad V'_{A,ist} = f_{ist} * (Vol_{Tid,ist} - V_D).$$

Das tatsächliche alveoläre Minutenvolumen $V'_{A,ist}$ korreliert mit dem Volumenfluss Vol', der an einem Messpunkt gemessen wird, beispielsweise mittels des Messwertaufnehmers 2.1 und des Drucksensors 2.2. In der Fluidverbindung zwischen dem Beatmungsgerät 1 und der Lunge Lu des Patienten P tritt ein Totraum (deadspace) auf, in dem kein Gasaustausch stattfindet. Nur derjenige geräteseitige Totraum ist zu berücksichtigen, der sich zwischen dem Messpunkt 2.1 und dem Patienten P befindet und in beiden Richtungen von Gas durchströmt wird. In vielen Fällen hat dieser geräteseitige Totraum ein vernachlässigbar kleines Volumen $V_{D,Ger}$, sodass als gesamtes Volumen $V_D$ nur das Volumen $V_{D,Pat}$ des patientenseitigen Totraums verwendet wird

Im Ausführungsbeispiel wird ein angestrebtes alveoläres Minutenvolumen vorgegeben, das ist das Volumen pro Zeiteinheit des Gasgemisches, welches den alveolären Lungenraum durchspült und daher für den Austausch von Sauerstoff und Kohlendioxid mit dem Blut des Patienten P zur Verfügung steht. Dieses Minutenvolumen wird beispielsweise in Litern pro Minute angegeben und mit $V'_{A,req}$ bezeichnet. Dieses angestrebte alveoläre Minutenvolumen $V'_{A,req}$ wird vorgegeben oder automatisch eingestellt. Beispielsweise gibt ein Benutzer das angestrebte alveoläre Minutenvolumen $V'_{A,req}$ mithilfe der Anzeige- und Bedieneinheit 12 am Beatmungsgerät 1 ein. In einer Ausgestaltung wird das angestrebte alveoläre Minutenvolumen $V'_{A,req}$ automatisch oder von einem Benutzer hergeleitet, beispielsweise abhängig von einem geforderten zeitlichen Verlauf des $CO_2$-Gehalts in der ausgeatmeten Atemluft. Ein solcher zeitlicher Verlauf wird auch als Kapnogramm oder Kapnographiekurve bezeichnet und lässt sich mithilfe eines $CO_2$-Sensors messen.

Anstelle eines angestrebten alveolären Minutenvolumens $V'_{A,req}$ lässt sich auch ein angestrebtes proximales Minutenvolumen vorgeben. Das tatsächliche proximale Minutenvolumen $V'_{P,ist}$ ist der Volumenfluss am Mund des Patienten P, insbesondere der Volumenfluss an einen Tubuskonnektor.

Der Volumenfluss teilt sich auf den alveolären Luftstrom und den Fluss durch den Totraum auf. Das Volumen $V_{D,Ger}$ des geräteseitigen Totraums ist bei einem Messpunkt am oder nahe dem Patienten P vernachlässigbar klein. Das tatsächliche proximale Minutenvolumen $V'_{P,ist}$ hängt wie folgt mit dem tatsächlichen alveolären Minutenvolumen $V'_{A,ist}$ zusammen:

$$(2) \qquad V'_{P,ist} = V'_{A,ist} + V_D * f_{ist} = V'_{A,ist} + (V_{D,Pat} + V_{D,Ger}) * f_{ist}.$$

Hierbei sind $V_D$ das gesamte Volumen des Totraumsund $f_{ist}$ die tatsächliche Beatmungsfrequenz des Beatmungsgeräts 1. Im Ausführungsbeispiel ist das gesamte Totraum-Volumen $V_D$ die Summe aus dem Volumen $V_{D,Pat}$ des patientenseitigen Totraums und dem Volumen $V_{D,Ger}$ des geräteseitigen Totraums. Dieses geräteseitige Volumen $V_{D,Ger}$ ist in der Regel aufgrund der Konstruktion des Beatmungsgeräts 1 und der Konstruktion der Schläuche der Fluidverbindung bekannt und lässt sich außerdem in vielen Fällen vernachlässigen.

Beispielsweise werden das angestrebte alveoläre Minutenvolumen $V'_{A,req}$ oder das angestrebte proximale Minutenvolumen $V'_{P,req}$ so vorgegeben, dass angestrebte Werte für die Konzentration oder für den Partialdruck von bestimmten Gasen im Blut des Patienten P möglichst gut erreicht werden. Das Minutenvolumen $V'_{A,req}$, $V'_{P,req}$ lässt sich von einem Anwender oder von einem übergeordneten Automatisierungssystem vorgeben oder ist fest in dem Datenspeicher 11 des Beatmungsgeräts 1 abgespeichert.

Erfindungsgemäß berechnet die Signalverarbeitungseinheit 10 automatisch eine Soll-Beatmungsfrequenz $f_{set}$ des Beatmungsgeräts 1, und zwar in Abhängigkeit von einem angestrebten alveolären Minutenvolumen $V'_{A,req}$. Bevorzugt empfängt die Signalverarbeitungseinheit 10 Messwerte von den Sensoren 2, 3, 5.1.1, ..., 5.2.2, 7 und führt abhängig von den empfangenen Messwerten die erforderlichen Berechnungsschritte durch. In einer Ausgestaltung wird diese Berechnung in festen Zeitabschnitten oder bei einem auslösenden Ereignis, beispielsweise bei einer Zustandsveränderung des Patienten P, wiederholt durchgeführt.

Die berechnete Soll-Beatmungsfrequenz $f_{set}$ wird im Ausführungsbeispiel in einer untergeordneten Regelung für den Druck verwendet. Der tatsächlich erzielte Druck ist die Regelgröße und wird gemessen, bevorzugt als der erzielte Atemwegsdruck $P_{aw}$. Vorgegeben wird als Führungsgröße ein gewünschter zeitlicher Verlauf $P_{set}$ des Drucks. Die berechnete Soll-Beatmungsfrequenz $f_{set}$ legt die Frequenz der Druckanstiege und somit der Beatmungshübe in diesem gewünschten zeitlichen Verlauf von $P_{set}$ fest.

Ein nicht gezeigtes untergeordnetes Steuergerät bewirkt, dass ein Stellglied des Beatmungsgeräts 1 angesteuert wird, wobei dieses Stellglied die Beatmungshübe erzeugt. In der Regel ist die tatsächlich erzielte Beatmungsfrequenz $f_{ist}$ gleich der automatisch berechneten Soll-Beatmungsfrequenz $f_{set}$. Zur Verdeutlichung werden im Folgenden trotzdem unterschiedliche Bezeichnungen verwendet.

Bei einer mandatorischen Beatmung ist eine Sollfrequenz vorgegeben, während bei einer unterstützenden Beatmung die Frequenz von der eigenen Atmungsaktivität des Patienten P abhängen und daher variieren kann. Im Folgenden wird zunächst beschrieben, wie die Soll-Beatmungsfrequenz für eine mandatorische Beatmung berechnet wird. Diese wird im Folgenden mit $f_{set,mand}$ bezeichnet.

Der Austausch von Sauerstoff und Kohlendioxid mit der Lunge Lu des Patienten P während der Atmung und / oder der Beatmung findet nur in dem alveolären Lungenraum statt. Zu der Fluidverbindung zwischen dem patientenseitigen Anschluss des Beatmungsgeräts 1 und der Lunge Lu gehören aber zusätzlich der obere und mittlere Atemweg des Patienten P, insbesondere dessen Luftröhre, sowie ein optionaler Tubus, Schläuche und / oder sonstige Fluidführungseinheiten, die den Patienten P mit dem Beatmungsgerät 1 verbinden, außerdem optional Messkammern. Die gerade genannten Bestandteile werden bei jedem Beatmungshub mit der Atemluft oder dem sonstigen Gasgemisch durchströmt, tragen aber nicht zum Austausch von Sauerstoff oder Kohlendioxid bei und werden daher als Totraum (deadspace) bezeichnet. Dieser Totraum setzt sich aus einem patientenseitigen Totraum und einen geräteseitigen Totraum zusammen. Das gesamte Totraum-Volumen $V_D$ ist die Summe aus dem Volumen $V_{D,Pat}$ des patientenseitigen Totraums und dem Volumen $V_{D,Ger}$ des geräteseitigen Totraums.

Aus diesem Totraum-Volumen $V_D$ resultiert eine Randbedingung für die Berechnung der mandatorische Soll-Beatmungsfrequenz $f_{set,mand}$. Die Beatmungsfrequenz $f_{ist}$ muss nämlich so niedrig sein, dass genügend Atemluft den Totraum durchströmt und das alveoläre Lungenvolumen $V_A$ erreicht.

Bei einem Beatmungshub speist das Beatmungsgerät 1 in die Fluidverbindung Atemluft oder ein sonstiges Gasgemisch mit Sauerstoff ein. Bei einem Atemzug nimmt der Patient P das Gasgemisch auf. Das Volumen des eingespeisten oder des aufgenommenen Gasgemisches wird als Tidalvolumen $Vol_{Tid,ist}$ bezeichnet. Idealerweise wird die Lunge Lu bei jedem Beatmungshub um dieses Tidalvolumen $Vol_{Tid,ist}$ vergrößert. In einer Ausgestaltung wird gefordert, dass das Tidalvolumen $Vol_{Tid,ist}$ mindestens doppelt so groß sein soll wie das Totraum-Volumen $V_D$. Hieraus und aus dem Zusammenhang (1) resultiert die Randbedingung

$$(3) \qquad f_{set,mand} <= V'_{A,req} / V_D.$$

In einer Ausgestaltung des Schritts, das patientenseitige Totraum-Volumen $V_{D,Pat}$ zu ermitteln, wird automatisch detektiert, wann der Patient P ausatmet. Beim Ausatmen strömt Luft aus dem oberen und mittleren Atemweg des Patienten P aus dem Körper und außerdem Luft aus der Lunge Lu in den oberen und mittleren Atemweg und dann aus dem Körper. Ein Volumenfluss-Sensor, beispielsweise die Sensoreinheit 2 oder der Sensor 15, misst zu mehreren Abtast-Zeitpunkten den jeweiligen Volumenfluss Vol' an einer Stelle in der Fluidverbindung. Dieser gemessene Volumenfluss Vol' korreliert mit dem Volumenfluss, der in den Körper des Patienten P hineinströmt und aus diesem herausströmt.

Mithilfe eines $CO_2$-Sensors wird der Anteil an $CO_2$ in der Fluidverbindung gemessen. Nur der alveoläre Lungenraum, nicht aber der obere und mittlere Atemweg, kann $CO_2$ in die Luft einspeisen. Sobald dieser Anteil oberhalb einer vorgegebenen Schranke ist, enthält die Fluidverbindung ausgeatmetes Gas aus der Lunge Lu. Im Zeitraum zwischen dem Beginn eines Ausatmungsvorgangs und dem Zeitpunkt, an dem ein relevanter $CO_2$-Anteil in dem ausgeatmeten Gas enthalten ist, fließt Gas aus dem oberen mittlere Atemweg des Patienten P in die Fluidverbindung. Die Werte für den Volumenfluss, die in dem Zeitraum bis zur Detektion von $CO_2$ gemessen werden, liefern nach einer numerischen Integration ein Maß für das Volumen $V_{D,Pat}$ des Totraums im oberen und mittleren Atemweg des Patienten P. Das patientenseitige Totraum-Volumen $V_{D,Pat}$ lässt sich beispielsweise mithilfe der Bohr-Formel ermitteln.

Das Volumen $V_{D,Ger}$ des geräteseitigen Totraums in der Fluidverbindung außerhalb des Körpers des Patienten P ist durch die Konstruktion des Beatmungsgeräts 1 und der Schläuche in der Regel ausreichend genau bekannt. Falls der Volumenfluss nahe dem Mund des Patienten P gemessen wird, kann das Volumen $V_{D,Ger}$ des geräteseitigen Totraums vernachlässigt werden.

In einer anderen Ausgestaltung wird kein $CO_2$-Sensor benötigt. Vielmehr wird ein Idealgewicht $Gew_{id}$ des Patienten P ermittelt, und das Volumen $V_{D,Pat}$ des patientenseitigen Totraums im oberen und mittleren Atemweg des Patienten P wird gemäß der Formel

$$(4) \qquad V_{D,Pat} = y * Gew_{id}$$

geschätzt. Hierbei ist y ein empirisch ermittelter Faktor.

Eine weitere Randbedingung resultiert aus der Anforderung, dass die tatsächliche Beatmungsfrequenz $f_{ist}$ klein genug sein muss, um auch eine wenig elastische Lunge Lu, also eine Lunge mit einer hohen Lungenzeitkonstante $\tau$, noch ausreichend zu dehnen. Anders gesagt: Bei einer zu hohen Beatmungsfrequenz $f_{ist}$ reicht der Druck der Beatmung nicht aus, um die Lunge Lu ausreichend zu dehnen. Die Lunge Lu kann dann nicht genug Sauerstoff aufnehmen und auch nicht genug $CO_2$ abgeben. Diese weitere Randbedingung hängt von der Lunge Lu des Patienten P ab.

Die Lungenzeitkonstante $\tau$ variiert von Patient zu Patient und kann sich auch bei ein und demselben Patienten P im Laufe der Zeit verändern. Um die gerade genannte weitere Randbedingung zu erfüllen, wird am Patienten P die Lungenzeitkonstante $\tau$ gemessen. Vereinfachend kann man die Lunge als ein lineares pneumatisches System ansehen, wobei ein an der Lunge Lu anliegender Druck pneumatischen Widerstand überwindet und eine Dehnung der elastischen Lunge Lu bewirkt. Unter dieser vereinfachenden Annahme gilt bei der Einatmung:

$$(5) \qquad Vol_{Lu}(t) = Vol_{Lu,max} * [1 - e^{-\frac{T_i}{RC}}]$$

Hierbei sind R der Lungenwiderstand (resistance) und C die Compliance. $Vol_{Lu}(t)$ ist das Volumen der Lunge zum Zeitpunkt t, $Vol_{Lu,max}$ das maximale Lungenvolumen. Vereinfacht gesagt gilt für den Lungenwiderstand R

$$(6) \qquad R = \Delta P / Vol',$$

wobei $\Delta P$ die Differenz zwischen dem maximalen und dem minimalen Druck im Verlaufe eines Beatmungshubs ist und Vol' die Volumenänderung der Lunge Lu aufgrund dieses Differenzdrucks. Die Compliance C ist der Kehrwert der Elastizität E (elastance), wobei gilt

$$(7) \qquad C = Vol / \Delta P.$$

Die Lungenzeitkonstante $\tau$ ist das Produkt aus dem Lungenwiderstand R und der Compliance C, also

$$(8) \qquad \tau = R*C.$$

Die Lunge Lu lässt sich vereinfacht als ein passives pneumatisches System beschreiben und dadurch modellieren. Die Lungenzeitkonstante $\tau$ ist ein Parameter, der idealisiert die Antwort der Lunge Lu gemäß diesem Lungenmodell auf eine Sprunganregung beschreibt. Bei einer eigenen Atmungsaktivität des Patienten P resultiert die Sprunganregung aus einer schlagartigen Erhöhung oder auch Reduzierung des vom Zwerchfell Zw erzeugten Drucks $P_{mus}$ . Bei einer künstlichen Beatmung resultiert die Sprunganregung aus einer schlagartigen Erhöhung oder Reduzierung des Atemwegsdrucks $P_{aw}$. Als Reaktion auf die Sprunganregung steigt bzw. fällt das Volumen exponentiell, vgl. die Modellgleichung (5) und die Festlegung (8).

Wenn seit der Sprunganregung die Lungenzeitkonstante $\tau$ verstrichen ist, so sind zwei Drittel, genauer 63 %, des gesamten inspiratorischen Tidalvolumens in die Lunge Lu verbracht. Die Lungenzeitkonstante $\tau$ wird beispielsweise in [msec] oder in [sec] angegeben.

Um die Lungenzeitkonstante $\tau$ des Patienten P zu ermitteln, werden mindestens ein bereits erwähnter Volumenfluss-Sensor 2, 15 sowie mindestens einer der Drucksensoren 2, 3, 7 verwendet, die den Volumenfluss Vol' bzw. den Atemwegs-Druck $P_{aw}$ in der Fluidverbindung sowie optional den Speiseröhren-Druck $P_{es}$ messen. Aus dem Volumenfluss Vol' lässt sich der zeitliche Verlauf des Volumens der Lunge Lu herleiten. Ein typischer Wert für die Lungenzeitkonstante $\tau$ eines COPD-Patienten (Patient mit Raucherlunge) beträgt 450 msec. Bei anderen Patienten ist die Lungenzeitkonstante $\tau$ in der Regel deutlich kürzer.

Aus der Lungenzeitkonstante $\tau$ wird die Randbedingung bevorzugt wie folgt abgeleitet: Ein Faktor x wird vorgegeben und hängt nicht von der Lunge Lu des Patienten P ab. Der Faktor x liegt bevorzugt zwischen 4 und 12 und beträgt beispielsweise 5,5 oder 8. Die resultierende Randbedingung ist

$$(9) \qquad f_{set,mand} <= 1 / (x* \tau).$$

Aus den Randbedingungen (3) und (9) resultiert eine obere Schranke $f_{set,mand,max}$ für die mandatorische Soll-Beatmungsfrequenz $f_{set,mand}$, nämlich

$$(10) \quad f_{set,mand,max} = \min \{ V'_{A,req} / V_D,\ 1 / (x^* \tau) \}.$$

Möglich ist, einen "Sicherheitsabschlag" zu verwenden, also

$$(11) \quad f_{set,mand,max} = \min \{ (1-\lambda_1)^* V'_{A,req} / V_D,\ 1-\lambda_2 / (x^* \tau) \}$$

mit vorgegebenen Werten $\lambda_1$, $\lambda_2$.

Hierbei gilt $0 < \lambda_1, \lambda_2 < 1$, bevorzugt $\lambda_1, \lambda_2 <= 0{,}2$.

Es muss gelten: $f_{set,mand} <= f_{set,mand,max}$.

Die Festlegung $f_{set,mand} = f_{set,mand,max}$ würde in vielen Fällen zu einer unnötig hohen Beatmungsfrequenz $f_{ist}$ führen, insbesondere zu einer Beatmungsfrequenz, welche die Lunge Lu stärker als erforderlich belastet.

Daher wird eine ideale mandatorische Soll-Beatmungsfrequenz $f_{set,mand,id}$ berechnet, welche kleiner als die obere Schranke $f_{set,mand,max}$ sein kann, aber nicht größer. Die mandatorische Sollfrequenz $f_{set,mand}$ wird gemäß der Vorschrift

$$(12) \quad f_{set,mand} = \min \{ f_{set,mand,id},\ f_{set,mand,max} \}.$$

festgelegt. Wie die Signalverarbeitungseinheit die ideale mandatorische Soll-Beatmungsfrequenz $f_{set,mand,id}$ berechnet, wird im Folgenden beschrieben.

Ein Beatmungshub bewirkt einen Einatmungsvorgang. Bei mandatorischer Beatmung hat der Einatmungsvorgang $T_i$ die gleiche Dauer wie der verursachende Beatmungshub. Ein erwartetes oder angestrebtes Verhältnis für die Dauer $T_i$ der Einatmung (Inspiration) im Vergleich zur Dauer $T_e$ der Ausatmung (Exspiration) während eines einzelnen Beatmungsvorgang wird vorgegeben, also der I:E ratio. Typische Werte liegen zwischen 3:5 und 4:5. Möglich ist, dass dieses Verhältnis von der Lungenzeitkonstante $\tau$ des Patienten P abhängt. Hieraus resultiert ein vorgegebener Faktor D1, das ist der geforderte zeitliche Anteil der Inspiration an einem gesamten Atmungszyklus (Atmungsvorgang), also

$$(13) \quad D1 = T_i / (T_i + T_e)$$

Dieser Faktor D1 fungiert als der geforderte Einatmungsanteil. Möglich ist auch, als Einatmungsanteil direkt den Quotienten $T_i / T_e$ vorzugeben, also den I:E ratio. Möglich ist auch, eine geforderte durchschnittliche Dauer eines Einatmungsvorgangs oder eines Ausatmungsvorgangs vorzugeben. Aus dieser Dauer des Einatmungsvorgangs bzw. Ausatmungsvorgangs sowie aus der Soll-Beatmungsfrequenz $f_{set}$ resultiert ein geforderter Einatmungsanteil D1.

Die mandatorische Beatmung des Patienten P leistet eine mechanische Arbeit, die sich auf eine resistive (viskose) Arbeit $W_R$ und eine elastische Arbeit Wc aufteilt. Die resistive Arbeit $W_R$ überwindet den Lungenwiderstand R. Die elastische Arbeit Wc dehnt die Lunge Lu und wirkt gegen die Elastizität und damit die Compliance C der Lunge Lu. Sowohl die resistive Arbeit $W_R$ als auch die elastische Arbeit Wc hängen von der tatsächlichen Beatmungsfrequenz $f_{ist}$ ab.

Wie bereits dargelegt, wird die Lunge Lu vereinfacht als ein lineares pneumatisches System modelliert. Dieses System entspricht einem elektrischen RC-Glied, also einer Reihenschaltung eines elektrischen Widerstandes R und eines elektrischen Kondensators C. Für die nachfolgende Herleitung wird vereinfachend angenommen, dass bei einem Beatmungshub der jeweils neue Druck sofort anliegt. Mit anderen Worten: Ein Beatmungshub wird als eine Sprunganregung am pneumatischen System Lunge behandelt. Diese vereinfachenden Annahmen führen zu folgenden lungenmechanischen Gleichungen für die resistive Leistung $W'_R$ sowie für die elastische Leistung $W'_C$:

(14)
$$W'_R = \frac{f_{ist}}{2C}\left(\frac{\frac{V'_{A,ist}}{f_{ist}} + V_D}{1 - e^{-\frac{T_i}{RC}}}\right)^2\left(1 - e^{-2\frac{T_i}{RC}}\right) = \frac{f_{ist}}{2C}\left(\frac{V'_{A,ist}}{f_{ist}} + V_D\right)^2 \coth\left(\frac{T_i}{2RC}\right)$$

(15)
$$W'_C = \frac{f_{ist}}{2C}\left(\frac{V'_{A,ist}}{f_{ist}} + V_D\right)^2$$

Die beiden lungenmechanischen Gleichungen (14) und (15) bilden zusammen zwei Bestandteile eines vorgegebenen Lungenmodells. Sie beschreiben die resistive Leistung $W'_R$ bzw. die elastische Leistung $W'_C$, welche das Beatmungsgerät 1 bei mandatorischer Beatmung während eines Inspirationsvorgangs an der Lunge Lu umsetzt. Sie gelten mit ausreichender Genauigkeit für jeden in Betracht kommenden Wert für die Parameter C, R und $f_{ist}$. Gemäß diesem Lungenmodell (14) und (15) ist die resistive Leistung $W'_R$ in jedem Fall größer als die elastische Leistung W'c, egal wie groß der Lungenwiderstand R und die Lungen-Compliance C sind und egal welche tatsächliche Beatmungsfrequenz $f_{ist}$ bei der mandatorischen Beatmung verwendet wird.

Figur 2 zeigt beispielhaft die resistive Leistung $W'_R$, die elastische Leistung $W'_C$ sowie die gesamte Leistung, also die Summe $W'_R + W'_C$, in Abhängigkeit von der tatsächlichen Beatmungsfrequenz $f_{ist}$. Auf der x-Achse ist die Beatmungsfrequenz $f_{ist}$ in [1/min] aufgetragen, auf der y-Achse die Leistung in [ma/sec]. Außerdem ist in Figur 2 als senkrechte Linie die obere Schranke für die Beatmungsfrequenz $f_{ist}$ eingetragen, welche aus der Randbedingung (9) mit dem Wert x = 8 resultiert.

Figur 3 und Figur 4 zeigen beispielhaft den zeitlichen Verlauf des Drucks P (oben) und den zeitlichen Verlauf der geleisteten Arbeit W (unten) während eines Beatmungshubs. Auf der x-Achse ist die Zeit t in Sekunden aufgetragen, auf der y-Achse der Druck P in [mbar] sowie der Volumenfluss Vol' in [l/sec] (oben) bzw. die Arbeit W in [Nm] (unten). Die zeitlichen Verläufe wurden mithilfe der beiden lungenmechanischen Gleichungen (14) und (15) ermittelt. In diesem Beispiel beträgt C = 30 ml/mbar. Das erzielte alveoläre Minutenvolumen $V'_{A,ist}$ beträgt $V'_{A,ist}$ = 7,9 l/min. In Figur 3 beträgt R = 5 mbar/(l*sec), in Figur 4 ist R = 20 mbar/(l*sec). Die tatsächliche Beatmungsfrequenz beträgt im Beispiel von Figur 3 $f_{ist}$ = 15/min, im Beispiel von Figur 4 ist $f_{ist}$ = 30/min.

In den Darstellungen der zeitlichen Verläufe bezeichnen

- $P_{set}$ den zeitlichen Verlauf des vom Beatmungsgerät 1 zu erzeugenden Drucks, wobei der zeitliche Verlauf $P_{set}$ eine Führungsgröße für eine untergeordnete Regelung des tatsächlich erzielten Drucks ist, wobei der tatsächliche Druck (Regelgröße) bevorzugt als der erzielte Atemwegsdruck $P_{aw}$ gemessen wird,
- $P_{lung}$ den zeitlichen Verlauf des resultierenden Drucks in der Lunge Lu,
- Vol' den Volumenfluss in der Fluidverbindung zu dem und optional von dem Patienten P, wobei ein positiver Wert einen Volumenfluss vom Beatmungsgerät 1 zum Patienten P und ein negativer Wert einen Volumenfluss vom Patienten P zum Beatmungsgerät 1 bezeichnet,
- $W_R$ die bislang geleistete resistive Arbeit,
- Wc die bislang geleistete elastische Arbeit,
- $W_{R,ins}$ die am Ende eines Einatmungsvorgangs geleistete resistive Arbeit und
- $W_{C,ins}$ die am Ende eines Einatmungsvorgangs geleistete elastische Arbeit.

Im Beispiel von Figur 3 betragen $W_{R,ins}$ = 0,76 Nm und $W_{C,ins}$ = 0,76 Nm, im Beispiel von Figur 4 sind $W_{R,ins}$ = 0,50 Nm und $W_{C,ins}$ = 0,28 Nm.

In den beiden Beispielen, die in Figur 3 und Figur 4 gezeigt werden, steigt der Soll-Druck $P_{set}$ bei einem Beatmungshub sofort auf den Maximalwert. Figur 3 und Figur 4 zeigen daher die Antwort des pneumatischen Systems Lunge auf eine Sprunganregung durch das Beatmungsgerät 1. In der Realität kann der durch die untergeordnete Regelung erzielte tatsächliche Druck $P_{aw}$ nicht sofort auf den vollen Wert ansteigen. Außerdem muss ausgeschlossen oder wenigstens die Gefahr reduziert werden, dass die Lunge Lu des Patienten P durch einen zu raschen Druckanstieg geschädigt wird. Daher wird der gewünschte zeitliche Verlauf $P_{set}$ des Drucks so vorgegeben, dass er zu Beginn eines Beatmungshubs ansteigt, bis er den Maximalwert erreicht. Diese Zeitspanne zwischen Beginn des Beatmungshubs und Erreichen des Maximalwerts für den Druck $P_{aw}$ wird als tatsächliche Rampenzeit $t_{R,ist}$ bezeichnet. Der gewünschte zeitliche Verlauf $P_{set}$ des Drucks umfasst einen Druckanstieg in einer vorgegebenen Soll-Rampenzeit $t_{R,set}$ und nachfolgend einen maximalen Solldruck. Indem eine Soll-Rampenzeit $t_{R,set}$ vorgegeben wird, wird die resistive Arbeit $W_R$

verglichen mit einem sprungartigen Anstieg des Solldrucks $P_{set}$ reduziert, während die elastische Arbeit Wc unverändert bleibt. Ein Grund hierfür: Die maximalen Volumenströme werden reduziert.

In einer bevorzugten Ausgestaltung wird die Soll-Rampenzeit $t_{R,set}$ abhängig von der ermittelten Lungenzeitkonstante $\tau$ des Patienten P festgelegt, beispielsweise gemäß der Rechenvorschrift

$$(16) \quad t_{R,set} = \gamma * \tau.$$

Hierbei ist der Faktor $\gamma$ vorgegeben und im Datenspeicher 11 abgespeichert. Er liegt bevorzugt zwischen 0,3 und 0,7 und beträgt beispielsweise $\gamma$ = 0,5.

Figur 5 zeigt die zeitlichen Verläufe, die auch in Figur 3 und Figur 4 gezeigt werden, wobei im Beispiel von Figur 5 ein geregelter Druckanstieg durchgeführt wird. Im Beispiel von Figur 5 betragen die Lungen-Compliance C = 30 ml/mbar, der Lungenwiderstand R = 5 mbar/(l*sec), die Beatmungsfrequenz $f_{ist}$ = 15/min und die Rampenzeit $t_{R,ist}$ = 0,25 sec. Ein Minutenvolumen von $V'_{A,ist}$ = 7,9 l/min wird erzielt. Verglichen mit einer Beatmung ohne eine Rampenzeit (Sprungantwort, d.h. $t_{R,ist}$ = 0) ist die resistive Arbeit $W_R$ dann geringer, wenn die Rampenzeit $t_{R,ist}$ berücksichtigt wird. Die elastische Arbeit Wc bleibt unverändert.

Die am Ende eines Einatmungsvorgangs geleistete resistive Arbeit beträgt $W_{R,ins}$ = 0,47 Nm, die am Ende eines Einatmungsvorgangs geleistete elastische Arbeit beträgt $W_{C,ins}$ = 0,76 Nm. Die am Ende eines Ausatmungsvorgangs geleistete resistive Arbeit beträgt $W_{R,exp}$ = 0,76 Nm, die am Ende eines Ausatmungsvorgangs geleistete elastische Arbeit beträgt $W_{C,exp}$ = -0,76 Nm.

Sowohl die resistive Leistung $W'_R$ als auch die elastische Leistung $W'_C$ hängen von der tatsächlichen Beatmungsfrequenz $f_{ist}$ ab. Die resistive Leistung $W'_R$ ist stets größer als die elastische Leistung W'c. Die ideale mandatorische Sollfrequenz $f_{set,mand,id}$ wird abhängig von der Auswirkung der tatsächlichen Beatmungsfrequenz $f_{ist}$ auf die resistive Leistung $W'_R$ und auf die elastische Leistung $W'_C$ festgelegt.

Die Beatmungshübe bewirken bei mandatorischer Beatmung jeweils einen Einatmungsvorgang des Patienten P. Hieraus folgt:

$$(17) \quad f_{ist} * T_i = \frac{T_i}{T_e + T_i} = D1$$

Für die folgende Herleitung wird angenommen, dass die tatsächliche Frequenz $f_{ist}$ der mandatorischen Beatmung gleich der gesuchten idealen mandatorischen Soll-Beatmungsfrequenz $f_{set,mand,id}$ ist, dass also gilt:

$$(18) \quad f_{ist} = f_{set,mand,id}.$$

Der Zusammenhang (17) zeigt, wie unter der Annahme (18) die gesuchte ideale Beatmungsfrequenz $f_{set,mand,id}$, die Dauer $T_i$ eines Beatmungshubs und der zeitliche Einatmungsanteil D1 miteinander zusammenhängen.

Eine bestimmte tatsächliche Beatmungsfrequenz $f_{ist}$ und eine bestimmte Dauer $T_i$ der Beatmungshübe führen gemäß den lungenmechanischen Modellgleichungen (14) und (15) zu einer bestimmten resistiven Leistung $W'_R$ und zu einer bestimmten elastischen Leistung W'c.

In einer Ausgestaltung wird die gesuchte ideale mandatorische Soll-Beatmungsfrequenz $f_{set,mand,id}$ so berechnet, dass eine Funktion, die von den beiden Leistungen $W'_R$ und $W'_C$ abhängt, minimiert wird. Diese Funktion ist beispielsweise die Summe der beiden Leistungen, also $W'_R + W'_C$, oder der Quotient $W'_R / W'_C$ aus den beiden Leistungen.

In einer bevorzugten Ausgestaltung des Ausführungsbeispiels wird die ideale mandatorische Soll-Beatmungsfrequenz $f_{set,mand,id}$ hingegen so berechnet, dass die elastische Leistung W'c etwa gleich der resistiven Leistung $W'_R$ ist. In vielen Fällen führt diese Vorgabe zu einer vergleichsweise geringen mechanischen Belastung der Lunge Lu des Patienten P. Genauer gesagt: Ein Faktor $\alpha$ wird vorgegeben, wobei $\alpha$ bevorzugt zwischen 0 und 0,2 liegt und besonders bevorzugt gleich 0,1 ist. Als Leistungsfaktor fungiert dann 1+$\alpha$. Die Randbedingung bei der Festlegung der idealen mandatorischen Soll-Beatmungsfrequenz $f_{set,mand,id}$ ist

$$(19)\qquad \frac{W'_R}{W'_C} = 1 + \alpha$$

Aus den Gleichungen (15) und (19) folgt

$$(20)\qquad \frac{W'_R}{W'_C} = \coth\left(\frac{T_i}{2RC}\right)$$

Löst man unter dieser Annahme die Gleichungen (20) und (17) jeweils nach $T_i$ auf, so resultiert folgende Berechnungsvorschrift für die ideale mandatorische Soll-Beatmungsfrequenz $f_{set,mand,id}$:

$$(21)\qquad f_{set,mand,id} = \frac{D1}{2*RC*\text{arccoth}\,(1+\alpha)}.$$

Hierbei gilt

$$(22)\qquad \text{arccoth}\,(1+\alpha) = \frac{1}{2}\ln\frac{2+\alpha}{\alpha}.$$

Außerdem sind die oben dargestellten Randbedingungen (3) und (9) zu beachten, also die obere Schranke $f_{set,mand,max}$. Die mandatorische Soll-Beatmungsfrequenz $f_{set,mand}$ wird gemäß der Rechenvorschrift (17) für die ideale mandatorische Soll-Beatmungsfrequenz $f_{set,mand,id}$ festgelegt, es sei denn, eine obere Schranke $f_{set,mand,max}$ führt zu einem niedrigeren Wert, vgl. die Festlegungsvorschrift (12). Hieraus resultiert folgende Berechnungsvorschrift für die mandatorische Soll-Beatmungsfrequenz $f_{set,mand}$:

$$(23)\qquad f_{set,mand} = \min\left\{\frac{D1}{2*RC*\text{arccoth}\,(1+\alpha)},\ \frac{V'_{A,req}}{V_D},\ \frac{1}{x*\tau}\right\}$$

Die Lungenzeitkonstante $\tau$ wird am Patienten P gemessen. Bevorzugt wertet die Signalverarbeitungseinheit 10 hierfür Messwerte aus, die von den Sensoren 3 und 15 geliefert worden sind, und ermittelt durch die Auswertung den Atemwegsdruck $P_{aw}$ und den Volumenfluss Vol'. Aus diesen Signalen $P_{aw}$ und Vol' leitet die Signalverarbeitungseinheit 10 eine Schätzung für das Produkt R*C her und verwendet diese Schätzung als die Lungenzeitkonstante $\tau$ des Patienten P. Vereinfachend lässt sich bei der Ermittlung der Lungenzeitkonstante $\tau$ ein idealer Sprunganstieg des Drucks $P_{aw}$ annehmen, und die Lungenzeitkonstante $\tau$ lässt sich dann allein aus dem gemessenen Volumenfluss Vol' herleiten.

Das Vorgehen, die mandatorische Soll-Beatmungsfrequenz $f_{set,mand}$ gemäß der Rechenvorschrift (23) festzulegen, führt zu einer einfachen und robusten Vorgabe eines Sollwerts $f_{set,mand}$ für die Beatmungsfrequenz $f_{ist}$ unter mandatorischer Beatmung. In vielen Fällen wird eine geringere Soll-Beatmungsfrequenz $f_{set,mand}$ berechnet als bei anderen Verfahren, sodass das erfindungsgemäße Vorgehen zu einer geringeren Belastung der künstlich beatmeten Lunge Lu führt, insbesondere bei einem Patienten P mit einer relativ kleinen Lungenzeitkonstante $\tau$.

Ein Vorteil der gerade beschriebenen Ausgestaltung ist, dass es nicht erforderlich ist, den Lungenwiderstand R und die Lungen-Compliance C getrennt voneinander zu messen. Dies ist insbesondere dann, wenn der Patient P spontan atmet, mit relativ großer Unsicherheit behaftet. Ausreichend ist, die Lungenzeitkonstante $\tau$, also das Produkt R*C, zu messen. Ein ausreichend zuverlässiger Wert für die Lungenzeitkonstante $\tau$ wird in der Regel nach wenigen Atemzügen, welche die eigene Atmungsmuskulatur des Patienten P ausführt, oder wenigen Beatmungshüben des Beatmungsgeräts 1 erhalten.

Im Gegensatz zu anderen Verfahren, welche automatisch eine Beatmungsfrequenz festlegen, erfordert die gerade beschriebene Ausgestaltung nicht, dass zur Laufzeit eine Optimierung durchgeführt wird. Insbesondere ist nicht erforderlich, zur Laufzeit die gesuchte Beatmungsfrequenz so zu bestimmen, dass eine von der Beatmungsfrequenz $f_{set}$ abhängige Funktion, die zu einem lungenmechanischen Modell gehört, minimiert wird. Bei anderen Verfahren beschreibt diese Funktion beispielsweise die Arbeit während eines Beatmungshubs oder die Leistung, die während der Beatmungshübe aufgebracht wird. Falls zur Laufzeit eine Optimierung erforderlich wäre, so werden eine relativ hohe Rechenkapazität und / oder eine relativ lange Rechenzeit benötigt. In der Praxis wird eine Optimierung häufig mittels eines iterativen Verfahrens durchgeführt, welches beendet wird, wenn ein Abbruchkriterium erfüllt ist. In manchen Fällen kann der gefundene Wert relativ weit von einem Optimum entfernt sein.

Das gerade beschriebene Vorgehen erfordert auch nicht notwendigerweise, dass während der künstlichen Beatmung ein sogenanntes Manöver durchgeführt wird, bei welchem ein Betriebsparameter des Beatmungsgeräts 1, beispielsweise ein geforderter zeitlicher Verlauf des Drucks oder des Volumenflusses, für eine kurze Zeitspanne gezielt auf einen abweichenden Wert eingestellt wird, um einen Vitalparameter des Patienten P zu messen. Insbesondere ist keine Okklusion erforderlich, bei der die künstliche Beatmung für einen kurzen Zeitraum eingestellt und optional auch die eigene Atmungsaktivität des Patienten P für diesen kurzen Zeitraum unterbunden wird, um den zeitlich veränderlichen Druck zu messen, der durch die eigene Atmungsaktivität des Patienten P hervorgerufen wird. Ein solches Manöver belastet häufig den Patienten P.

In vielen Fällen führt das gerade beschriebene erfindungsgemäße Vorgehen dazu, dass die mandatorische Beatmung mit einer Beatmungsfrequenz $f_{ist}$ durchgeführt wird, die nicht höher ist als erforderlich, um das angestrebte alveoläre Minutenvolumen $V'_{A,req}$ zu erzielen. Dieses Vorgehen reduziert aus diesem Grund in vielen Fällen die mechanische Belastung der Lunge Lu des Patienten P. Aus diesem Grunde reduziert das Vorgehen die Gefahr, dass die Lunge Lu des Patienten P mechanisch geschädigt wird.

Das angestrebte alveoläre Minutenvolumen $V'_{A,req}$ wird dem Beatmungsgerät 1 bei der künstlichen Beatmung vorgegeben. Vorgehensweisen, wie dies getan werden kann, werden beispielsweise in J. Fernändez, D. Miguelena, H. Mulett, J. Godoy, and F. Martinón-Torres, "Adaptive support ventilation: State of the art review," Indian J. Crit. Care Med., vol. 17, no. 1, p. 16, 2013, beschrieben.

Ebenfalls zu Beginn der künstlichen Beatmung wird das gesamte Totraum-Volumen $V_D$ ermittelt, bevorzugt auf eine der beiden oben beschriebenen Methoden. Bevorzugt wird während der gesamten künstlichen Beatmung wiederholt das patientenseitige Totraum-Volumen $V_{D,Pat}$ gemessen, und dadurch wird insbesondere eine Veränderung des patientenseitigen Totraum-Volumens $V_{D,Pat}$ detektiert und berücksichtigt. Die Faktoren $\alpha$ und x werden bevorzugt einmal fest vorgegeben und sind im Beatmungsgerät 1 abgespeichert. Bei dieser Ausgestaltung wird zu Beginn der künstlichen Beatmung des Patienten P oder auch vorab eine Konstante const ermittelt und abgespeichert, und zwar im Ausführungsbeispiel gemäß der Rechenvorschrift

$$\text{(24)} \quad const = \min\left\{\frac{D1}{2*\operatorname{arccoth}(1+\alpha)}, \frac{1}{x}\right\}$$

Dies führt zu folgender Berechnungsvorschrift:

$$\text{(25)} \quad f_{set,mand} = \min\left\{\frac{V'_{A,req}}{V_D}, \frac{const}{\tau}\right\}$$

In der bislang beschriebenen Ausgestaltung wird ein Wert für den Faktor $\alpha$ vorgegeben. Möglich ist auch, n Werte $\alpha_1, ..., \alpha_n$ für den Faktor $\alpha$ vorzugeben. Die gerade beschriebene Berechnung wird für jeden vorgegebenen Faktor $\alpha_1, ..., \alpha_n$ durchgeführt. Dies liefert n Werte $f_{set,mand}(\alpha_1), ..., f_{set,mand}(\alpha_n)$. Als mandatorische Soll-Beatmungsfrequenz $f_{set,mand}$ wird ein Wert verwendet, der durch eine geeignete Mittelung oder Bildung des Medians oder sonstige Aggregation aus diesen n Werten berechnet wird. Beispielsweise wird der kleinste dieser n Werte verwendet.

Das oben beschriebene Vorgehen zeigt einen Weg auf, um eine Soll-Beatmungsfrequenz $f_{set,mand}$ für eine mandatorische künstliche Beatmung des Patienten P automatisch herzuleiten. Hierfür wird ein angestrebtes alveoläres Minutenvolumen $V'_{A,req}$ oder auch ein proximales Minutenvolumen vorgegeben. Diese Soll-Beatmungsfrequenz $f_{set,mand}$ wird dann für die oben beschriebene untergeordnete Regelung verwendet, wenn der Patient P vollständig narkotisiert ist und daher keine eigene Atmungsaktivität ausführt.

Wie bereits erwähnt, wird dem Verfahren ein angestrebtes alveoläres Minutenvolumen $V'_{A,req}$ vorgegeben. Falls der Patient P vollständig narkotisiert ist, so wird dieses Minutenvolumen $V'_{A,req}$ ausschließlich durch die künstliche Beatmung erzeugt (mandatorische Beatmung). Diese mandatorische Beatmung wird mit der Soll-Beatmungsfrequenz $f_{set,mand}$ durchgeführt, die so wie gerade beschrieben festgelegt wird.

Möglich ist aber auch, dass der Patient P eine eigene Atmungsaktivität durchführt, insbesondere spontan atmet, und einen Anteil des angestrebten alveolären Minutenvolumens $V'_{A,req}$ selber aufbringt. Falls das alveoläre Minutenvolumen, welches der Patient P durch seine eigene Atmungsaktivität aufbringt, mit $V'_{A,spon}$ bezeichnet wird, so gilt.

$$(26)\ SML = V'_{A,spon} / V'_{A,req}$$

Dieser Faktor SML wird ebenfalls ermittelt und verwendet.

In einer Ausgestaltung wird ein Maß für den Unterdruck $P_{mus}$, der durch die Aktivität des Zwerchfells Zw und der Interkostalmuskulatur des Patienten P erzeugt wird, gemessen. Dann sind sowohl der Atemwegsdruck $P_{aw}$, der von außen an der Lunge Lu anliegt, als auch der von innen auf die Lunge Lu einwirkende Unterdruck $P_{mus}$ bekannt. Aus den beiden Signalen $P_{aw}$ und $P_{mus}$ wird dasjenige alveoläre Minutenvolumen $V'_{A,spon}$ hergeleitet, welches ausschließlich durch die die eigene Atmungsaktivität des Patienten P erzielt wird. Denn das Signal $P_{aw}$ wird durch eine Überlagerung der eigenen Atmungsaktivität mit der künstlichen Beatmung bewirkt, das Signal $P_{mus}$ ausschließlich von der eigenen Atmungsaktivität.

In einer Realisierung, um ein Maß für den Unterdruck $P_{mus}$ zu messen, wird der Speiseröhren-Druck $P_{es}$ gemessen. Diese Realisierung setzt voraus, dass die Sonde 3 in die Speiseröhre Sp des Patienten P eingesetzt ist und ein Maß für den Speiseröhren-Druck $P_{es}$ misst. In einer anderen Realisierung werden Signale von den Messelektroden 5.1.1 bis 5.1.2 verwendet, um die elektrische Aktivität der Muskeln des Atmungsapparats des Patienten P näherungsweise zu messen. Diese elektrische Aktivität bewirkt die eigene Atmungsaktivität des Patienten P und hängt nicht von der künstlichen Beatmung ab.

In einer anderen Ausgestaltung wird die Tatsache ausgenutzt, dass dann, wenn die eigene Atmungsaktivität des Patienten P durch das Bearbeitungsgerät 1 unterstützt wird, spontane oder stimulierte Atemzüge des Patienten P detektiert werden und jeder detektierte ausreichend große spontane oder stimulierte Atemzug einen Beatmungshub des Bearbeitungsgeräts 1 auslöst. Je stärker seine eigene Atmungsaktivität ist, desto mehr Beatmungshübe des Beatmungsgeräts 1 löst der Patient P aus. Gezählt wird, wie viele Beatmungshübe pro Zeiteinheit die eigene Atmungsaktivität des Patienten P auslöst. Diese andere Ausgestaltung erfordert nicht notwendigerweise, das alveoläre Minutenvolumen $V'_{A,spon}$ zu ermitteln.

Figur 6 und Figur 7 veranschaulichen zwei Ausführungsformen, wie das Maß für die Intensität der eigenen Atmungsaktivität des Patienten P ermittelt wird. Zu Beginn des jeweils berücksichtigten Zeitraums führt der Patient P keine eigene Atmungsaktivität durch, und das Bearbeitungsgerät 1 führt auch keinen Beatmungshub durch, sodass am Anfang des Zeitraums die beiden Drücke $P_{aw}$ und $P_{es}$ annähernd übereinstimmen. Anschließend differieren diese beiden Drücke voneinander, und zwar aufgrund der eigenen Atmungsaktivität und / oder der künstlichen Beatmung, die später einsetzen.

In der Ausführungsform gemäß Figur 6 wird die Arbeit (work of breathing, WOB) verwendet, die während eines Atemzugs zum Einatmen aufgewendet wird. Auf der x-Achse ist der Druck P in [mbar] aufgetragen, auf der y-Achse das in die Lunge Lu und aus der Lunge Lu fließende Volumen Vol in [l]. Als Maß für den vom Zwerchfell Zw des Patienten P erzeugten Druck wird der Speiseröhren-Druck $P_{es}$ verwendet, der mithilfe der Sonde 3 gemessen wird.

Die in Figur 6 gezeigten Kurven sind durch eine Überlagerung einer Vielzahl von Atemzügen gewonnen, im Ausführungsbeispiel indem der jeweilige Median gebildet wird, alternativ der Mittelwert. Die gezeigten Parameter werden jeweils als eine normalverteilte Zufallsvariable aufgefasst. Dargestellt wird als Messunsicherheit die Standardabweichung, also ein Toleranzband um den Median.

In Figur 6 bezeichnen:

- $PV_{es}$ den Druck-Volumen-Verlauf, den das Zwerchfell Zw aufbringt, also das erzielte Volumen als Funktion des Drucks $P_{mus}$,
- $PV_{aw}$ den Druck-Volumen-Verlauf, den Beatmungsgerät 1 aufbringt, gemessen mit der Sensoreinheit 2 mit dem Messwertaufnehmer 2.1 am Mund des Patienten P und optional mit dem Sensor 15 am Beatmungsgerät 1,
- TL eine Trennlinie, die durch den Schnittpunkt von $PV_{es}$ und $PV_{aw}$ verläuft,
- $WOB_{Pat}$ die vom Patienten P bei einem Einatemzug durch seine eigene Atmungsaktivität aufgebrachte Arbeit, wobei die Arbeit gleich der Fläche zwischen dem Druck-Volumen-Verlauf $PV_{es}$ und der Trennlinie TL ist,

- $WOB_{Vent}$ die vom Beatmungsgerät 1 bei einem Einatemzug durch die künstliche Beatmung aufgebrachte Arbeit, wobei die Arbeit gleich der Fläche zwischen dem Druck-Volumen-Verlauf $PV_{aw}$ und der Trennlinie TL ist, und
- std die Standardabweichung um den Median.

In einer Ausgestaltung wird als Maß für den Druck $P_{mus}$, den das Zwerchfell Zw aufbringt, der Speiseröhren-Druck $P_{es}$ gemessen, und zwar mithilfe der Sonde 3 in der Speiseröhre Sp des Patienten P. In einer anderen Ausgestaltung werden die zeitlichen Verläufe des Drucks $P_{aw}$ und des Volumenflusses Vol' am Mund des Patienten P gemessen, bevorzugt mithilfe des Sensors 2 und optional des Sensors 15, und aus diesen zeitlichen Verläufen wird mithilfe eines Lungenmodells ein Maß für den Druck $P_{mus}$ abgeleitet. Bevorzugt wird über mehrere Einatemzüge gemittelt.

Die gesamte bei einem Einatemzug aufgebrachte Arbeit WOB ist die Summe aus $WOB_{Pat}$ und $WOB_{Vent}$. Als Maß für den Anteil der eigenen Atmungsaktivität des Patienten P wird der Anteil der vom Patienten P aufgebrachten Arbeit der gesamten Arbeit WOB berechnet und verwendet, also

$$(27)\ SML = WOB_{Pat} / (WOB_{Pat} + WOB_{Vent}).$$

Im Beispiel von Figur 6 beträgt der Faktor SML im Beispiel links etwa 41 %, im Beispiel rechts nur 13 %.

Bei der gerade beschriebenen Ausführungsform wird bevorzugt eine gemittelte Arbeit berechnet. Bei diesem Ansatz wird relativ gut die eigene Atmungsaktivität des Patienten P quantitativ erfasst.

In der Ausgestaltung gemäß Figur 7 wird der gemittelte Druck P während eines Einatemzugs verwendet. PTP bedeutet "pressure-time product". Auf der x-Achse ist die Zeit t in [sec] aufgetragen, auf der y-Achse der Druck in [mbar] für die beiden Drücke $P_{aw}$ und $P_{es}$. Die Trennlinie TL verläuft durch den Schnittpunkt der Verläufe von $P_{aw}$ und $P_{es}$. Die Fläche $PTP_{Pat}$ zwischen dem zeitlichen Verlauf des Drucks $P_{es}$ und der Trennlinie TL ist ein Maß für den mittleren Druck $P_{mus}$, den der Patient P durch seine eigene Atmungsaktivität aufbringt, und korreliert mit der mechanischen Leistung der eigenen Atmungsaktivität des Patienten P. Die Fläche PTPvent zwischen dem zeitlichen Verlauf des Drucks $P_{aw}$ und der Trennlinie TL ist ein Maß für den vom Beatmungsgerät 1 aufgebrachten mittleren Druck $P_{aw}$ und korreliert mit der mechanischen Leistung der künstlichen Beatmung. Als Maß für den Anteil der eigenen Atmungsaktivität des Patienten P wird folgender Quotient verwendet:

$$(28)\ SML = PTP_{Pat} / (PTP_{Pat} + PTP_{Vent}).$$

In dem in Figur 7 oben gezeigtem Beispiel ist der Anteil der eigenen Atmungsaktivität deutlich größer als in dem unten gezeigten Beispiel.

Zu Anfang der künstlichen Beatmung wird das Beatmungsgerät 1 so betrieben, dass bei der künstlichen Beatmung ein angestrebter zeitlicher Verlauf des Atemwegsdrucks $P_{aw}$ oder auch des Volumenflusses Vol' erzielt wird. Ein angestrebter zeitlicher Verlauf wird vorgegeben, und eine untergeordnete Regelung oder Steuerung wird mit dem Ziel durchgeführt, dass der tatsächliche zeitliche Verlauf gleich dem angestrebten zeitlichen Verlauf ist. Bei der künstlichen Beatmung führt das Beatmungsgerät 1 eine Abfolge von Beatmungshüben aus. Ein Teil dieser Beatmungshübe wird durch die eigene Atmungsaktivität des Patienten P ausgelöst, der Rest automatisch vom Beatmungsgerät 1, um den angestrebten zeitlichen Verlauf des Drucks oder Volumenflusses zu erzielen. Bei einer ausreichend intensiven eigenen Atmungsaktivität ist es auch möglich, dass jeder Beatmungshub des Beatmungsgeräts 1 von einem Atemzug des Patienten P ausgelöst wird.

Der Faktor SML wird z.B. gemäß einer der Rechenvorschriften (26), (27), (28) oder gemäß

$$(29)\ SML = n_{spon} / n_{ges}$$

berechnet. Hierbei bezeichnen $n_{spon}$ die Anzahl der Beatmungshübe, die innerhalb eines Zeitraums durch die eigene Atmungsaktivität des Patienten P ausgelöst werden, und $n_{ges}$ die Anzahl der insgesamt in diesem Zeitraum durchgeführten Beatmungshübe des Beatmungsgeräts 1.

Außerdem wird eine ideale Frequenz $f_{spon}$ der eigenen Atmungsaktivität des Patienten P berechnet. Hierbei wird bevorzugt vereinfachend angenommen, dass die eigene Atmungsaktivität des Patienten P einen sinusförmigen zeitlichen Verlauf hat. Falls der Patient P das angestrebte alveoläre Minutenvolumen $V'_{A,req}$ ausschließlich durch seine eigene Atmungsaktivität aufnehmen kann, so atmet er gemäß dieser Vereinfachung mit der idealen Frequenz

(30)
$$f_{spon} = \frac{\sqrt{1 + 4*\pi^2*\tau*\frac{V'_{A,req}}{V_D}} - 1}{2*\pi^2*\tau}$$

Diese ideale Frequenz $f_{spon}$ erfordert die geringste Leistung $W'_R + W'_C$, um durch die eigene Atmungsaktivität das angestrebte alveoläre Minutenvolumen $V'_{A,req}$ zu erzielen. Anders gesagt: Jede andere Frequenz der eigenen Atmungsaktivität erfordert eine höhere Leistung.

In dieser Berechnung wird das gesamte Totraum-Volumen $V_D$ berücksichtigt, also zusätzlich zum patientenseitigen Totraum-Volumen $V_{D,Pat}$ auch das geräteseitige Totraum-Volumen $V_{D,Ger}$.

Eine ähnliche Berechnungsvorschrift ist bereits in J. Mead: "Control of respiratory frequency," J. Appl. Physiol., vol. 15, no. 3, pp. 325 - 336, 1960, angegeben.

Andere Berechnungsvorschriften sind ebenfalls möglich. Beispielsweise wird eine ideale Frequenz der eigenen Atmungsaktivität ermittelt, welche bei gegebenem angestrebten alveolären Minutenvolumen $V'_{A,req}$ zu einer minimalen Amplitude des zeitlich veränderlichen erzeugten Drucks führt.

Die Soll-Beatmungsfrequenz $f_{set}$ des Beatmungsgeräts 1 wird wie folgt festgelegt:

$$(31)\ f_{set} = SML*f_{spon} + (1-SML)*f_{set,mand}.$$

Hierbei sind $f_{spon}$ die ideale Frequenz der eigenen Atmungsaktivität, die beispielsweise gemäß der Rechenvorschrift (30) berechnet wird, $f_{set,mand}$ die erfindungsgemäß festgelegte Soll-Beatmungsfrequenz für die mandatorische Beatmung und SML der Faktor, der beispielsweise gemäß der Berechnungsvorschrift (29) oder unter Verwendung des Speiseröhren-Drucks $P_{es}$ gemäß der Rechenvorschrift (26) oder gemäß (27) oder (28) festgelegt wird.

Die Ausgestaltung gemäß der Festlegungsvorschrift (31) vermeidet einen abrupten Übergang zwischen einer unterstützenden und einer mandatorischen Beatmung, wenn die eigene Atmungsaktivität des Patienten P sich ändert. Vielmehr wird ein allmählicher Übergang erreicht, wobei die mandatorische Beatmung umso schwächer wird, je stärker die eigene Atmungsaktivität des Patienten P ist, und umgekehrt.

Durch eine Abweichung wird sichergestellt, dass die Soll-Beatmungsfrequenz $f_{set}$ mindestens so groß wie die ideale Frequenz $f_{spon}$ der eigenen Atmungsaktivität. Die Rechenvorschrift gemäß der Abweichung lautet

(32)
$$f_{set,mand} = \min\left\{\frac{V'_{A,req}}{V_D}, \frac{const}{\tau}\right\}, \text{ falls } \frac{V'_{A,req}}{V_D} > f_{spon} \text{ und } \frac{const}{\tau}$$

$$f_{set,mand} = f_{spon} \text{ ansonsten}$$

Diese abweichende Rechenvorschrift stellt sicher, dass $f_{set,mand} >= f_{spon}$ und daher auch $f_{set} >= f_{spon}$ gilt. Auch bei dieser abweichenden Rechenvorschrift wird die Soll-Beatmungsfrequenz $f_{set}$ gemäß der Rechenvorschrift (31) festgelegt.

Ein Betriebsparameter, der sich am Beatmungsgerät 1 einstellen lässt, ist das Soll-Tidalvolumen $Vol_{Tid,set}$, also das Volumen, welches bei einem Atemzyklus in den Mund des Patienten P appliziert werden soll. Dieses Volumen stimmt im Wesentlichen mit dem Volumen überein, welches bei einem Beatmungshub aus dem Beatmungsgerät 1 ausgestoßen werden soll. Eine untergeordnete Regelung oder Steuerung erhält das Soll-Tidalvolumen $Vol_{Tid,set}$ als Vorgabe und steuert das Beatmungsgerät 1 mit dem Ziel an, dass das durch die Beatmungshübe tatsächlich erzielte Tidalvolumen $Vol_{Tid,ist}$ gleich dem berechneten Soll-Tidalvolumen $Vol_{Tid,set}$ ist.

Das tatsächlich erzielte Tidalvolumen $Tid_{Vol,ist}$ wird während eines Einatmungsvorgangs in die Fluidverbindung ausgestoßen und teilt sich auf das erzielte alveoläre Lungenvolumen $V_{A,ist}$ sowie auf den Totraum in Schläuchen der Fluidverbindung sowie im oberen und mittleren Atemweg des Patienten P auf, wobei der gesamte Totraum das Volumen $V_D$ aufweist. Das tatsächliche Tidalvolumen $Tid_{Vol,ist}$ soll das geforderte alveoläre Minutenvolumen $V'_{A,req}$ erzeugen und hängt von der tatsächlichen Beatmungsfrequenz $f_{ist}$ und dem Totraum-Volumen $V_D$ ab.

Die Einatmungszeit $T_i$ und somit die Dauer eines Beatmungshubs hängen gemäß dem Zusammenhang (17) von der tatsächlichen Beatmungsfrequenz $f_{ist}$ und dem Verhältnis D1 wie folgt ab:

(33)
$$T_i = \frac{D1}{f_{ist}}$$

Möglich ist, das Soll-Tidalvolumen $Vol_{Tid,set}$ durch Umstellung des Zusammenhangs (1) wie folgt zu berechnen:

(34)
$$Vol_{Tid,set} = \frac{V'_{A,req}}{f_{set}} + V_D$$

In einer Ausgestaltung wird das Soll-Tidalvolumen $Vol_{Tid,set}$ hingegen gemäß der nachfolgenden Rechenvorschrift berechnet. Die Anwendung dieser Rechenvorschrift führt zu einem größeren Soll-Tidalvolumen $Vol_{Tid,set}$, und die Berechnung hängt von der Dauer $T_i$ eines Einatmungsvorgangs und von der Lungenzeitkonstante $\tau$ ab:

(35)
$$Vol_{Tid,set} = \frac{\frac{V'_{A,req}}{f_{set}} + V_D}{1 - e^{-\frac{T_i}{RC}}} = \frac{\frac{V'_{A,req}}{f_{set}} + V_D}{1 - e^{-\frac{T_i}{\tau}}}$$

Figur 8 veranschaulicht die Festlegung der Soll-Beatmungsfrequenz $f_{set}$. In diesem Beispiel wurden folgende Parameter vorgegeben oder gemessen:

- Das Idealgewicht $Gew_{id}$ des Patienten P beträgt 75 kg.
- Der Faktor x beträgt 5,5. Zur Erinnerung: $f_{set,mand} <= 1 / (x^* \tau)$.
- Das geforderte alveoläre Minutenvolumen $V'_{A,req}$ beträgt 5,1 l/min.
- Das Volumen des Totraums beträgt 2,2 ml/kg.
- Die Compliance C der Lunge Lu beträgt 50 ml/mbar.
- Die Resistance R der der Lunge Lu beträgt 5 mbar*sec/l.
- Somit beträgt die Lungenzeitkonstante $\tau$ = R*C = 250 msec.

Auf der x-Achse von Figur 8 ist die tatsächliche Beatmungsfrequenz $f_{ist}$ in [1/min] aufgetragen, auf der y-Achse die resultierende Leistung in [W].

Die ideale mandatorische Soll-Beatmungsfrequenz $f_{set,mand,id}$ wird gemäß der Rechenvorschrift (21) hergeleitet, was zu einem Wert $f_{set,mand,id}$ = 21 / min führt. Dieser Wert liegt oberhalb der idealen Frequenz $f_{spon}$ für die eigene Atmungsaktivität ($f_{spon}$ = 17 / min) und unterhalb der oberen Schranke $f_{set,mand,max}$ für die mandatorische Soll-Beatmungsfrequenz, denn $V'_{A,req} / V_D$ = 28 / min und 1 / (x* $\tau$) = 43 / min. Somit wird gemäß der Rechenvorschrift (32) die Festlegung $f_{set,mand} = f_{set,mand,id}$ = 21 / min getroffen.

In Figur 8 ist außerdem diejenige Beatmungsfrequenz $f_{minW}$ eingetragen, welche zu einer minimalen gesamten Leistung $W'_R+W'_C$ führt. Diese Beatmungsfrequenz $f_{minW}$ lässt sich durch eine Minimierung berechnen. Wie bereits dargelegt, erspart das erfindungsgemäße Verfahren eine solche Minimierung. Wie zu sehen ist, ist die Beatmungsfrequenz $f_{minW}$ für die minimale Leistung größer als die ideale mandatorische Soll-Beatmungsfrequenz $f_{set,mand,id}$.

Ein beispielhafter Ablauf, um automatisch Werte für verschiedene Parameter bei der künstlichen Beatmung zu berechnen, wird im Folgenden dargelegt. Dieser Ablauf umfasst die folgenden Schritte:

- Die Faktoren $\alpha$ und x für die mandatorische Beatmung sowie der Faktor $\gamma$ für die Soll-Rampenzeit $t_{R,set}$ und der Einatmungsanteil D1 werden vorgegeben, beispielsweise indem sie eingegeben und im Beatmungsgerät 1 abgespeichert werden

- Ein angestrebtes alveoläres Minutenvolumen $V'_{A,req}$ oder proximales Minutenvolumen wird ermittelt und dem Ablauf vorgegeben.
- Das gesamte Totraum-Volumen $V_D = V_{D,Pat} + V_{D,Ger}$ wird ermittelt. Das Volumen $V_{D,Pat}$ des patientenseitigen Totraums wird mithilfe eines $CO_2$-Sensors näherungsweise gemessen, beispielsweise unter Anwendung der Bohr-Formel. Oder es wird mithilfe des Zusammenhangs (4) geschätzt. Das Volumen $V_{D,Ger}$ des geräteseitigen Totraums ist durch die Konstruktion des Beatmungsgerät 1 und der Verbindung zum Patienten P ausreichend genau bekannt oder ist vernachlässigbar klein.
- Die künstliche Beatmung durch das Beatmungsgerät 1 wird gestartet.
- Die Lungenzeitkonstante $\tau$ des Patienten P wird gemessen, wofür bevorzugt der bereits erwähnte Volumenfluss-Sensor 2, 15 sowie mindestens einer der Drucksensoren 2, 3, 7 verwendet werden.
- Eine Soll-Rampenzeit $t_{R,set}$ für den Druckanstieg bei einem Beatmungshub wird berechnet, beispielsweise gemäß der Berechnungsvorschrift (16).
- Der Faktor SML, also der Anteil am angestrebten alveolären Minutenvolumen $V'_{A,req}$, den der Patient P durch seine eigene Atmungsaktivität selber aufbringt, wird ermittelt, vgl. den Zusammenhang (26). Beispielsweise wird der Faktor SML gemäß dem Zusammenhang (29) oder mithilfe der Messsonde 3, die den Speiseröhren-Druck $P_{es}$ misst, ermittelt.
- Eine ideale Frequenz $f_{spon}$ der eigenen Atmungsaktivität des Patienten P wird berechnet, beispielsweise gemäß der Rechenvorschrift (30).
- Eine Soll-Beatmungsfrequenz $f_{set,mand}$ für die mandatorische Beatmung wird berechnet, bevorzugt gemäß der Rechenvorschrift (23) oder (25).
- Eine Soll-Beatmungsfrequenz $f_{set}$, welche die eigene Atmungsaktivität des Patienten P berücksichtigt, wird berechnet, bevorzugt gemäß der Rechenvorschrift (31).
- Die Dauer $T_i$ eines Einatmungsvorgangs und somit die Dauer $T_i$ eines Beatmungshubs werden berechnet, und zwar bevorzugt gemäß der Rechenvorschrift (33).
- Ein Soll-Tidalvolumen $Vol_{Tid,set}$ wird festgelegt, bevorzugt gemäß der Rechenvorschrift (35).
- Aus der erfindungsgemäß berechneten Soll-Beatmungsfrequenz $f_{set,mand}$, dem festgelegten Soll-Tidalvolumen $Vol_{Tid,set}$, der hergeleiteten Soll-Rampenzeit $t_{R,set}$ und dem vorgegebenen Einatmungsanteil D1 resultiert ein zeitlicher Verlauf des Solldrucks $P_{set}$, den das Beatmungsgerät 1 erzielen soll.
- Eine untergeordnete Regelung oder Steuerung wird mit dem Ziel durchgeführt, dass der tatsächliche Verlauf des erzielten Drucks, beispielsweise gemessen als Atemwegsdruck $P_{aw}$, gleich dem Verlauf des Drucks $P_{set}$ ist.

In einer Ausgestaltung werden die berechnete Soll-Beatmungsfrequenz $f_{set}$ und das festgelegte Soll-Tidalvolumen $Vol_{Tid,set}$ auf der Anzeige- und Bedieneinheit 12 dargestellt. Ein Benutzer kann diese Werte bestätigen oder einen angezeigten Wert durch einen manuell eingegebenen Wert überschreiben.

**Bezugszeichenliste**

| | |
|---|---|
| 1 | Beatmungsgerät, beatmet den Patienten P künstlich, umfasst die Anzeige- und Bedieneinheit 12 und die Signalverarbeitungseinheit 10 |
| 2 | pneumatische Sensoreinheit vor dem Mund des Patienten P, misst den Atemwegsdruck $P_{aw}$ und optional den Volumenstrom Vol', fungiert als der Atemwegsdruck-Sensor, umfasst die Bestandteile 2.1 und 2.2 |
| 2.1 | Messwertaufnehmer der Sensoreinheit 2, greift eine Gasprobe aus der Fluidverbindung zwischen der Lunge Lu des Patienten P und dem Beatmungsgerät 1 ab |
| 2.2 | eigentlicher Drucksensor der Sensoreinheit 2, wertet die Gasprobe vom Messwertaufnehmer 2.1 aus |
| 3 | Sonde in der Speiseröhre Sp des Patienten P, misst den Speiseröhren-Druck $P_{es}$ und optional den gastralen Druck $P_{ga}$, mit dem Messkatheter 6 verbunden |
| 4 | Anschlussstück im Mund des Patienten P, mit dem Messkatheter 6 in der Speiseröhre Sp verbunden |
| 5.1.1, 5.1.2 | herznahes Paar von Messelektroden auf der Haut des Patienten P |
| 5.2.1, 5.2.2 | zwerchfellnahes Paar von Messelektroden auf der Haut des Patienten P |
| 6 | Messkatheter in der Speiseröhre Sp des Patienten P, mit der Messsonde 3 und dem Anschlussstück 4 verbunden |
| 7 | gastrale Sonde im Magen Ma des Patent P, misst den gastralen Druck $P_{ga}$ |

(fortgesetzt)

| | |
|---|---|
| 10 | datenverarbeitende Signalverarbeitungseinheit, empfängt Signale von den Sensoren 2, 3, 5.1.1 bis 5.2.2 sowie 15, berechnet eine Soll-Beatmungsfrequenz $f_{set}$, hat wenigstens zeitweise Lesezugriff auf den Datenspeicher 11 |
| 11 | Datenspeicher, in dem ein Maß für einen angestrebten Volumenflusses in die Lunge Lu des Patienten P abgespeichert ist |
| 12 | Anzeige- und Bedieneinheit des Beatmungsgeräts 1 |
| 15 | Sensor am Beatmungsgerät 1, misst den Volumenstrom Vol' |
| $\alpha$ | Faktor, um den die resistive Leistung $W'_R$ größer sein soll als die elastische Leistung W'c |
| C | Compliance der Lunge Lu des Patienten P, gleich Vol / $\Delta$P |
| D1 | D1 = $T_i$ / ($T_i$ + $T_e$), Einatmungsanteil, zeitlicher Anteil des Einatmungsvorgangs an einem Beatmungsvorgang |
| $f_{ist}$ | tatsächliche Frequenz der eigenen Atmungsaktivität oder der künstlichen Beatmung des Patienten P |
| E | Elastizität der Lunge, Kehrwert der Compliance C |
| $f_{minW}$ | Beatmungsfrequenz, welche zu einer minimalen gesamten Leistung $W'_R$+$W'_C$ führt |
| $f_{set}$ | berechnete Soll-Beatmungsfrequenz für eine künstliche Beatmung des Patienten P, enthält einen Anteil $f_{set,mand}$ für die mandatorische Beatmung und einen von $f_{spon}$ abhängenden Anteil für die unterstützende Beatmung |
| $f_{set,mand}$ | Soll-Beatmungsfrequenz für eine mandatorische Beatmung, um ein angestrebtes alveoläres Minutenvolumen $V'_{A,req}$ zu erzielen, wird automatisch berechnet |
| $f_{set,mand,id}$ | ideale mandatorische Soll-Beatmungsfrequenz, hängt von dem Faktor $\alpha$ ab |
| $f_{set,mand,max}$ | obere Schranke für die mandatorische Soll-Beatmungsfrequenz |
| $f_{spon}$ | ideale Frequenz der eigenen Atmungsaktivität des Patienten P, um das angestrebte alveoläre Minutenvolumen $V'_{A,req}$ zu erzielen |
| $\gamma$ | Faktor für die Festlegung der Soll-Rampenzeit $t_{R,set}$ |
| $Gew_{id}$ | Idealgewicht des Patienten P |
| Ma | Magen des Patienten P |
| nspon | Anzahl der Beatmungshübe, die innerhalb eines Zeitraums durch die eigene Atmungsaktivität des Patienten P ausgelöst werden |
| nges | Anzahl der insgesamt in diesem Zeitraum durchgeführten Beatmungshübe |
| P | Patient, der vom Beatmungsgerät 1 künstlich beatmet wird, hat die Lunge Lu, den Magen Ma, die Speiseröhre Sp und das Zwerchfell Zw |
| $P_{set}$ | gewünschter zeitlicher Verlauf des Drucks, der bei einem Einatmungsvorgang durch einen Beatmungshub des Beatmungsgeräts 1 erzielt wird |
| $PTP_{Pat}$ | Maß für den mittleren Druck, den die eigene Atmungsaktivität des Patienten P bei einem Einatemzug erzeugt |
| PTPvent | Maß für den mittleren Druck, den die künstliche Beatmung des Beatmungsgeräts 1 bei einem Einatemzug erzeugt |
| $PV_{aw}$ | Druck-Volumen-Verlauf, wobei das Zwerchfell Zw und das Beatmungsgerät 1 zusammen den Druck aufbringen und das Volumen aufweisen |
| $PV_{es}$ | Druck-Volumen-Verlauf, den das Zwerchfell Zw aufbringt |
| R | pneumatischer Widerstand der Lunge Lu des Patienten, gleich $\Delta$P / Vol' |
| SML | Faktor, der den Anteil des alveolären Minutenvolumens $V'_{A,spon}$, der durch die eigene Atmungsaktivität des Patienten P aufgebracht wird, am gesamten angestrebten alveolären Minutenvolumen $V'_{A,req}$ angibt |

(fortgesetzt)

| | |
|---|---|
| Sp | Speiseröhre des Patienten P, nimmt optional die Sonde 3 auf |
| T | Lungenzeitkonstante, als Produkt R*C ermittelt |
| $T_e$ | Dauer der Ausatmung (Exspiration) |
| $T_i$ | Dauer der Einatmung (Inspiration), zugleich Dauer eines Beatmungshubs |
| TL | Trennlinie, die durch den Schnittpunkt von $PV_{es}$ und $PV_{aw}$ bzw. durch den Schnittpunkt von $P_{es}$ und $P_{aw}$ verläuft |
| $t_{R,ist}$ | erzielte Rampenzeit zu Beginn eines Beatmungshubs, die verstreicht, bis der volle Druck $P_{set}$ erreicht ist |
| $t_{R,set}$ | Soll-Rampenzeit, Sollwert für die tatsächliche Rampenzeit $t_{R,ist}$ |
| $V'_{A,ist}$ | tatsächliches alveoläres Minutenvolumen in [l/min], tatsächlich in den alveolären Lungenraum des Patienten P fließendes Luftvolumen |
| $V'_{A,req}$ | angestrebtes alveoläres Minutenvolumen in [l/min] bei der künstlichen Beatmung, wird vorgegeben |
| $V'_{A,spon}$ | alveoläres Minutenvolumen in [l/min], welches der Patient P durch seine eigene Atmungsaktivität erzielt |
| $V'_{P,ist}$ | tatsächliches proximales Minutenvolumen in [l/min], tatsächlich durch den Mund des Patienten P fließendes Luftvolumen |
| $V'_{P,req}$ | angestrebtes proximales Minutenvolumen in [l/min] bei der künstlichen Beatmung, wird vorgegeben |
| $V_D$ | Totraum-Volumen in der Fluidverbindung zwischen der Lunge Lu des Patienten P und dem Beatmungsgerät 1, Volumen des Totraums, der bei jedem Beatmungshub mit Luft durchströmt |
| | wird, aber nicht zum Austausch mit dem Blut des Patienten P verwendet wird, Summe aus geräteseitigem Totraum-Volumen $V_{D,Ger}$ und patientenseitigem Totraum-Volumen $V_{D,Pat}$ |
| $V_{D,Ger}$ | geräteseitiges Totraum-Volumen, Volumen des Totraums im Bereich der Fluidverbindung außerhalb des Patienten P |
| $V_{D,Pat}$ | patientenseitiges Totraum-Volumen, Volumen des Totraums im oberen und mittleren Atemweg des Patienten P |
| Vol‘ | Volumenfluss in der Fluidverbindung, vom Sensor 3 und / oder vom Sensor 15 gemessen |
| $Vol_{Tid,ist}$ | tatsächliches Tidalvolumen, welches bei einem Einatmungsvorgang in das Atemsystem des Patienten P fließt |
| $Vol_{Tid,set}$ | Soll-Tidalvolumen, welches bei einem Atemzyklus in den Mund des Patienten P appliziert werden soll, im Wesentlichen gleich dem Volumen, das bei einem Beatmungshub aus dem Beatmungsgerät 1 ausgestoßen werden soll |
| Wc | elastische Arbeit |
| Wc | elastische Leistung |
| $WOB_{Pat}$ | vom Patienten P bei einem Einatemzug durch seine eigene Atmungsaktivität aufgebrachte mechanische Arbeit |
| WOBvent | vom Beatmungsgerät 1 bei einem Einatemzug durch die künstliche Beatmung aufgebrachte mechanische Arbeit |
| x | Faktor für eine obere Schranke der mandatorischen Soll-Beatmungsfrequenz $f_{set,mand}$ |
| $W_{C,exp}$ | am Ende eines Ausatmungsvorgangs geleistete elastische Arbeit |
| $W_{C,ins}$ | am Ende eines Einatmungsvorgangs geleistete elastische Arbeit |
| WR | resistive (viskose) Arbeit |
| $W'_R$ | resistive Leistung |
| $W_{R,exp}$ | am Ende eines Ausatmungsvorgangs geleistete resistive Arbeit |

(fortgesetzt)

| | |
|---|---|
| $W_{R,ins}$ | am Ende eines Einatmungsvorgangs geleistete resistive Arbeit |
| y | Faktor zur Schätzung des Volumens $V_{D,Pat}$ des patientenseitigen Totraums |
| Zw | Zwerchfell des Patienten P |

## Patentansprüche

**1.** Signalverarbeitungseinheit (10), die zum automatischen Berechnen einer Soll-Beatmungsfrequenz ($f_{set}$) für ein Beatmungsgerät (1) ausgestaltet ist,

wobei eine Fluidverbindung zwischen der Lunge (Lu) eines künstlich zu beatmenden Patienten (P) und dem Beatmungsgerät (1) herstellbar oder hergestellt ist,
wobei das Beatmungsgerät (1) dazu ausgestaltet ist, abhängig von der berechneten Soll-Beatmungsfrequenz ($f_{set}$) eine Abfolge von Beatmungshüben in die Fluidverbindung hinein auszuführen,
wobei die Signalverarbeitungseinheit (10) einen Datenspeicher (11) umfasst oder wenigstens zeitweise Lesezugriff auf einen Datenspeicher (11) aufweist,
wobei in dem Datenspeicher (11) in einer von einem Rechner auswertbaren Form ein Maß für einen angestrebten Volumenfluss in die Lunge (Lu) des Patienten (P),
insbesondere ein gefordertes alveoläres Minutenvolumen ($V'_{A,req}$) oder ein gefordertes proximales Minutenvolumen ($V'_{P,req}$),
abgespeichert ist,
wobei die Signalverarbeitungseinheit (10) dazu ausgestaltet ist,

- Messwerte von mindestens einem Patienten-Sensor (2, 3, 7) zu empfangen,
wobei der oder jeder Patienten-Sensor (2, 3, 7) jeweils mindestens einen respiratorischen Parameter des Patienten (P) zu messen vermag,
- abhängig von den Messwerten des oder mindestens eines Patienten-Sensors (2, 3, 7) eine Lungenzeitkonstante ($\tau$) für die Lunge des Patienten (P) zu ermitteln,
- das Volumen ($V_D$) eines Totraums in der Fluidverbindung zu ermitteln, wobei dieser Totraum zwischen dem Beatmungsgerät (1) und einem zum Gasaustausch geeigneten Bereich der Lunge (Lu) des Patienten (P) auftritt,
- abhängig von dem abgespeicherten angestrebten Volumenfluss ($V'_{A,req}$, $V'_{P,req}$), der ermittelten Lungenzeitkonstante ($\tau$) und dem ermittelten Totraum-Volumen ($V_D$)

eine mandatorische Sollfrequenz ($f_{set,mand}$) für die mandatorische Beatmung des Patienten (P) durch das Beatmungsgerät (1) zu berechnen,
wobei die mandatorische Sollfrequenz ($f_{set,mand}$) eine Frequenz ist, mit welcher allein durch künstliche Beatmung der angestrebte Volumenfluss ($V'_{A,req}$, $V'_{P,req}$) erzielt wird,

- abhängig von dem angestrebten Volumenfluss ($V'_A$), der ermittelten Lungenzeitkonstante ($\tau$) und dem ermittelten Totraum-Volumen ($V_D$) eine ideale Spontanatmungs-Frequenz ($f_{spon}$) für die eigene Atmungsaktivität des Patienten (P) zu berechnen,
wobei die ideale Spontanatmungs-Frequenz ($f_{spon}$) eine Frequenz ist, mit welcher der Patient (P) allein durch eigene Atmungsaktivität den angestrebten Volumenfluss ($V'_{A,req}$, $V'_{P,req}$) zu erzielen vermag,
- ein Maß ($n_{spon}$ / $n_{ges}$) für die aktuelle tatsächliche Intensität der eigenen Atmungsaktivität des Patienten (P) zu ermitteln,
wobei das Maß ($n_{spon}$ / $n_{ges}$) für die Intensität der eigenen Atmungsaktivität angibt, welchen absoluten oder relativen Beitrag die eigene Atmungsaktivität des Patienten zu der gesamten Belüftung und Entlüftung der Lunge des Patienten liefert, und
- die Soll-Beatmungsfrequenz ($f_{set}$) als gewichtetes Mittel der mandatorischen Sollfrequenz ($f_{set,mand}$) und der idealen Spontanatmungs-Frequenz ($f_{spon}$) dergestalt zu berechnen, dass bei der Mittelung ein Gewichtsfaktor (SML) vom ermittelten Maß ($n_{spon}$ / $n_{ges}$) für die eigene Atmungsaktivität abhängt,
- wobei der Gewichtsfaktor (SML), mit dem die ideale spontane Atmungsfrequenz ($f_{spon}$) in die Soll-Beatmungsfrequenz ($f_{set}$) eingeht, umso größer ist, je größer das Maß ($n_{spon}$ / $n_{ges}$) für die aktuelle tatsächliche Intensität der eigenen Atmungsaktivität des Patienten (P) ist.

**2.** Signalverarbeitungseinheit (10) nach Anspruch 1,
**dadurch gekennzeichnet, dass**

in dem Datenspeicher (11) eine rechnerauswertbare Festlegung eines geforderten Einatmungsanteils (D1) abgespeichert ist,
wobei der Einatmungsanteil (D1) mit dem zeitlichen Anteil eines Einatmungsvorgangs an einem gesamten Beatmungsvorgang und / oder mit dem Verhältnis zwischen der Dauer ($T_i$) eines Einatmungsvorgangs und der Dauer ($T_e$) eines Atmungsvorgangs korreliert,
wobei die Signalverarbeitungseinheit (10) dazu ausgestaltet ist, bei der Berechnung der mandatorischen Sollfrequenz ($f_{set,mand}$)

- abhängig von der ermittelten Lungenzeitkonstante ($\tau$) eine obere Schranke ($f_{set,mand,max}$) für die mandatorische Sollfrequenz ($f_{set,mand}$) zu berechnen,
- abhängig von dem vorgegebenen geforderten Einatmungsanteil (D1) eine ideale mandatorische Sollfrequenz ($f_{set,mand,id}$) zu berechnen und
- die mandatorische Sollfrequenz ($f_{set,mand}$) unter Verwendung der idealen mandatorischen Sollfrequenz ($f_{set,mand,id}$) dergestalt zu berechnen dass die mandatorische Sollfrequenz ($f_{set,mand}$) kleiner oder gleich der oberen Schranke ($f_{set,mand,max}$) für die mandatorische Sollfrequenz ($f_{set,mand}$) ist,

wobei die Signalverarbeitungseinheit (10) bevorzugt dazu ausgestaltet ist, die mandatorische Sollfrequenz ($f_{set,mand}$) als Minimum von idealer mandatorischer Sollfrequenz ($f_{set,mand,id}$) und oberer Schranke ($f_{set,mand,max}$) zu berechnen.

**3.** Signalverarbeitungseinheit (10) nach Anspruch 2,
**dadurch gekennzeichnet, dass**

in dem Datenspeicher (11) ein rechnerauswertbares Lungenmodell abgespeichert ist,
wobei das Lungenmodell näherungsweise das pneumatische Verhalten der Lunge (Lu) eines Menschen (P) beschreibt,
wobei die Signalverarbeitungseinheit (10) dazu ausgestaltet ist, die ideale mandatorische Sollfrequenz ($f_{set,mand,id}$) abhängig vom geforderten Einatmungsanteil (D1) und zusätzlich abhängig von einer resistiven Leistung ($W'_R$) und von einer elastischen Leistung (Wc) zu berechnen,
wobei die resistive Leistung ($W'_R$) die Leistung ist, die gemäß dem Lungenmodell bei einer mandatorischen Beatmung mit dieser idealen mandatorischen Sollfrequenz ($f_{set,mand,id}$) aufzuwenden ist, und
wobei die elastische Leistung (Wc) die Leistung ist, die gemäß dem Lungenmodell bei einer mandatorischen Beatmung mit dieser Frequenz ($f_{set,mand,id}$) aufzuwenden ist, um die Lunge zu dehnen, und
wobei die Signalverarbeitungseinheit (10) bevorzugt dazu ausgestaltet ist, die resultierende resistive Leistung und die resultierende elastische Leistung für mindestens eine mögliche ideale mandatorische Sollfrequenz ($f_{set,mand,id}$) vorherzusagen.

**4.** Signalverarbeitungseinheit (10) nach Anspruch 3,
**dadurch gekennzeichnet, dass**

in dem Datenspeicher (11) ein Leistungsfaktor ($1+\alpha$) abgespeichert ist, wobei der Leistungsfaktor ($1+\alpha$) größer oder gleich 1 und bevorzugt kleiner als 1,2 ist, und
wobei die Signalverarbeitungseinheit (10) dazu ausgestaltet ist, die ideale mandatorische Sollfrequenz ($f_{set,mand,id}$) dergestalt zu berechnen,
dass bei einer mandatorischen Beatmung mit dieser Frequenz ($f_{set,mand,id}$) das Verhältnis aus

- der resistiven Leistung ($W'_R$), die gemäß dem Lungenmodell bei der mandatorischen Beatmung aufzuwenden ist, und
- der elastischen Leistung (Wc), die gemäß dem Lungenmodell bei der mandatorischen Beatmung aufzuwenden ist,

gleich dem vorgegebenen Leistungsfaktor ($1+\alpha$) ist.

**5.** Signalverarbeitungseinheit (10) nach Anspruch 4,
**dadurch gekennzeichnet, dass**

die Signalverarbeitungseinheit (10) dazu ausgestaltet ist,
in einer Initialisierungsphase unter Verwendung des Lungenmodells und des optionalen Leistungsfaktors ($1+\alpha$) eine Konstante zu berechnen und
die ideale mandatorische Sollfrequenz ($f_{set,mand,id}$) in einer nachfolgenden Nutzungsphase zu berechnen und für die Berechnung der idealen mandatorischen Sollfrequenz ($f_{set,mand,id}$)

- den vorgegebenen Einatmungsanteil (D1),
- die ermittelte Lungenzeitkonstante ($\tau$) und
- die in der Initialisierungsphase berechnete Konstante

zu verwenden.

**6.** Signalverarbeitungseinheit (10) nach einem der Ansprüche 2 bis 5,
**dadurch gekennzeichnet, dass**

die Signalverarbeitungseinheit (10) dazu ausgestaltet ist, die ideale mandatorische Sollfrequenz ($f_{set,mand,id}$) dergestalt zu berechnen,
dass die ideale mandatorische Sollfrequenz ($f_{set,mand,id}$) umso größer ist, je größer der vorgegebene geforderte Einatmungsanteil (D1) ist.

**7.** Signalverarbeitungseinheit (10) nach einem der Ansprüche 2 bis 6,
**dadurch gekennzeichnet, dass**

die Signalverarbeitungseinheit (10) dazu ausgestaltet ist,
die obere Schranke ($f_{set,mand,max}$) für die mandatorische Sollfrequenz ($f_{set,mand}$)

- abhängig von der ermittelten Lungenzeitkonstante ($\tau$) und
- zusätzlich abhängig von dem angestrebten Volumenfluss ($V'_{A,req}$) und / oder abhängig von dem ermittelten Totraum-Volumen ($V_D$)
zu berechnen,
wobei die Signalverarbeitungseinheit (10) bevorzugt dazu ausgestaltet ist, die obere Schranke ($f_{set,mand,max}$) abhängig von

  - der ermittelten Lungenzeitkonstante ($\tau$) und
  - dem Quotienten aus dem angestrebten Volumenfluss ($V'_{A,req}$) und dem Totraum-Volumen ($V_D$) zu berechnen.

**8.** Signalverarbeitungseinheit (10) nach einem der vorhergehenden Ansprüche, **dadurch gekennzeichnet, dass**

die Signalverarbeitungseinheit (10) dazu ausgestaltet ist,
die mandatorische Sollfrequenz ($f_{set,mand}$) für die mandatorische Beatmung dergestalt zu berechnen,
dass die mandatorische Sollfrequenz ($f_{set,mand}$) größer oder gleich der idealen Spontanatmungs-Frequenz ($f_{spon}$) für die eigene Atmungsaktivität des Patienten (P) ist.

**9.** Signalverarbeitungseinheit (10) nach einem der vorhergehenden Ansprüche, **dadurch gekennzeichnet, dass**

die Signalverarbeitungseinheit (10) dazu ausgestaltet ist ,zu ermitteln,
welchen Anteil die mechanische Arbeit ($WOB_{Pat}$) oder die mechanische Leistung,
welche die eigene Atmungsaktivität des Patienten (P) während eines Einatemzugs erbringt,
an der gesamten während des Einatemzugs aufgebrachten Arbeit ($WOB_{Pat}+WOB_{Vent}$) bzw. Leistung hat,
wobei die Signalverarbeitungseinheit (10) weiterhin dazu ausgestaltet ist, das Maß ($n_{spon}$ / $n_{ges}$) für die Intensität der eigenen Atmungsaktivität des Patienten (P) abhängig von diesem Anteil zu ermitteln,
bevorzugt diesen Anteil als das Maß ($n_{spon}$ / $n_{ges}$) zu verwenden.

**10.** Signalverarbeitungseinheit (10) nach einem der vorhergehenden Ansprüche, **dadurch gekennzeichnet, dass**

die Signalverarbeitungseinheit (10) dazu ausgestaltet ist,
abhängig von

- dem angestrebten Volumenfluss ($V'_{A,req}$, $V'_{P,req}$),
- dem ermittelten Totraum-Volumen ($V_D$) und
- der berechneten Soll-Beatmungsfrequenz ($f_{set}$)

ein Soll-Tidalvolumen ($Vol_{Tid,set}$) zu berechnen,
wobei das Soll-Tidalvolumen ($Vol_{Tid,set}$) ein Wert für das Volumen ist, welches das Beatmungsgerät (1) bei einem Beatmungshub in die Fluidverbindung einspeisen soll, und
wobei das Beatmungsgerät (1) dazu ausgestaltet ist, mindestens einen Beatmungshub, bevorzugt jeden Beatmungshub einer Abfolge von Beatmungshüben, dergestalt geregelt oder gesteuert auszuführen,
dass das Ziel der Regelung oder Steuerung ist, dass das tatsächlich bei diesem Beatmungshub erzeugte Tidalvolumen ($Vol_{Tid,ist}$) gleich dem berechneten Soll-Tidalvolumen ($Vol_{Tid,set}$) ist,
wobei bevorzugt die Signalverarbeitungseinheit (10) dazu ausgestaltet ist, die Regelung oder Steuerung für das tatsächlich erzeugte Tidalvolumen ($Vol_{Tid,ist}$) abhängig von dem berechneten Soll-Tidalvolumen ($Vol_{Tid,set}$) durchzuführen.

**11.** Signalverarbeitungseinheit (10) nach einem der vorhergehenden Ansprüche, **dadurch gekennzeichnet, dass**

die Signalverarbeitungseinheit (10) dazu ausgestaltet ist,
eine geforderte Soll-Rampenzeit ($t_{R,set}$) für einen Beatmungshub zu berechnen,
das ist die Zeitspanne, die seit Beginn eines Beatmungshubs verstreicht, bis der maximale Druck erreicht ist, mit dem das Beatmungsgerät (1) den Beatmungshub ausführt,
wobei das Beatmungsgerät (1) dazu ausgestaltet ist, mindestens einen Beatmungshub, bevorzugt jeden Beatmungshub einer Abfolge von Beatmungshüben, dergestalt geregelt oder gesteuert auszuführen,
dass das Ziel der Regelung oder Steuerung ist, dass die tatsächlich bei diesem Beatmungshub erzielte Rampenzeit ($t_{R,ist}$) gleich der berechneten geforderten Soll-Rampenzeit ($t_{R,set}$) ist,
wobei bevorzugt die Signalverarbeitungseinheit (10) dazu ausgestaltet ist, die Regelung oder Steuerung für die tatsächlich erzielte Rampenzeit ($t_{R,ist}$) abhängig von der berechneten Soll-Rampenzeit ($t_{R,set}$) durchzuführen.

**12.** Beatmungsgerät (1) zur künstlichen Beatmung eines Patienten (P),

wobei eine Fluidverbindung zwischen der Lunge (Lu) eines künstlich zu beatmenden Patienten (P) und dem Beatmungsgerät (1) herstellbar oder hergestellt ist,
wobei das Beatmungsgerät (1) wenigstens zeitweise mit mindestens einem Patienten-Sensor (2, 3, 7) verbindbar oder verbunden ist, welcher mindestens einen respiratorischen Parameter des Patienten (P) zu messen vermag,
wobei das Beatmungsgerät (1) einen Datenspeicher (11) umfasst oder wenigstens zeitweise Lesezugriff auf einen Datenspeicher (11) aufweist,
wobei in dem Datenspeicher (11) in einer von einem Rechner auswertbaren Form ein Maß für einen angestrebten Volumenfluss in die Lunge (Lu) des Patienten (P), insbesondere ein gefordertes alveoläres Minutenvolumen ($V'_{A,req}$) oder ein gefordertes proximales Minutenvolumen ($V'_{P,req}$), abgespeichert ist,
wobei das Beatmungsgerät (1) dazu ausgestaltet ist,

- eine Soll-Beatmungsfrequenz ($f_{set}$) zu berechnen und
- abhängig von der berechneten Soll-Beatmungsfrequenz ($f_{set}$) eine Abfolge von Beatmungshüben in die Fluidverbindung hinein auszuführen,
bevorzugt die Beatmungshübe mit der berechneten Soll-Beatmungsfrequenz ($f_{set}$) auszuführen,

wobei das Beatmungsgerät (1) dazu ausgestaltet ist,

- Messwerte von mindestens einem Patienten-Sensor (2, 3, 7) zu empfangen,
wobei der oder jeder Patienten-Sensor (2, 3, 7) jeweils mindestens einen respiratorischen Parameter des Patienten (P) zu messen vermag,
- abhängig von den Messwerten des oder mindestens eines Patienten-Sensors (2, 3, 7) eine Lungenzeitkonstante ($\tau$) für die Lunge des Patienten (P) zu ermitteln,
- das Volumen ($V_D$) eines Totraums in der Fluidverbindung zu ermitteln,
wobei dieser Totraum in der Fluidverbindung zwischen dem Beatmungsgerät (1) und einem zum Gasaustausch geeigneten Bereich der Lunge (Lu) des Patienten (P) auftritt,
- abhängig von dem abgespeicherten angestrebten Volumenfluss ($V'_{A,req}$, $V'_{P,req}$), der ermittelten Lungenzeitkonstante ($\tau$) und dem ermittelten Totraum-Volumen ($V_D$)

eine mandatorische Sollfrequenz ($f_{set,mand}$) für die mandatorische Beatmung des Patienten (P) durch das Beatmungsgerät (1) zu berechnen, wobei die mandatorische Sollfrequenz ($f_{set,mand}$) eine Frequenz ist, mit welcher allein durch künstliche Beatmung der angestrebte Volumenfluss ($V'_{A,req}$, $V'_{P,req}$) erzielt wird,
- abhängig von dem angestrebten Volumenfluss ($V'_A$), der ermittelten Lungenzeitkonstante ($\tau$) und dem ermittelten Totraum-Volumen ($V_D$) eine ideale Spontanatmungs-Frequenz ($f_{spon}$) für die eigene Atmungsaktivität des Patienten (P) zu berechnen,
wobei die ideale Spontanatmungs-Frequenz ($f_{spon}$) eine Frequenz ist, mit welcher der Patient (P) allein durch seine eigene Atmungsaktivität den angestrebten Volumenfluss ($V'_{A,req}$, $V'_{P,req}$) zu erzielen vermag,
- ein Maß ($n_{spon}$ / $n_{ges}$) für die aktuelle tatsächliche Intensität der eigenen Atmungsaktivität des Patienten (P) zu ermitteln, wobei das Maß ($n_{spon}$ / $n_{ges}$ ) für die Intensität der eigenen Atmungsaktivität angibt, welchen absoluten oder relativen Beitrag die eigene Atmungsaktivität des Patienten zu der gesamten Belüftung und Entlüftung der Lunge des Patienten liefert, und
- die Soll-Beatmungsfrequenz ($f_{set}$) als gewichtetes Mittel der

mandatorischen Sollfrequenz ($f_{set,mand}$) und der idealen Spontanatmungs-Frequenz ($f_{spon}$) dergestalt zu berechnen,
dass bei der Mittelung ein Gewichtsfaktor (SML) vom ermittelten Maß ($n_{spon}$ / $_{nges}$ ) für die eigene Atmungsaktivität abhängt,- wobei der Gewichtsfaktor (SML), mit dem die ideale spontane Atmungsfrequenz ($f_{spon}$ ) in die Soll-Beatmungsfrequenz ($f_{se}$ t) eingeht, umso größer ist, je größer das Maß ($n_{spon}$ / $n_{ges}$ ) für die aktuelle tatsächliche Intensität der eigenen Atmungsaktivität des Patienten (P) ist.

**13.** Computerprogramm,

welches auf einer Signalverarbeitungseinheit (10) ausführbar ist und
bei der Ausführung auf der Signalverarbeitungseinheit (10) dann, wenn die Signalverarbeitungseinheit (10) Messwerte von mindestens einem Patienten-Sensor (2, 3, 7) empfängt, welcher mindestens einen respiratorischen Parameter des Patienten (P) zu messen vermag,
bewirkt, dass die Signalverarbeitungseinheit (10) ein Verfahren zum automatischen Berechnen einer Soll-Beatmungsfrequenz ($f_{set}$) für ein Beatmungsgerät (1) durchführt,
wobei das Beatmungsgerät (1) dazu ausgestaltet ist, dann, wenn eine Fluidverbindung zwischen der Lunge (Lu) eines künstlich zu beatmenden Patienten (P) und dem Beatmungsgerät (1) hergestellt oder herstellbar ist, abhängig von der berechneten Soll-Beatmungsfrequenz ($f_{set}$) eine Abfolge von Beatmungshüben in die Fluidverbindung hinein auszuführen,
bevorzugt mit der berechneten Soll-Beatmungsfrequenz ($f_{set}$), und
wobei das Computerprogramm bewirkt, dass das Verfahren die Schritte umfasst, dass

- ein Maß für einen angestrebten Volumenfluss in die Lunge (Lu) und aus der Lunge (Lu) des Patienten (P), insbesondere ein gefordertes alveoläres Minutenvolumen ($V'_{A,req}$) oder ein gefordertes proximales Minutenvolumen ($V'_{P,req}$), vorgegeben wird,
- abhängig von gemessenen Werten mindestens eines respiratorischen Parameters des Patienten (P) eine Lungenzeitkonstante ($\tau$) für die Lunge (Lu) des Patienten (P) ermittelt wird,
- das Volumen ($V_D$) eines Totraums in der Fluidverbindung ermittelt wird, wobei dieser Totraum in der Fluidverbindung zwischen dem Beatmungsgerät (1) und einem zum Gasaustausch geeigneten Bereich der Lunge (Lu) des Patienten (P) auftritt,
- abhängig von dem angestrebten Volumenfluss ($V'_{A,req}$, $V'_{P,req}$), der ermittelten Lungenzeitkonstante ($\tau$) und dem ermittelten Totraum-Volumen ($V_D$) eine mandatorische Sollfrequenz ($f_{set,mand}$) für die mandatorische Beatmung des Patienten (P) durch das Beatmungsgerät (1) berechnet wird,
wobei die mandatorische Sollfrequenz ($f_{set,mand}$) eine Frequenz ist, mit welcher allein durch künstliche Beatmung der angestrebte Volumenfluss ($V'_{A,req}$, $V'_{P,req}$) erzielt wird,
- abhängig von dem angestrebten Volumenfluss ($V'_A$), der ermittelten Lungenzeitkonstante ($\tau$) und dem ermittelten Totraum-Volumen ($V_D$) eine ideale Spontanatmungs-Frequenz ($f_{spon}$) für die eigene Atmungsaktivität des Patienten (P) berechnet wird,
wobei die ideale Spontanatmungs-Frequenz ($f_{spon}$) eine Frequenz ist, mit welcher der Patient (P) allein durch seine eigene Atmungsaktivität den angestrebten Volumenfluss ($V'_{A,req}$, $V'_{P,req}$) zu erzielen vermag,
- ein Maß ($n_{spon}$ / $n_{ges}$) für die aktuelle tatsächliche Intensität der eigenen Atmungsaktivität des Patienten (P) ermittelt wird,
wobei das Maß ($n_{spon}$ / $n_{ges}$) für die Intensität der eigenen Atmungsaktivität angibt, welchen absoluten oder relativen Beitrag die eigene Atmungsaktivität des Patienten zu der gesamten Belüftung und Entlüftung der Lunge des Patienten liefert, und
- die Soll-Beatmungsfrequenz ($f_{set}$) als gewichtetes Mittel der mandatorischen Sollfrequenz ($f_{set,mand}$) und der idealen Spontanatmungs-Frequenz ($f_{spon}$) berechnet wird,

wobei bei der Mittelung ein Gewichtsfaktor (SML) von dem ermittelten Maß ($n_{spon}$ / $n_{ges}$) für die eigene Atmungsaktivität abhängt und
wobei der Gewichtsfaktor (SML), mit dem die ideale spontane Atmungsfrequenz ($f_{spon}$) in die Soll-Beatmungsfrequenz ($f_{set}$) eingeht, umso größer ist, je größer das Maß ($n_{spon}$ / $n_{ges}$) für die aktuelle tatsächliche Intensität der eigenen Atmungsaktivität des Patienten (P) ist.

## Claims

**1.** Signal processing unit (10) configured for automatically calculating a setpoint ventilation frequency ($f_{set}$) for a ventilator (1),

wherein a fluid connection is establishable or is established between the lungs (Lu) of a patient (P) to be artificially ventilated and the ventilator (1),
wherein the ventilator (1) is configured to perform a sequence of ventilation strokes into the fluid connection depending on the calculated setpoint ventilation frequency ($f_{set}$),
wherein the signal processing unit (10) comprises a data memory (11) or has read access to a data memory (11) at least at times,
wherein a measure of an intended volumetric flow rate into the lungs (Lu) of the patient (P),
in particular a required alveolar minute volume ($V'_{A,req}$) or a required proximal minute volume ($V'_{P,req}$),
is stored in the data memory (11) in a computer-evaluable form,
wherein the signal processing unit (10) is configured

- to receive measured values from at least one patient sensor (2, 3, 7),
wherein the or each patient sensor (2, 3, 7) is able to measure in each case at least one respiratory parameter of the patient (P),
- to determine a lung time constant ($\tau$) for the lungs of the patient (P) depending on the measured values of the or at least one patient sensor (2, 3, 7),
- to determine the volume ($V_D$) of a dead space in the fluid connection,
wherein said dead space occurs between the ventilator (1) and a region of the lungs (Lu) of the patient (P) that is suitable for gas exchange,
- to calculate a mandatory setpoint frequency ($f_{set,mand}$) for the mandatory ventilation of the patient (P) by the ventilator (1)

depending on the stored intended volumetric flow rate ($V'_{A,req}$, $V'_{P,req}$), the determined lung time constant ($\tau$) and the determined dead space volume ($V_D$),
wherein the mandatory setpoint frequency ($f_{set,mand}$) is a frequency with which the intended volumetric flow rate ($V'_{A,req}$, $V'_{P,req}$) is obtained solely by artificial ventilation,

- to calculate an ideal spontaneous breathing frequency ($f_{spon}$) for the own breathing activity of the patient (P) depending on the intended volumetric flow rate ($V'_A$), the determined lung time constant ($\tau$) and the determined dead space volume ($V_D$), wherein the ideal spontaneous breathing frequency ($f_{spon}$) is a frequency with which the patient (P) is able to attain the intended volumetric flow rate ($V'_{A,req}$, $V'_{P,req}$) solely by their own breathing activity,
- to determine a measure ($\eta_{spon}$ / $\eta_{ges}$) for the present actual intensity of the own breathing activity of the patient (P),
wherein the measure ($\eta_{spon}$ / $\eta_{ges}$) of the intensity of the own breathing activity indicates what absolute or relative contribution to the total inspiration and expiration of the patient's lungs is made by the patient's own breathing activity, and
- to calculate the setpoint ventilation frequency ($f_{set}$) as a weighted average of the mandatory setpoint frequency ($f_{set,mand}$) and the ideal spontaneous breathing frequency ($f_{spon}$) such that in the averaging a weight factor (SML) depends on the determined measure ($\eta_{spon}$ / $\eta_{ges}$) of the own breathing activity,
- wherein the weight factor (SML) with which the ideal spontaneous breathing frequency ($f_{spon}$) influences the setpoint ventilation frequency ($f_{set}$) is all the greater, the greater the measure ($\eta_{spon}$ / $\eta_{ges}$) of the present actual intensity of the own breathing activity of the patient (P).

**2.** Signal processing unit (10) according to Claim 1,
**characterized in that**

a computer-evaluable definition of a required inhalation proportion (D1) is stored in the data memory (11), wherein the inhalation proportion (D1) correlates with that temporal proportion of an entire ventilation process which is constituted by an inhalation process, and/or with the ratio between the duration ($T_i$) of an inhalation process and the duration ($T_e$) of a respiration process,
wherein the signal processing unit (10) is configured, when calculating the mandatory setpoint frequency ($f_{set,mand}$),

- to calculate an upper limit ($f_{set,mand,max}$) for the mandatory setpoint frequency ($f_{set,mand}$) depending on the determined lung time constant ($\tau$),
- to calculate an ideal mandatory setpoint frequency ($f_{set,mand,id}$) depending on the predefined required inhalation proportion (D1), and
- to calculate the mandatory setpoint frequency ($f_{set,mand}$) using the ideal mandatory setpoint frequency ($f_{set,mand,id}$) such that the mandatory setpoint frequency ($f_{set,mand}$) is less than or equal to the upper limit ($f_{set,mand,max}$) for the mandatory setpoint frequency ($f_{set,mand}$),

wherein the signal processing unit (10) is preferably configured to calculate the mandatory setpoint frequency ($f_{set,mand}$) as the minimum of ideal mandatory setpoint frequency ($f_{set,mand,id}$) and upper limit ($f_{set,mand,max}$).

**3.** Signal processing unit (10) according to Claim 2,
**characterized in that**

a computer-evaluable lung model is stored in the data memory (11),
wherein the lung model approximately describes the pneumatic behaviour of the lungs (Lu) of a person (P),
wherein the signal processing unit (10) is configured to calculate the ideal mandatory setpoint frequency ($f_{set,mand,id}$) depending on the required inhalation proportion (D1) and additionally depending on a resistive power ($W'_R$) and an elastic power ($W'_c$),
wherein the resistive power ($W'_R$) is the power which is to be expended according to the lung model during a mandatory ventilation with said ideal mandatory setpoint frequency ($f_{set,mand,id}$), and
wherein the elastic power ($W'_c$) is the power which is to be expended according to the lung model during a mandatory ventilation with said frequency ($f_{set,mand,id}$) in order to expand the lungs, and
wherein the signal processing unit (10) is preferably configured to predict the resulting resistive power and the resulting elastic power for at least one possible ideal mandatory setpoint frequency ($f_{set,mand,id}$).

**4.** Signal processing unit (10) according to Claim 3,
**characterized in that**

a power factor ($1+\alpha$) is stored in the data memory (11)
wherein the power factor ($1+\alpha$) is greater than or equal to 1 and preferably less than 1.2, and
wherein the signal processing unit (10) is configured to calculate the ideal mandatory setpoint frequency ($f_{set,mand,id}$) such that during a mandatory ventilation with said frequency ($f_{set,mand,id}$) the ratio of

- the resistive power ($W'_R$) which is to be expended according to the lung model during the mandatory ventilation and
- the elastic power ($W'_C$) which is to be expended according to the lung model during the mandatory ventilation

is equal to the predefined power factor ($1+\alpha$).

**5.** Signal processing unit (10) according to Claim 4,
**characterized in that**

the signal processing unit (10) is configured
to calculate a constant in an initialization phase using the lung model and the optional power factor ($1+\alpha$), and
to calculate the ideal mandatory setpoint frequency ($f_{set,mand,id}$) in a subsequent use phase, and

to use

- the predefined inhalation proportion (D1),
- the determined lung time constant ($\tau$) and
- the constant calculated in the initialization phase

for the calculation of the ideal mandatory setpoint frequency ($f_{set,mand,id}$).

**6.** Signal processing unit (10) according to any of Claims 2 to 5,
**characterized in that**
the signal processing unit (10) is configured to calculate the ideal mandatory setpoint frequency ($f_{set,mand,id}$) such that the ideal mandatory setpoint frequency ($f_{set,mand,id}$) is all the greater, the greater the predefined required inhalation proportion (D1).

**7.** Signal processing unit (10) according to any of Claims 2 to 6,
**characterized in that**

the signal processing unit (10) is configured to calculate the upper limit ($f_{set,mand,max}$) for the mandatory setpoint frequency ($f_{set,mand}$)

- depending on the determined lung time constant ($\tau$) and
- additionally depending on the intended volumetric flow rate ($V'_{A,req}$) and/or depending on the determined dead space volume ($V_D$),

wherein the signal processing unit (10) is preferably configured to calculate the upper limit ($f_{set,mand,max}$) depending on

- the determined lung time constant ($\tau$) and
- the quotient of the intended volumetric flow ($V'_{A,req}$) and the dead space volume ($V_D$).

**8.** Signal processing unit (10) according to any of the preceding claims,
**characterized in that**

the signal processing unit (10) is configured
to calculate the mandatory setpoint frequency ($f_{set,mand}$) for the mandatory ventilation such that the mandatory setpoint frequency ($f_{set,mand}$) is greater than or equal to the ideal spontaneous breathing frequency ($f_{spon}$) for the own breathing activity of the patient (P).

**9.** Signal processing unit (10) according to any of the preceding claims,
**characterized in that**

the signal processing unit (10) is configured to determine what proportion of the total work ($WOB_{Pat} + WOB_{Vent}$) or power applied during an inhaled breath is constituted by the mechanical work ($WOB_{Pat}$) or the mechanical power produced by the own breathing activity of the patient (P) during the inhaled breath,
wherein the signal processing unit (10) is furthermore configured to determine the measure ($\eta_{spon} / \eta_{ges}$) of the intensity of the own breathing activity of the patient (P) depending on said proportion, preferably to use that proportion as the measure ($\eta_{spon} / \eta_{ges}$).

**10.** Signal processing unit (10) according to any of the preceding claims,
**characterized in that**

the signal processing unit (10) is configured
to calculate a setpoint tidal volume ($Vol_{Tid,set}$) depending on

- the intended volumetric flow rate ($V'_{A,req}$, $V'_{P,req}$),
- the determined dead space volume ($V_D$) and
- the calculated setpoint ventilation frequency ($f_{set}$),

wherein the setpoint tidal volume ($Vol_{Tid,set}$) is a value for the volume which the ventilator (1) is intended to feed into the fluid connection during a ventilation stroke, and
wherein the ventilator (1) is configured to perform at least one ventilation stroke, preferably each ventilation stroke of a sequence of ventilation strokes, under closed-loop control or open-loop control such that the aim of the closed-loop control or open-loop control is that the tidal volume ($Vol_{Tid,ist}$)actually generated during said ventilation stroke is equal to the calculated setpoint tidal volume ($Vol_{Tid,set}$),
wherein preferably the signal processing unit (10) is configured to carry out the closed-loop control or open-loop control for the actually generated tidal volume ($Vol_{Tid,ist}$)depending on the calculated setpoint tidal volume ($Vol_{Tid,set}$) .

**11.** Signal processing unit (10) according to any of the preceding claims,
**characterized in that**

the signal processing unit (10) is configured
to calculate a required setpoint ramp time ($t_{R,set}$) for a ventilation stroke,
that is to say the period of time which elapses from the beginning of a ventilation stroke until the maximum pressure is reached with which the ventilator (1) performs the ventilation stroke,
wherein the ventilator (1) is configured to perform at least one ventilation stroke, preferably each ventilation stroke of a sequence of ventilation strokes, under closed-loop control or open-loop control such that the aim of the closed-loop control or open-loop control is that the ramp time ($t_{R,ist}$) actually attained during said ventilation stroke is equal to the calculated required setpoint ramp time ($t_{R,set}$),
wherein preferably the signal processing unit (10) is configured to carry out the closed-loop control or open-loop control for the actually attained ramp time ($t_{R,ist}$) depending on the calculated setpoint ramp time ($t_{R,set}$).

**12.** Ventilator (1) for artificial ventilation of a patient (P),

wherein a fluid connection is establishable or is established between the lungs (Lu) of a patient (P) to be artificially ventilated and the ventilator (1),
wherein the ventilator (1) is connectable or is connected at least at times to at least one patient sensor (2, 3, 7) which is able to measure at least one respiratory parameter of the patient (P),
wherein the ventilator (1) comprises a data memory (11) or has read access to a data memory (11) at least at times,
wherein a measure of an intended volumetric flow rate into the lungs (Lu) of the patient (P), in particular a required alveolar minute volume ($V'_{A,req}$) or a required proximal minute volume ($V'_{P,req}$), is stored in the data memory (11) in a computer-evaluable form,
wherein the ventilator (1) is configured

- to calculate a setpoint ventilation frequency ($f_{set}$), and
- to perform a sequence of ventilation strokes into the fluid connection depending on the calculated setpoint ventilation frequency ($f_{set}$),

preferably to perform the ventilation strokes with the calculated setpoint ventilation frequency ($f_{set}$),
wherein the ventilator (1) is configured

- to receive measured values from at least one patient sensor (2, 3, 7),
wherein the or each patient sensor (2, 3, 7) is able to measure in each case at least one respiratory parameter of the patient (P),
- to determine a lung time constant ($\tau$) for the lungs of the patient (P) depending on the measured values of the or at least one patient sensor (2, 3, 7),
- to determine the volume ($V_D$) of a dead space in the fluid connection,
wherein said dead space occurs in the fluid connection between the ventilator (1) and a region of the lungs (Lu) of the patient (P) that is suitable for gas exchange,
- to calculate a mandatory setpoint frequency ($f_{set,mand}$) for the mandatory ventilation of the patient (P) by the ventilator (1)

depending on the stored intended volumetric flow rate ($V'_{A,req}$, $V'_{P,req)}$, the determined lung time constant ($\tau$) and the determined dead space volume ($V_D$),
wherein the mandatory setpoint frequency ($f_{set,mand}$) is a frequency with which the intended volumetric flow rate ($V'_{A,req}$, $V'_{P,req}$) is obtained solely by artificial ventilation,

- to calculate an ideal spontaneous breathing frequency ($f_{spon}$) for the own breathing activity of the patient (P) depending on the intended volumetric flow rate ($V'_A$), the determined lung time constant ($\tau$) and the determined dead space volume ($V_D$), wherein the ideal spontaneous breathing frequency ($f_{spon}$) is a frequency with which the patient (P) is able to attain the intended volumetric flow rate ($V'_{A,req}$, $V'_{P,req}$) solely by their own breathing activity,
- to determine a measure ($\eta_{spon}$ / $\eta_{ges}$) for the present actual intensity of the own breathing activity of the patient (P),
wherein the measure ($\eta_{spon}$ / $\eta_{ges}$) of the intensity of the own breathing activity indicates what absolute or relative contribution to the total inspiration and expiration of the patient's lungs is made by the patient's own breathing activity, and
- to calculate the setpoint ventilation frequency ($f_{set}$) as a weighted average of the mandatory setpoint frequency ($f_{set,mand}$) and the ideal spontaneous breathing frequency ($f_{spon}$) such that in the averaging a weight factor (SML) depends on the determined measure ($\eta_{spon}$ / $\eta_{ges}$) of the own breathing activity,
- wherein the weight factor (SML) with which the ideal spontaneous breathing frequency ($f_{spon}$) influences the setpoint ventilation frequency ($f_{set}$) is all the greater, the greater the measure ($\eta_{spon}$ / $\eta_{ges}$) of the present actual intensity of the own breathing activity of the patient (P).

**13.** Computer program

which is executable on a signal processing unit (10) and,
during execution on the signal processing unit (10), if the signal processing unit (10) receives measured values from at least one patient sensor (2, 3, 7) which is able to measure at least one respiratory parameter of the patient (P),
causes the signal processing unit (10) to carry out a method for automatically calculating a setpoint ventilation frequency ($f_{set}$) for a ventilator (1),
wherein the ventilator (1) is configured, if a fluid connection is established or is establishable between the lungs (Lu) of a patient (P) to be artificially ventilated and the ventilator (1),
to perform a sequence of ventilation strokes into the fluid connection depending on the calculated setpoint ventilation frequency ($f_{set}$),
preferably with the calculated setpoint ventilation frequency ($f_{set}$), and
wherein the computer program causes the method to comprise the steps that

- a measure of an intended volumetric flow rate into the lungs (Lu) and out of the lungs (Lu) of the patient (P),

in particular a required alveolar minute volume ($V'_{A,req}$) or a required proximal minute volume ($V'_{P,req}$), is predefined,

- a lung time constant ($\tau$) for the lungs (Lu) of the patient (P) is determined depending on measured values of at least one respiratory parameter of the patient (P),
- the volume ($V_D$) of a dead space in the fluid connection is determined,

wherein said dead space occurs in the fluid connection between the ventilator (1) and a region of the lungs (Lu) of the patient (P) that is suitable for gas exchange,

- a mandatory setpoint frequency ($f_{set,mand}$) for the mandatory ventilation of the patient (P) by the ventilator (1) is calculated depending on the intended volumetric flow rate ($V'_{A,req}$, $V'_{P,req}$), the determined lung time constant ($\tau$) and the determined dead space volume ($V_D$),

wherein the mandatory setpoint frequency ($f_{set,mand}$) is a frequency with which the intended volumetric flow rate ($V'_{A,req}$, $V'_{P,req}$) is attained solely by artificial ventilation,

- an ideal spontaneous breathing frequency ($f_{spon}$) for the own breathing activity of the patient (P) is calculated depending on the intended volumetric flow rate ($V'_A$), the determined lung time constant ($\tau$) and the determined dead space volume ($V_D$),
wherein the ideal spontaneous breathing frequency ($f_{spon}$) is a frequency with which the patient (P) is able to attain the intended volumetric flow rate ($V'_{A,req}$, $V'_{P,req}$) solely by their own breathing activity,

- a measure ($\eta_{spon}$ / $\eta_{ges}$) for the present actual intensity of the own breathing activity of the patient (P) is determined,

wherein the measure ($\eta_{spon}$ / $\eta_{ges}$) of the intensity of the own breathing activity indicates what absolute or relative contribution to the total inspiration and expiration of the patient's lungs is made by the patient's own breathing activity, and

- the setpoint ventilation frequency ($f_{set}$) as a weighted average of the mandatory setpoint frequency ($f_{set,mand}$) and the ideal spontaneous breathing frequency ($f_{spon}$) is calculated
- wherein in the averaging a weight factor (SML) depends on the determined measure ($\eta_{spon}$ / $\eta_{ges}$) of the own breathing activity, and
- wherein the weight factor (SML) with which the ideal spontaneous breathing frequency ($f_{spon}$) influences the setpoint ventilation frequency ($f_{set}$) is all the greater, the greater the measure ($\eta_{spon}$ / $\eta_{ges}$) of the present actual intensity of the own breathing activity of the patient (P).

## Revendications

**1.** Unité de traitement de signal (10), qui est conçue pour calculer automatiquement une fréquence de ventilation de consigne ($f_{set}$) pour un respirateur (1),

une liaison fluidique entre les poumons (Lu) d'un patient (P) sous ventilation artificielle et le respirateur (1) pouvant être établie ou étant établie,

le respirateur (1) étant conçu pour exécuter une séquence de courses de ventilation dans la liaison fluidique en fonction de la fréquence de ventilation de consigne calculée ($f_{set}$),

l'unité de traitement de signal (10) comprenant une mémoire de données (11) ou présentant au moins temporairement un accès en lecture à une mémoire de données (11),

dans la mémoire de données (11), sous une forme pouvant être évaluée par un ordinateur, une mesure du débit volumique souhaité dans les poumons (Lu) du patient (P),

en particulier un volume par minute alvéolaire exigé ($V'_{A,req}$) ou un volume par minute proximal exigé ($V'_{p,req}$), est mémorisée,

l'unité de traitement de signal (10) étant conçue pour

- recevoir des valeurs mesurées par au moins un capteur de patient (2, 3, 7), le ou chaque capteur de patient (2, 3, 7) étant capable de mesurer au moins un paramètre respiratoire du patient (P),
- en fonction des valeurs mesurées par le ou au moins un capteur de patient (2, 3, 7), déterminer une constante de temps pulmonaire ($\tau$) pour les poumons du patient (P),
- déterminer le volume ($V_D$) d'un espace mort dans la liaison fluidique, cet espace mort se produisant entre le respirateur (1) et une zone des poumons (Lu) du patient (P) appropriée pour un échange de gaz,
- en fonction du débit volumique souhaité mémorisé ($V'_{A,req}$, $V'_{P,req}$), de la constante de temps pulmonaire déterminée ($\tau$) et du volume d'espace mort déterminé ($V_D$), calculer une fréquence de consigne obligatoire ($f_{set,mand}$) pour la ventilation obligatoire du patient (P) par le respirateur (1),
la fréquence de consigne obligatoire ($f_{set,mand}$) étant une fréquence à laquelle le débit volumique souhaité ($V'_{A,req}$, $V'_{P,req}$) est atteint uniquement par ventilation artificielle,
- en fonction du débit volumique souhaité ($V'_A$), de la constante de temps pulmonaire déterminée ($\tau$) et du volume d'espace mort déterminé ($V_D$), calculer une fréquence de respiration spontanée idéale ($f_{spon}$) pour l'activité respiratoire propre du patient (P),
la fréquence de respiration spontanée idéale ($f_{spon}$) étant une fréquence à laquelle le patient (P) peut atteindre le débit volumique souhaité ($V'_{A,req}$, $V'_{P,req}$) uniquement par son activité respiratoire propre,
- déterminer une mesure ($n_{spon}/n_{ges}$) de l'intensité réelle actuelle de la propre activité respiratoire du patient (P),
la mesure ($n_{spon}/n_{ges}$) de l'intensité de l'activité respiratoire propre indiquant la contribution absolue ou relative de l'activité respiratoire propre du patient à l'ensemble de la ventilation et de l'aération des poumons du patient, et
- calculer la fréquence de ventilation de consigne ($f_{set}$) en tant que moyenne pondérée de la fréquence de consigne obligatoire ($f_{set,mand}$) et de la fréquence de ventilation spontanée idéale ($f_{spon}$),
de telle sorte que lors de l'établissement de la moyenne, un facteur de pondération (SML) dépend de la mesure déterminée ($n_{spon}/n_{ges}$) pour l'activité respiratoire propre,
- le facteur de pondération (SML) avec lequel la fréquence de respiration spontanée idéale ($f_{spon}$) entre dans la fréquence de ventilation souhaité ($f_{set}$) étant d'autant plus important que la mesure ($n_{spon}/n_{ges}$) de

l'intensité réelle actuelle de l'activité respiratoire propre du patient (P) est important.

**2.** Unité de traitement de signal (10) selon la revendication 1,
**caractérisée en ce que**

dans la mémoire de données (11) est mémorisée une détermination pouvant être évaluée par ordinateur d'une proportion d'inspiration exigée (D1),
la proportion d'inhalation (D1) est corrélée à la proportion temporelle d'une procédure d'inhalation dans une procédure de ventilation totale et/ou au rapport entre la durée ($T_i$) d'une procédure d'inhalation et la durée ($T_e$) d'une procédure respiratoire,
l'unité de traitement de signal (10) étant conçue pour être utilisée lors du calcul de la fréquence de consigne obligatoire ($f_{set,mand}$),

- calculer, en fonction de la constante de temps pulmonaire ($\tau$) déterminée, une barrière supérieure ($f_{set, mand,max}$) pour la fréquence de consigne obligatoire ($f_{set,mand}$),
- calculer une fréquence de consigne obligatoire idéale ($f_{set, mand, id}$) en fonction de la proportion d'inhalation exigée (D1) prédéterminée, et
- calculer ainsi la fréquence de consigne obligatoire ($f_{set,mand}$) en utilisant la fréquence de consigne obligatoire idéale ($f_{set,mand,id}$),

de telle sorte que la fréquence de consigne obligatoire ($f_{set,mand}$) est inférieure ou égale
à la limite supérieure ($f_{set,mand,max}$) de la fréquence de consigne obligatoire ($f_{set,mand}$),
l'unité de traitement de signal (10) étant conçue de préférence pour calculer la fréquence de consigne obligatoire ($f_{set,mand}$) en tant que minimum d'après la fréquence de consigne obligatoire idéale ($f_{set,mand,id}$) et la barrière supérieure ($f_{set,mand,max}$).

**3.** Unité de traitement de signal (10) selon la revendication 2,
**caractérisée en ce que**

un modèle pulmonaire exploitable par ordinateur est mémorisé dans la mémoire de données (11),
le modèle pulmonaire décrit approximativement le comportement pneumatique du poumon (Lu) d'une personne (P),
l'unité de traitement de signal (10) étant conçue pour calculer la fréquence de consigne obligatoire idéale ($f_{set,mand,id}$) en fonction de la proportion d'inspiration exigée (D1) et en outre en fonction d'une puissance résistive ($W'_R$) et d'une puissance élastique (Wc), la puissance résistive ($W'_R$) étant la puissance à utiliser, selon le modèle pulmonaire, lors d'une ventilation obligatoire à cette fréquence de consigne obligatoire idéale ($f_{set,mand,id}$), et
la puissance élastique (Wc) étant la puissance à utiliser selon le modèle pulmonaire lors d'une ventilation obligatoire à cette fréquence ($f_{set,mand,id}$) pour dilater les poumons, et
l'unité de traitement de signal (10) étant conçue de préférence pour prédire la puissance résistive résultante et la puissance élastique résultante pour au moins une fréquence de consigne obligatoire idéale possible ($f_{set,mand,id}$).

**4.** Unité de traitement de signal (10) selon la revendication 3,
**caractérisée en ce que**

un facteur de puissance ($1+\alpha$) est mémorisé dans la mémoire de données (11),
le facteur de puissance ($1+\alpha$) étant supérieur ou égal à 1 et de préférence inférieur à 1,2, et
l'unité de traitement de signal (10) étant conçue pour calculer la fréquence de consigne obligatoire idéale ($f_{set,mand,id}$) de telle sorte qu'en
cas de ventilation obligatoire à cette fréquence ($f_{set,mand,id}$), le rapport

- de la puissance résistive ($W'_R$) à utiliser lors de la ventilation obligatoire selon le modèle pulmonaire, et
- de la puissance élastique (Wc) à utiliser lors de la ventilation obligatoire selon le modèle pulmonaire,

est égal au facteur de puissance prédéterminé ($1+\alpha$).

**5.** Unité de traitement de signal (10) selon la revendication 4,

**caractérisée en ce que**

l'unité de traitement du signal (10) est conçue pour
calculer une constante dans une phase d'initialisation à l'aide du modèle pulmonaire et du facteur de puissance facultatif ($1+\alpha$), et
calculer la fréquence de consigne obligatoire idéale ($f_{set,mand,id}$) dans une phase d'exploitation ultérieure, et pour le calcul de la fréquence de consigne obligatoire idéale ($f_{set,mand,id}$), utiliser

- la proportion d'inhalation prédéterminée (D1),
- la constante de temps pulmonaire déterminée ($\tau$), et
- utiliser la constante calculée dans la phase d'initialisation.

**6.** Unité de traitement de signal (10) selon l'une quelconque des revendications 2 ou 5,
**caractérisée en ce que**

l'unité de traitement du signal (10) est conçue pour calculer la fréquence de consigne obligatoire idéale ($f_{set,mand,\ id}$),
de telle sorte que la fréquence de consigne obligatoire idéale ($f_{set,mand,id}$) est d'autant plus importante que la proportion d'inhalation exigée (D1) est importante.

**7.** Unité de traitement de signal (10) selon l'une quelconque des revendications 2 à 6, **caractérisée en ce que**

l'unité de traitement du signal (10) est conçue pour
calculer la barrière supérieure ($f_{set,mand,max}$) pour la fréquence de consigne obligatoire ($f_{set,mand}$)

- en fonction de la constante de temps pulmonaire déterminée ($\tau$) et
- de plus en fonction du débit volumique visé ($V'_{A,req}$) et/ou en fonction du volume d'espace mort déterminé ($V_D$),

l'unité de traitement de signal (10) étant conçue de préférence pour calculer la barrière supérieure ($f_{set,mand,max}$) en fonction de
- la constante de temps pulmonaire déterminée ($\tau$), et
- du quotient du débit volumique visé ($V'_{A,req}$) et du volume d'espace mort ($V_D$).

**8.** Unité de traitement de signal (10) selon l'une quelconque des revendications précédentes,
**caractérisée en ce que**

l'unité de traitement du signal (10) est conçue pour
calculer la fréquence de consigne obligatoire ($f_{set,mand}$) pour la ventilation obligatoire, de sorte que la fréquence de consigne obligatoire ($f_{set,mand}$) est supérieure ou égale à la fréquence de respiration spontanée idéale ($f_{spon}$) pour l'activité respiratoire propre du patient (P).

**9.** Unité de traitement de signal (10) selon l'une quelconque des revendications précédentes,
**caractérisée en ce que**
l'unité de traitement du signal (10) est conçue pour déterminer :

quelle est la proportion du travail mécanique ($WOB_{Pat}$) ou de la puissance mécanique, qui fournit l'activité respiratoire propre du patient (P) pendant une inhalation,
sur l'ensemble du travail ou de la puissance appliqué(e) pendant l'inhalation ($WOB_{Pat}+WOB_{Vent}$),
l'unité de traitement du signal (10) étant en outre conçue pour déterminer la mesure ($n_{spon}/n_{ges}$) de l'intensité de l'activité respiratoire propre du patient (P) en fonction de cette proportion,
cette proportion devant de préférence être utilisée comme mesure ($n_{spon}/n_{ges}$).

**10.** Unité de traitement de signal (10) selon l'une quelconque des revendications précédentes,
**caractérisée en ce que**

l'unité de traitement du signal (10) est conçue pour
en fonction

- du débit volumique visé ($V'_{A,req}$, $V'_{P,req}$),
- du volume d'espace mort déterminé ($V_D$), et
- de la fréquence de ventilation de consigne calculée ($f_{set}$), calculer un volume courant de consigne ($Vol_{Tid,set}$)

le volume courant de consigne ($Vol_{Tid,set}$) étant une valeur pour le volume que le respirateur (1) doit injecter dans la liaison fluidique lors d'une course de ventilation, et le respirateur (1) étant conçu pour effectuer au moins une course de ventilation, de préférence chaque course de ventilation d'une séquence de courses de ventilation, de manière régulée ou commandée,
de telle sorte que le but de la régulation ou de la commande est que le volume courant effectivement généré par cette course de ventilation ($Vol_{Tid,est}$) soit égal au volume courant de consigne calculé ($Vol_{Tid,set}$),
l'unité de traitement de signal (10) étant conçue de préférence pour effectuer la régulation ou la commande du volume courant effectivement généré ($Vol_{Tid,ist}$) en fonction du volume courant de consigne calculé ($Vol_{Tid,set}$).

**11.** Unité de traitement de signal (10) selon l'une quelconque des revendications précédentes,
**caractérisée en ce que**

l'unité de traitement du signal (10) est conçue pour
calculer un temps de rampe de consigne exigé ($t_{R,set}$) pour une course de ventilation, à savoir le temps qui s'écoule depuis le début d'une course de ventilation jusqu'à ce que la pression maximale à laquelle le respirateur (1) exécute la course de ventilation soit atteinte,
le respirateur (1) étant conçu pour effectuer au moins une course de ventilation, de préférence chaque course de ventilation d'une séquence de courses de ventilation, de manière régulée ou commandée,
de telle sorte que le but de la régulation ou de la commande est que le temps de rampe effectivement atteint à cette course de ventilation ($t_{R,ist}$) soit égal au temps de rampe de consigne exigé calculé ($t_{R,set}$),
l'unité de traitement de signal (10) étant conçue de préférence pour effectuer la régulation ou la commande pour que le temps de rampe ($t_{R,ist}$) soit effectivement atteint en fonction du temps de rampe de consigne calculé ($t_{R,set}$).

**12.** Respirateur (1) pour la ventilation artificielle d'un patient (P),

une liaison fluidique pouvant être établie ou étant établie entre les poumons (Lu) d'un patient (P) devant être ventilé artificiellement et le respirateur (1),
le respirateur (1) pouvant être relié ou étant relié au moins temporairement à au moins un capteur de patient (2, 3, 7) qui est capable de mesurer au moins un paramètre respiratoire du patient (P),
le respirateur (1) comprenant une mémoire de données (11) ou présentant au moins temporairement un accès en lecture à une mémoire de données (11),
la mémoire de données (11) stockant, sous une forme pouvant être évaluée par un ordinateur, une mesure d'un débit volumique souhaité dans les poumons (Lu) du patient (P), en particulier un volume par minute alvéolaire exigé ($V'_{A,req}$) ou un volume par minute proximal exigé ($V'_{P,req}$),
le respirateur (1) étant conçu pour

- calculer une fréquence de ventilation de consigne ($f_{set}$), et
- en fonction de la fréquence de ventilation de consigne calculée ($f_{set}$), effectuer une séquence de courses de ventilation dans la liaison fluidique, de préférence effectuer les courses de ventilation à la fréquence de ventilation de consigne calculée ($f_{set}$),
le respirateur (1) étant conçu pour
- recevoir les valeurs mesurées par au moins un capteur de patient (2, 3, 7),
le ou chaque capteur de patient (2, 3, 7) étant capable de mesurer au moins un paramètre respiratoire du patient (P),
- en fonction des valeurs mesurées par le ou au moins un capteur de patient (2, 3, 7), déterminer une constante de temps pulmonaire ($\tau$) pour les poumons du patient (P),
- déterminer le volume ($V_D$) d'un espace mort dans la liaison fluidique, cet espace mort se produisant dans la liaison fluidique entre le respirateur (1) et une zone des poumons (Lu) du patient (P) appropriée pour un échange de gaz,
- en fonction du débit volumique souhaité mémorisé ($V'_{A,teq}$, $V'_{P,teq}$), de la constante de temps pulmonaire déterminée ($\tau$) et du volume d'espace mort déterminé ($V_D$), calculer une fréquence de consigne obligatoire ($f_{set,mand}$) pour la ventilation obligatoire du patient (P) par le respirateur (1),

la fréquence de consigne obligatoire ($f_{set,mand}$) étant une fréquence à laquelle le débit volumique souhaité ($V'_{A,req}$, $V'_{P,req}$) est atteint uniquement par ventilation artificielle,
- en fonction du débit volumique souhaité ($V'_A$), de la constante de temps pulmonaire déterminée ($\tau$) et du volume d'espace mort déterminé ($V_D$), calculer une fréquence de respiration spontanée idéale ($f_{spon}$) pour l'activité respiratoire propre du patient (P),
la fréquence de respiration spontanée idéale ($f_{spon}$) est une fréquence à laquelle le patient (P) peut atteindre le débit volumique souhaité ($V'_{A,req}$, $V'_{P,req}$) uniquement par son activité respiratoire propre,
- déterminer une mesure ($n_{spon}/n_{ges}$) de l'intensité réelle actuelle de l'activité respiratoire propre du patient (P), la mesure ($n_{spon}/n_{ges}$) de l'intensité de l'activité respiratoire propre indiquant la contribution absolue ou relative de l'activité respiratoire propre du patient à l'ensemble de la ventilation et de l'aération des poumons du patient, et
- calculer la fréquence de ventilation de consigne ($f_{set}$) en tant que moyenne pondérée de la fréquence de consigne obligatoire ($f_{set,mand}$) et de la fréquence de ventilation spontanée idéale ($f_{set,mand}$) de telle sorte que lors de l'établissement de la moyenne, un facteur de pondération (SML) dépende de la mesure déterminée ($n_{spon}/n_{ges}$) pour l'activité respiratoire propre,
- le facteur de pondération (SML) avec lequel la fréquence de ventilation spontanée idéale ($f_{spon}$) entre dans la fréquence de ventilation souhaité ($f_{set}$) étant d'autant plus important que la mesure ($n_{spon}/n_{ges}$) de l'intensité réelle actuelle de l'activité respiratoire propre du patient (P) est importante.

**13.** Programme informatique,

qui peut être exécuté sur une unité de traitement de signal (10) et, dans le cas de l'unité de traitement de signal (10), lorsque l'unité de traitement de signal (10) reçoit des valeurs de mesure d'au moins un capteur de patient (2, 3, 7) qui est capable de mesurer au moins un paramètre respiratoire du patient (P), l'unité de traitement de signal (10) effectue un procédé de calcul automatique d'une fréquence de ventilation de consigne ($f_{set}$) pour un respirateur (1),
le respirateur (1) étant conçu pour, lorsqu'une liaison fluidique entre les poumons (Lu) d'un patient (P) devant être ventilé artificiellement et le respirateur (1) est établie ou peut être établie,
en fonction de la fréquence de ventilation de consigne calculée ($f_{set}$), effectuer une séquence de courses de ventilation dans la liaison fluidique,
de préférence avec la fréquence de ventilation de consigne calculée ($f_{set}$), et
le programme informatique faisant en sorte que le procédé comporte les étapes suivantes :

- une mesure est prédéterminée pour un débit volumique souhaité dans les poumons (Lu) et à partir des poumons (Lu) du patient (P), en particulier un volume par minute alvéolaire exigé ($V'_{A,req}$) ou un volume par minute proximal exigé ($V'_{P,teq}$),
- en fonction des valeurs mesurées d'au moins un paramètre respiratoire du patient (P), une constante de temps pulmonaire ($\tau$) est déterminée pour les poumons (Lu) du patient (P),
- le volume ($V_D$) d'un espace mort dans la liaison fluidique est déterminé,
cet espace mort se produisant dans la liaison fluidique entre le respirateur (1) et une zone des poumons (Lu) du patient (P) appropriée pour un échange de gaz,
- en fonction du débit volumique souhaité ($V'_{A,teq}$, $V'_{P,teq}$), de la constante de temps pulmonaire déterminée ($\tau$) et du volume d'espace mort déterminé ($V_D$), une fréquence de consigne obligatoire ($f_{set,mand}$) pour la ventilation obligatoire du patient (P) par le respirateur (1) est calculée,
la fréquence de consigne obligatoire ($f_{set,mand}$) étant une fréquence à laquelle le débit volumique souhaité ($V'_{A,req}$, $V'_{P,req}$) est atteint uniquement par ventilation artificielle,
- en fonction du débit volumique souhaité ($V'_A$), de la constante de temps pulmonaire déterminée ($\tau$) et du volume d'espace mort déterminé ($V_D$), une fréquence de respiration spontanée idéale ($f_{spon}$) est calculée pour l'activité respiratoire propre du patient (P),
la fréquence de respiration spontanée idéale ($f_{spon}$) est une fréquence à laquelle le patient (P) peut atteindre le débit volumique souhaité ($V'_{A,req}$,$V'_{P,req}$) uniquement par son activité respiratoire propre,
- une mesure ($n_{spon}/n_{ges}$) de l'intensité réelle actuelle de l'activité respiratoire propre du patient (P) est déterminée,
la mesure ($n_{spon}/n_{ges}$) de l'intensité de l'activité respiratoire propre indiquant la contribution absolue ou relative de l'activité respiratoire propre du patient à l'ensemble de la ventilation et de l'aération des poumons du patient, et
- la fréquence de ventilation de consigne ($f_{set}$) est calculée en tant que moyenne pondérée de la fréquence de consigne obligatoire ($f_{set,mand}$) et de la fréquence de ventilation spontanée idéale ($f_{spon}$),

dans l’établissement de la moyenne, un facteur de pondération (SML) dépendant de la mesure déterminée ($n_{spon}/n_{ges}$) pour l’activité respiratoire propre, et le facteur de pondération (SML) avec lequel la fréquence de respiration spontanée idéale ($f_{spon}$) entre dans la fréquence de ventilation de consigne ($f_{set}$) est d’autant plus important que la mesure ($n_{spon}/n_{ges}$) de l’intensité réelle actuelle de l’activité respiratoire propre du patient (P) est importante.

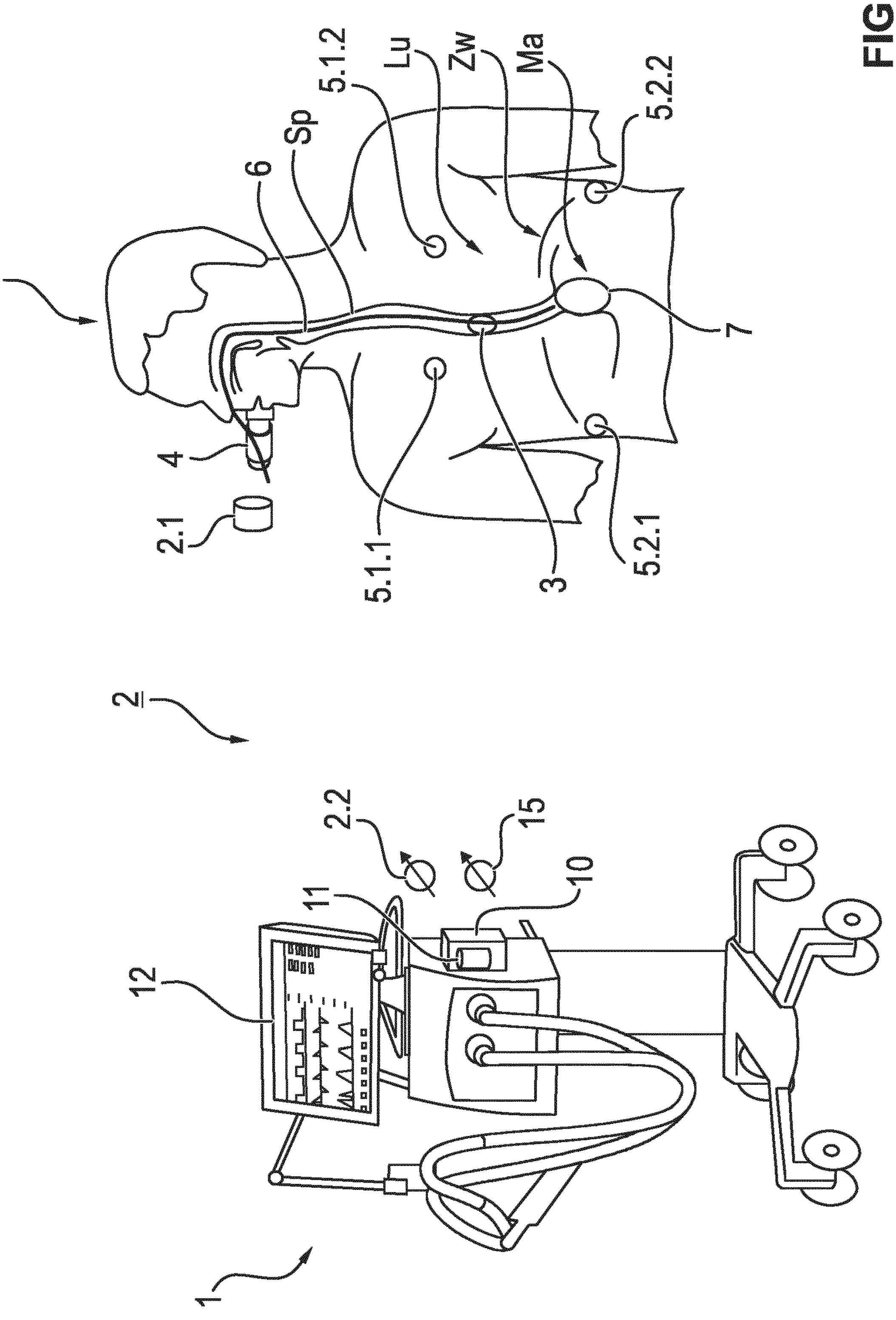


FIG. 1

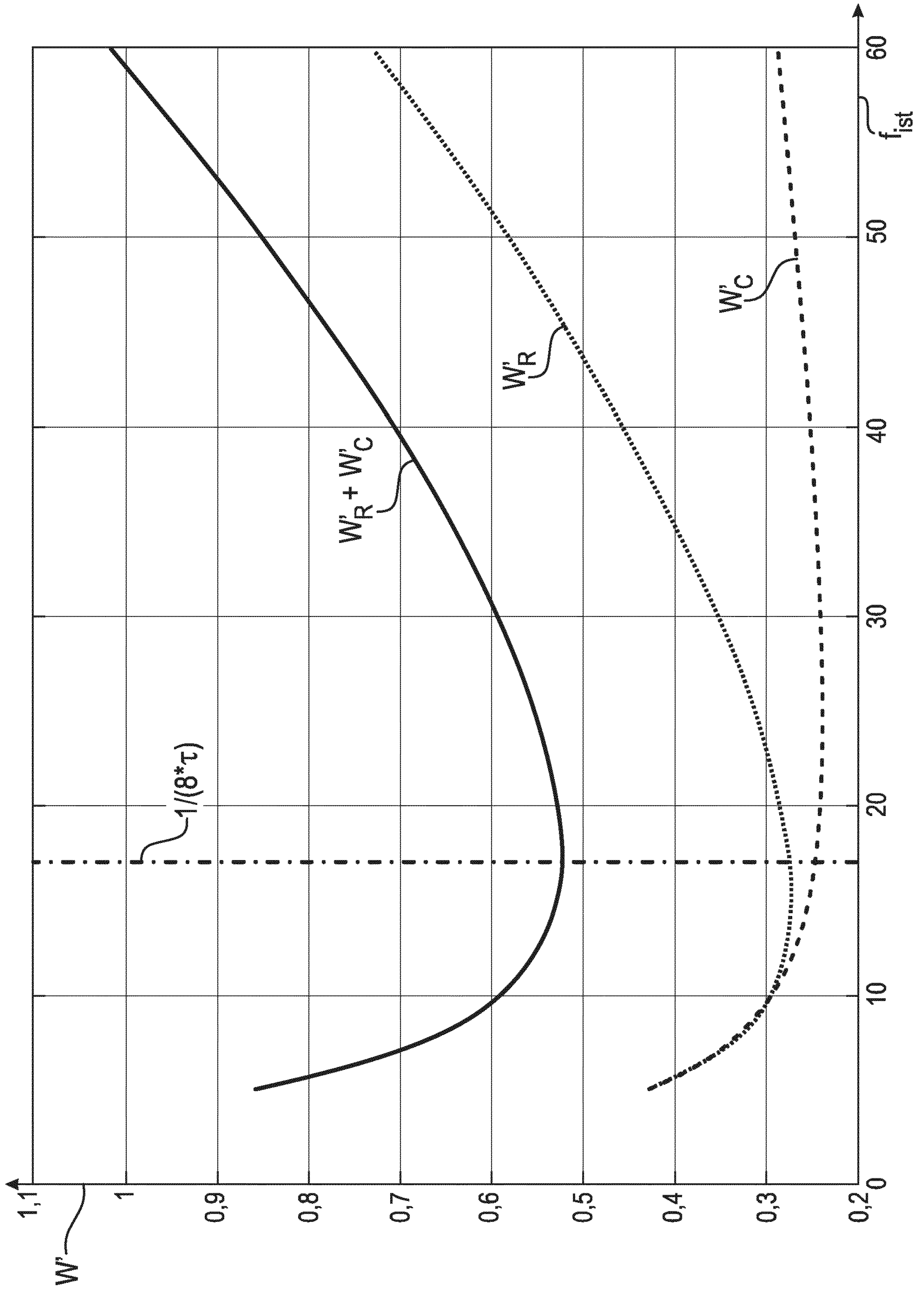


FIG. 2

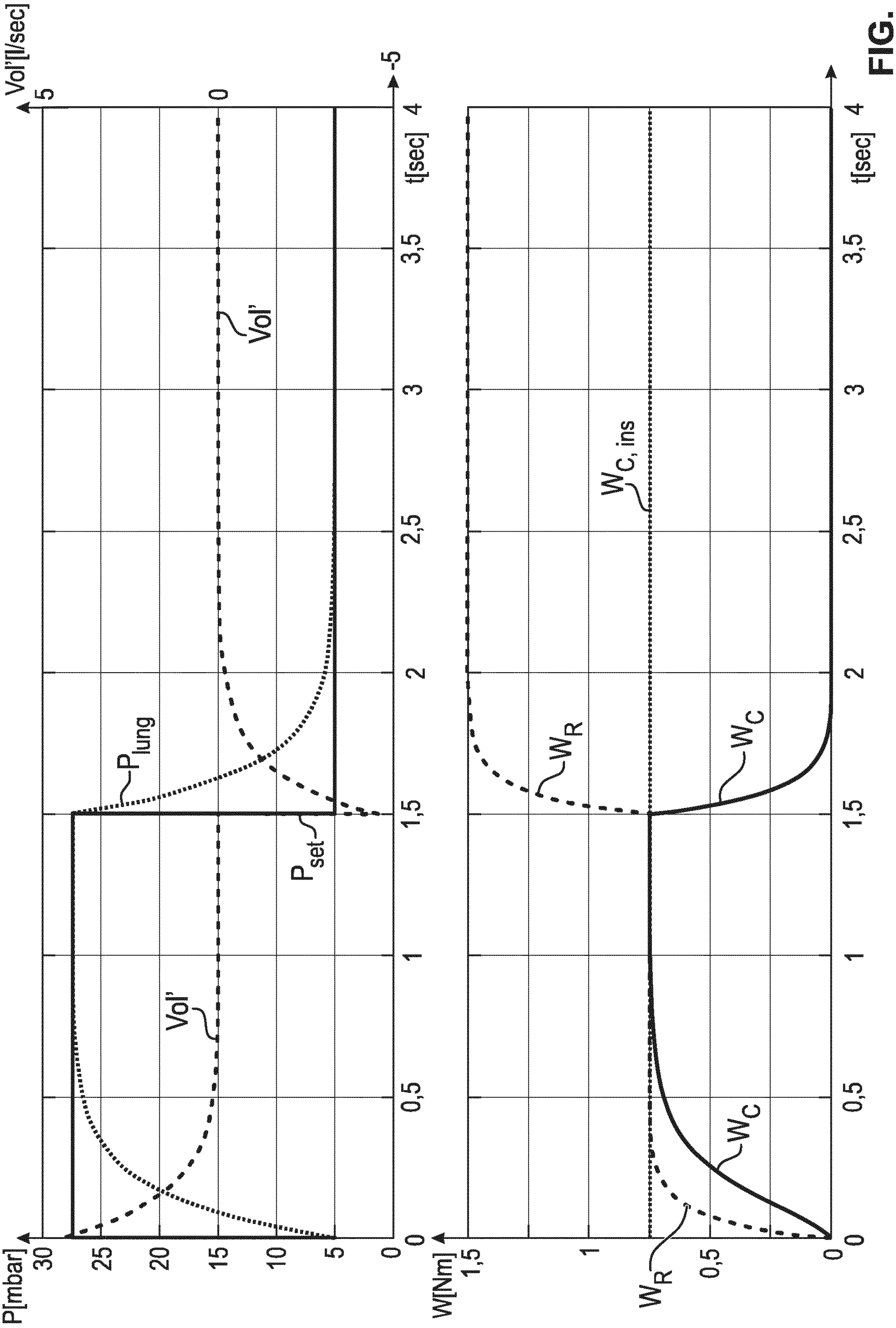


FIG. 3

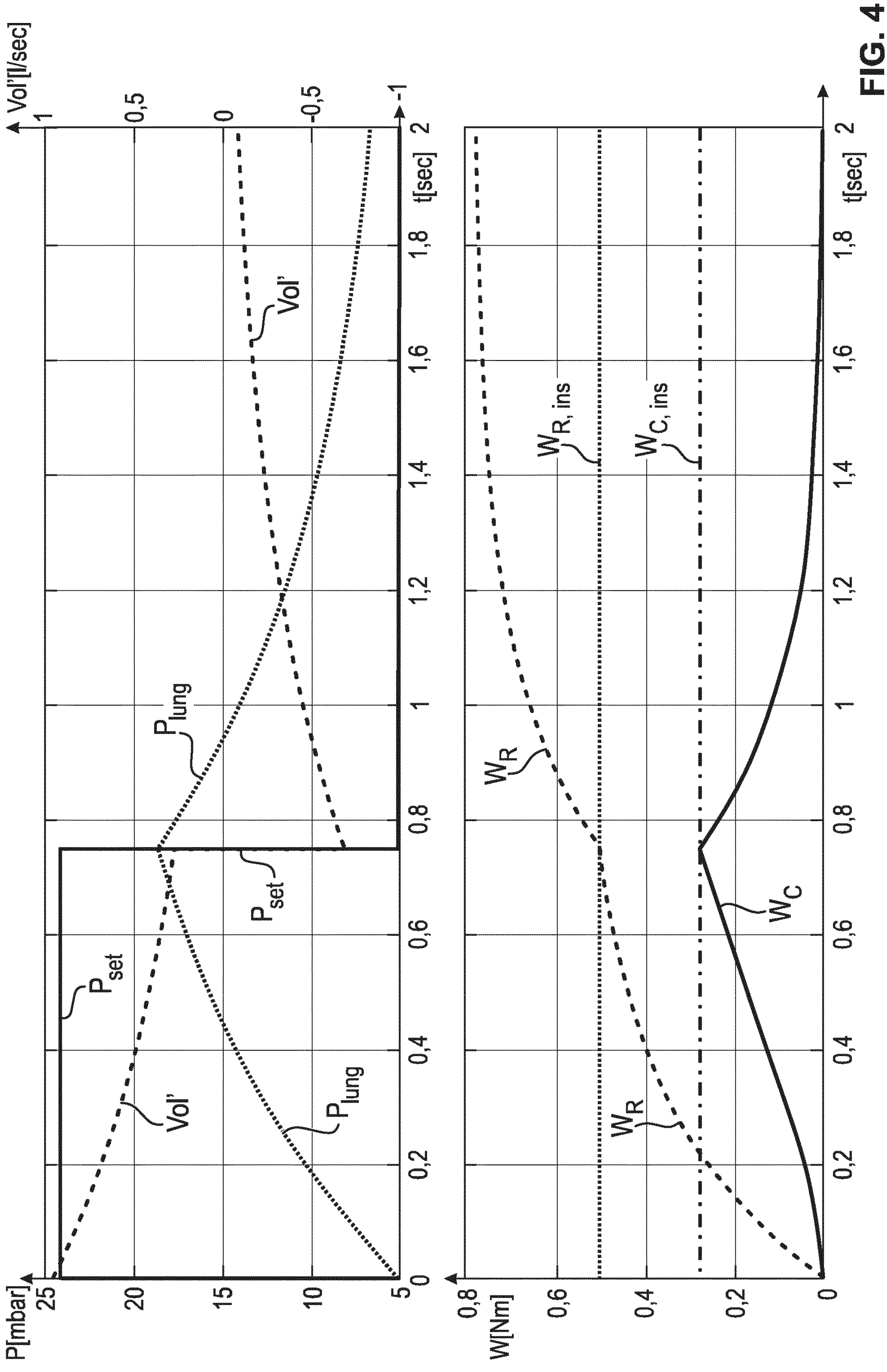
P[mbar]
25
20
15
10
5
Vol'[l/sec]
1
0,5
0
-0,5
-1
t[sec]
0
0,2
0,4
0,6
0,8
1
1,2
1,4
1,6
1,8
2
P_set
Vol'
P_lung
W[Nm]
0,8
0,6
0,4
0,2
W_R
W_C
W_R, ins
W_C, ins

FIG. 4

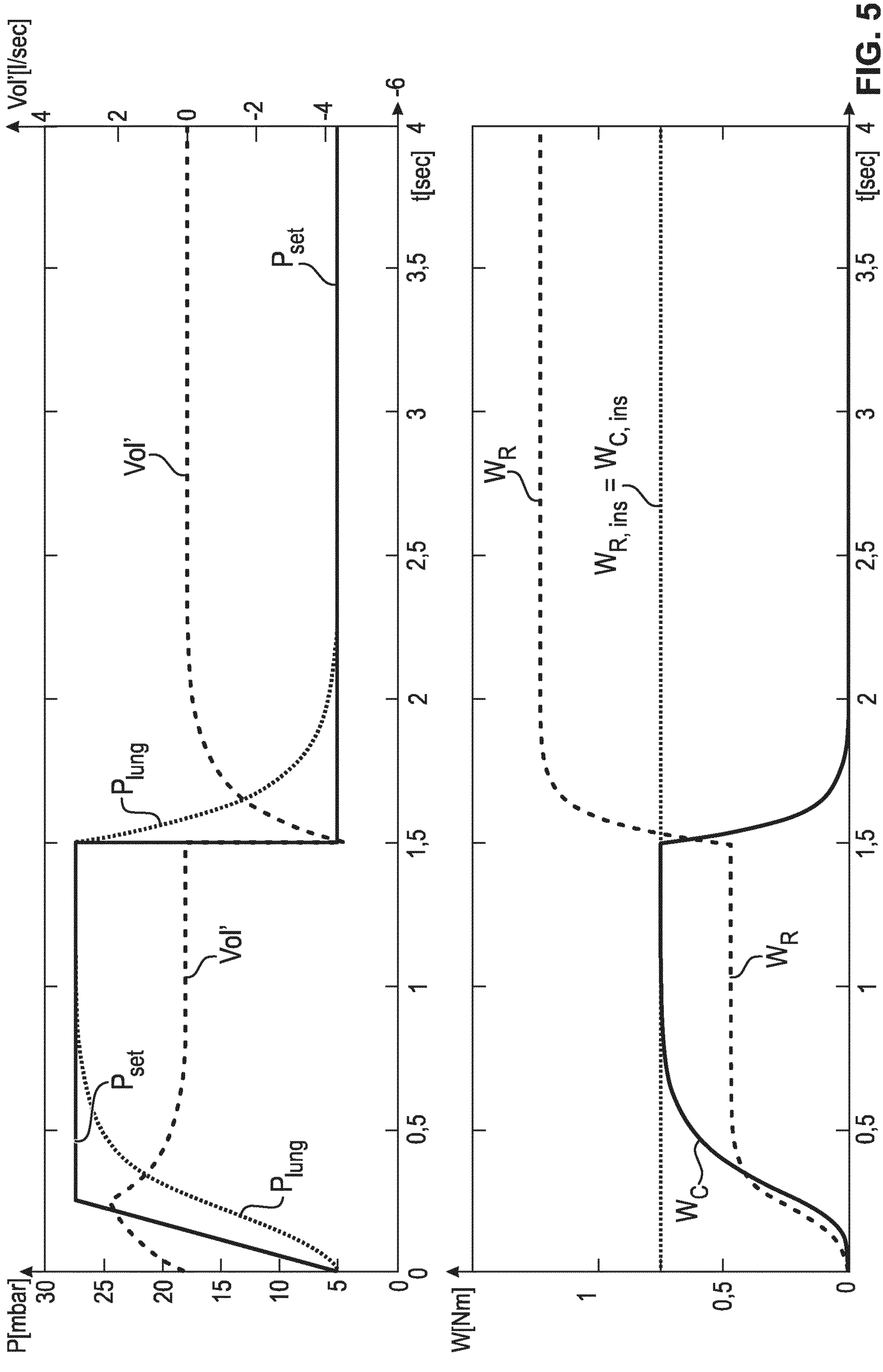


FIG. 5

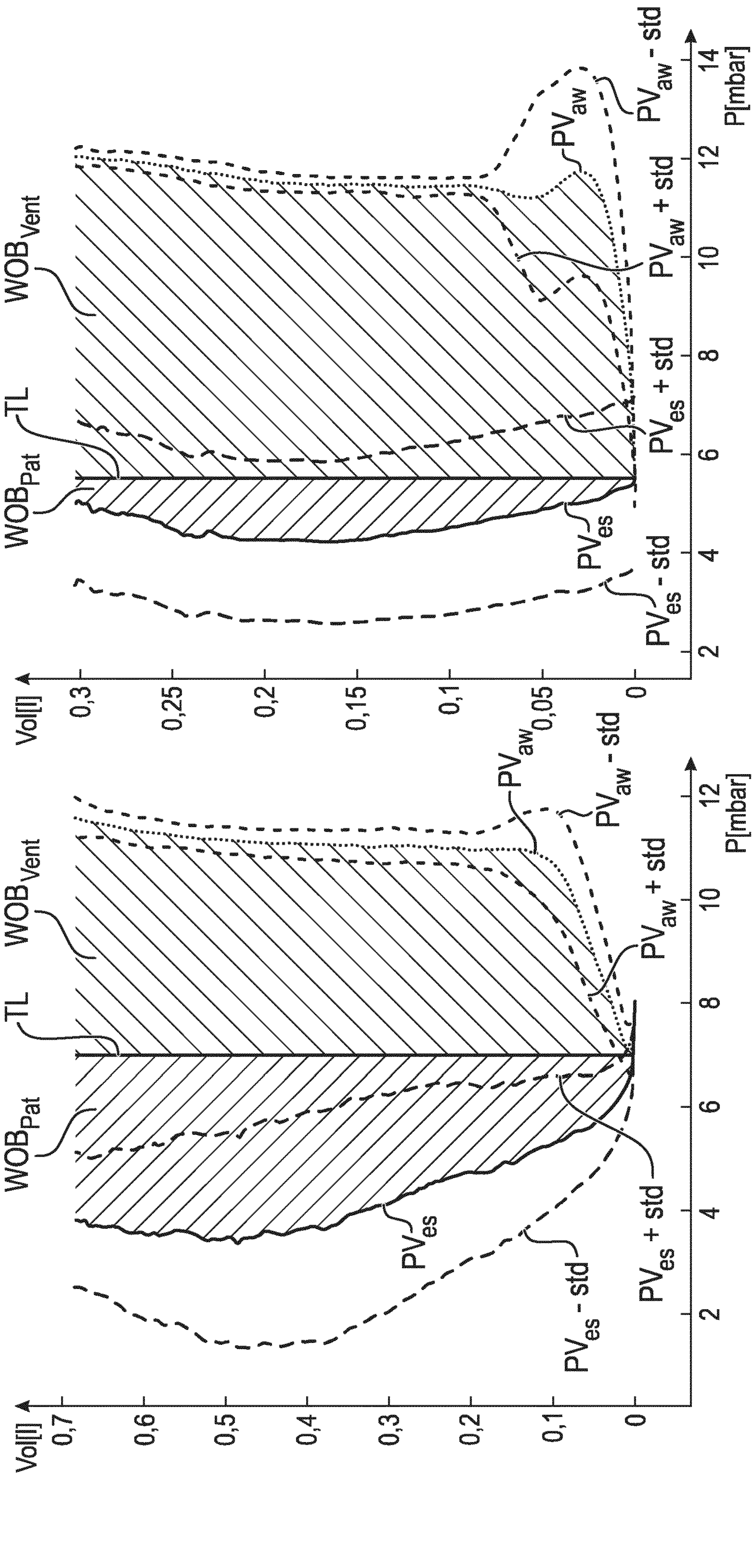
WOB_Pat
TL
WOB_Vent
PV_es
PV_es - std
PV_es + std
PV_aw
PV_aw + std
PV_aw - std
Vol[l]
P[mbar]
WOB_Pat
TL
WOB_Vent
PV_es
PV_es - std
PV_es + std
PV_aw
PV_aw + std
PV_aw - std
Vol[l]
P[mbar]

FIG. 6

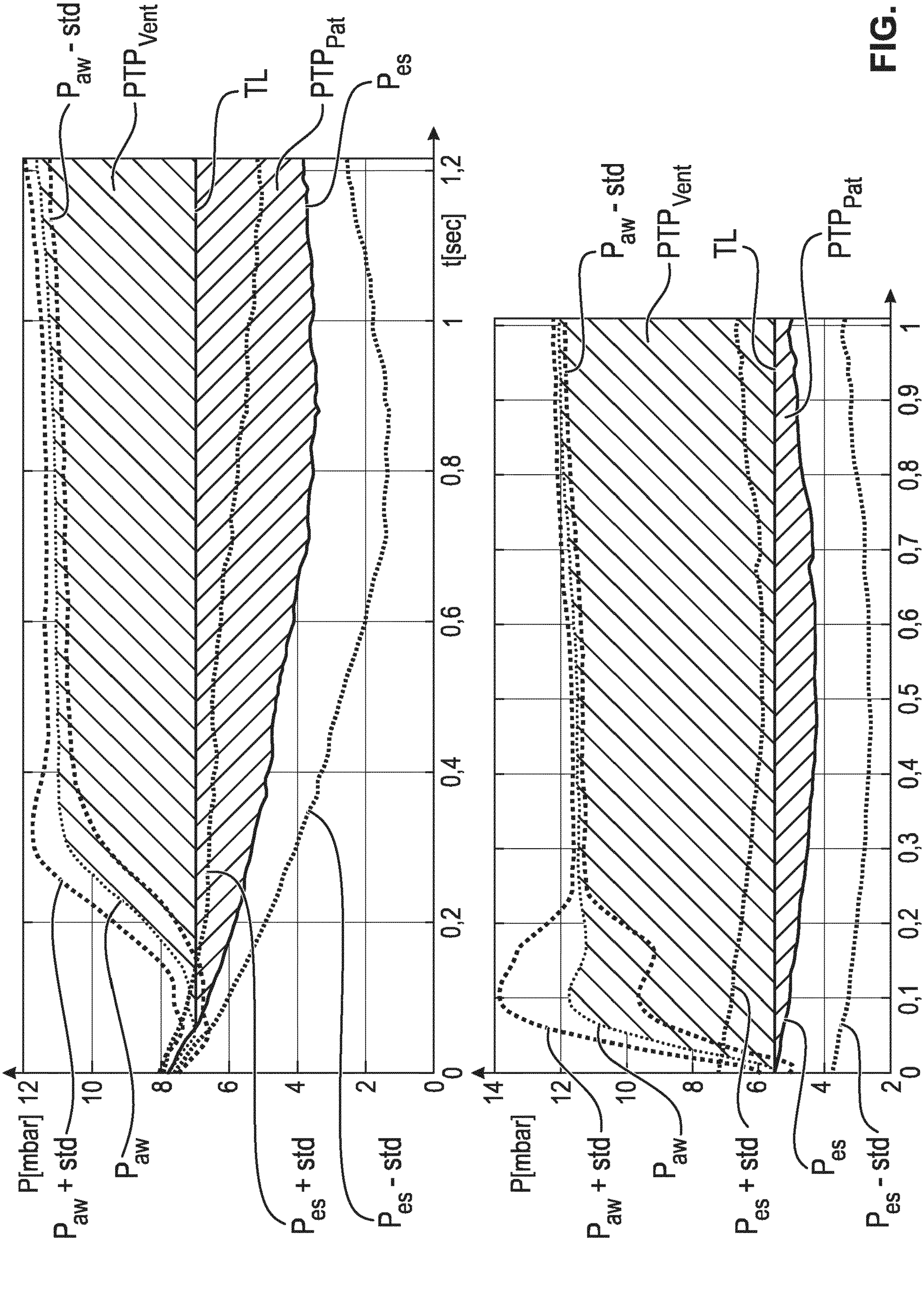
P[mbar]
t[sec]
$P_{aw}$ - std
$PTP_{Vent}$
TL
$PTP_{Pat}$
$P_{es}$
$P_{aw}$ + std
$P_{aw}$
$P_{es}$ + std
$P_{es}$ - std
12
10
8
6
4
2
0
14
0,2
0,4
0,6
0,8
1
1,2
0,1
0,3
0,5
0,7
0,9

FIG. 7

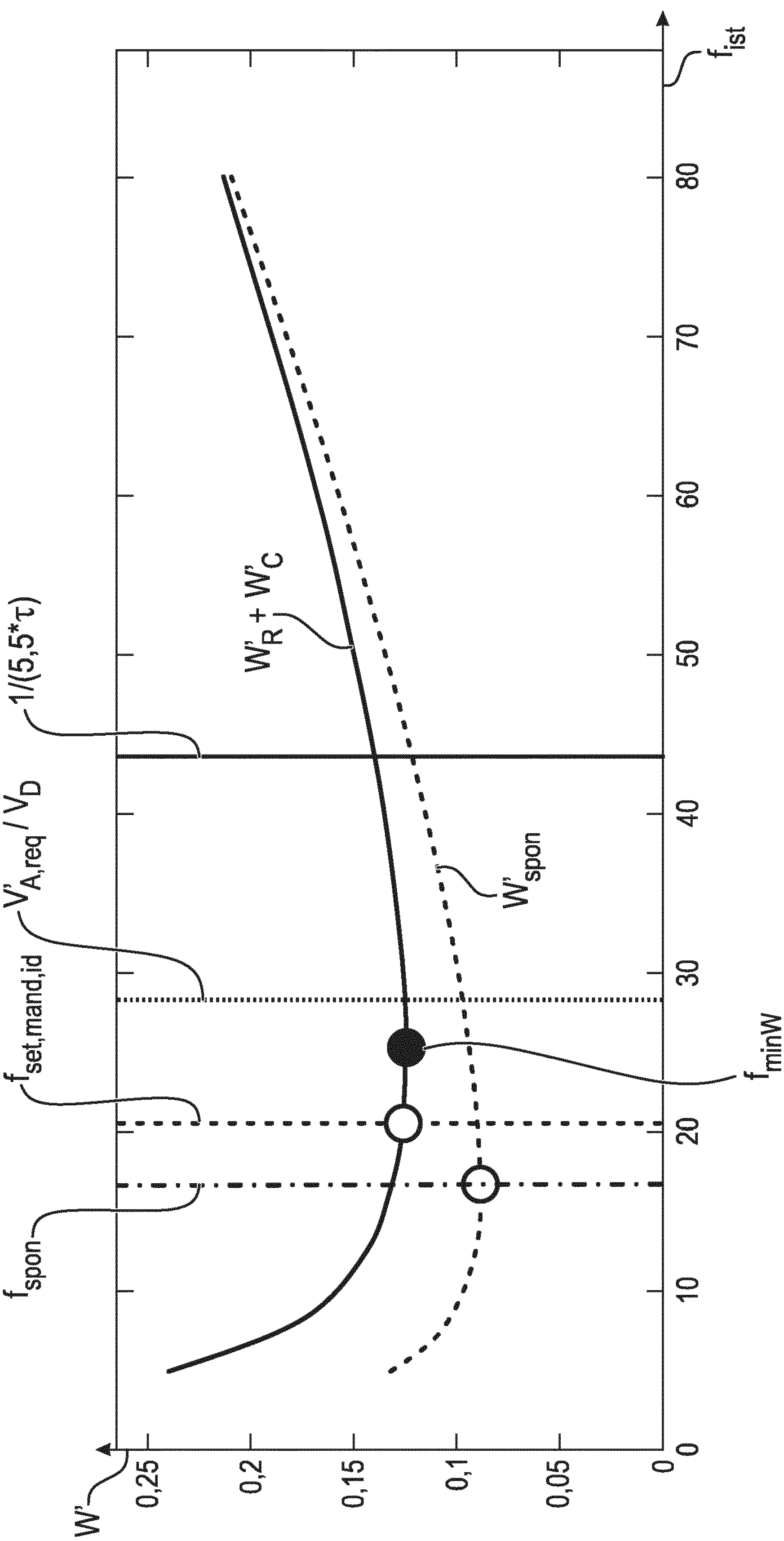
$W'$
0,25
0,2
0,15
0,1
0,05
0
10
20
30
40
50
60
70
80
$f_{ist}$
$f_{spon}$
$f_{set,mand,id}$
$V'_{A,req} / V_D$
1/(5,5*τ)
$W'_R + W'_C$
$W'_{spon}$
$f_{minW}$

FIG. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 3332827 A1
- US 20090007915 A1
- US 20080236582 A1

### In der Beschreibung aufgeführte Nicht-Patentliteratur

- **J. FERNÄNDEZ ; D. MIGUELENA ; H. MULETT ; J. GODOY ; F. MARTINÓN-TORRES.** Adaptive support ventilation: State of the art review. *Indian J. Crit. Care Med.,* 2013, vol. 17 (1), 16
- **J. MEAD.** Control of respiratory frequency. *J. Appl. Physiol.,* 1960, vol. 15 (3), 325-336